(19) Europäisches Patentamt
European Patent Office
Office européen des brevets

(11) EP 4 703 366 A1

(12) # EUROPEAN PATENT APPLICATION

(43) Date of publication:
**04.03.2026 Bulletin 2026/10**

(21) Application number: **24198221.4**

(22) Date of filing: **03.09.2024**

(51) International Patent Classification (IPC):
***C07F 9/6568*** *(2006.01)* ***C07D 213/50*** *(2006.01)*
***C07D 213/84*** *(2006.01)* ***C07D 213/89*** *(2006.01)*
***C07D 239/26*** *(2006.01)* ***C07D 241/12*** *(2006.01)*
***C07D 251/16*** *(2006.01)* ***C07D 409/04*** *(2006.01)*
***C09K 11/06*** *(2006.01)* ***H05B 33/14*** *(2006.01)*
***H10K 50/11*** *(2023.01)*

(52) Cooperative Patent Classification (CPC):
**C07F 9/65685; C07D 213/50; C07D 213/84; C07D 213/89; C07D 239/26; C07D 241/12; C07D 251/16; C07D 409/04; C09K 11/06; H05B 33/14; H10K 50/11**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicant: **Novaled GmbH**
**01099 Dresden (DE)**

(72) Inventors:
- **SCHULZE, Benjamin**
  **01099 Dresden (DE)**
- **UVAROV, Vladimir**
  **01099 Dresden (DE)**
- **PINTER, Piermaria**
  **01099 Dresden (DE)**
- **HEGGEMANN, Ulrich**
  **01099 Dresden (DE)**

(74) Representative: **Michalski Hüttermann & Partner Patentanwälte mbB**
**Kaistraße 16A**
**40221 Düsseldorf (DE)**

(54) **COMPOUND OF FORMULA (I), ORGANIC ELECTRONIC DEVICE COMPRISING A COMPOUND OF FORMULA (I), DISPLAY DEVICE COMPRISING THE ORGANIC ELECTRONIC DEVICE AS WELL AS COMPOUNDS OF FORMULA (I) FOR USE IN ORGANIC ELECTRONIC DEVICES**

(57) The present invention relates to a compound of formula (I) as well as to an organic electronic device comprising a semiconductor layer which comprises a compound of formula (I).

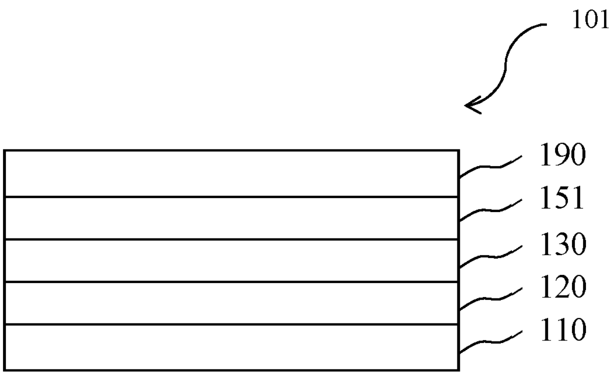


Fig.1

EP 4 703 366 A1

## Description

## Technical Field

The present invention relates to a compound of Formula (I), an organic electronic device comprising a compound of Formula (I) and a display device comprising the organic electronic device. The invention further relates to novel compounds of Formula (I) which can be of use in organic electronic devices.

## Background Art

Organic electronic devices, such as organic light-emitting diodes OLEDs, which are self-emitting devices, have a wide viewing angle, excellent contrast, quick response, high brightness, excellent operating voltage characteristics, and color reproduction. A typical OLED comprises an anode, a hole transport layer HTL, an emission layer EML, an electron transport layer ETL, and a cathode, which are sequentially stacked on a substrate. In this regard, the HTL, the EML, and the ETL are thin films formed from organic compounds.

When a voltage is applied to the anode and the cathode, holes injected from the anode move to the EML, via the HTL, and electrons injected from the cathode move to the EML, via the ETL. The holes and electrons recombine in the EML to generate excitons. When the excitons drop from an excited state to a ground state, light is emitted. The injection and flow of holes and electrons should be balanced, so that an OLED having the above-described structure has excellent efficiency and/or a long lifetime.

Performance of an organic light emitting diode may be affected by characteristics of the organic semiconductor layer, and among them, may be affected by characteristics of the compounds of Formula (I) which are also contained in the organic semiconductor layer.

There remains a need to improve the physical properties of compounds of Formula (I), in particular the thermal properties and/or LUMO energy level.

Furthermore, there remains a need to reduce the environmental impact and/or toxicity of compounds of Formula (I).

Furthermore, there remains a need to improve performance of organic semiconductor materials, semiconductor layers, as well as organic electronic devices thereof, in particular to achieve reduced operating voltage, improved cd/A efficiency, improved external quantum efficiency and/or improved operating voltage stability over time through improving the characteristics of the compounds comprised therein.

DISCLOSURE

An aspect of the present invention provides a compound of Formula (I)

$$M^{n\oplus}(L^{\ominus})_n\ (AL)_m \quad (I),$$

wherein:

- M is a metal ion,
- n is the valency of M and selected from 1 to 4;
- AL is an ancillary ligand which coordinates to the metal ion M;
- m is an integer selected from 0 to 2
- L is a ligand of formula (II)

$$E\text{-}U \quad (II)$$

wherein E is selected from formula (III)

$$^{\ominus}G{-}A \quad (III),$$

wherein

G is selected from O and S;

A is is bound to U via a single or double bond and selected from formula (IV)

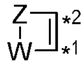 (IV)

wherein the asterisks "*1" denotes the binding position to U; and the asterisks "*2" denotes the binding position to G; whereby
W is selected from S, NR, C=S, and Q=O;
wherein Q is selected from C; S, SO, PR',
wherein R' is selected from substituted or unsubstituted $C_1$ to $C_{16}$ alkyl, substituted or unsubstituted $C_6$ to $C_{40}$ aryl, substituted or unsubstituted $C_3$ to $C_{40}$ heteroaryl;
wherein the one or more substituents on R' is independently selected from D, electron-withdrawing group, F, CN, $NO_2$, substituted or unsubstituted $C_1$ to $C_6$ alkyl, partially fluorinated or perfluorinated $C_1$ to $C_6$ alkyl, $CH_2F$, $CHF_2$, CHDF, $CD_2F$, $CDF_2$, $CF_3$, substituted or unsubstituted $C_1$ to $C_6$ alkoxy, partially fluorinated or perfluorinated $C_1$ to $C_6$ alkoxy, $OCH_2F$, $OCHF_2$, OCHDF, $OCD_2F$, $OCDF_2$, $OCF_3$;
Z is selected from formula (V);

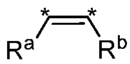 (V),

wherein the asterisks "*" denotes the binding positions to the other moieties of formula (IV);
$R^a$, and $R^b$ are independently selected from H, D, substituted or unsubstituted $C_1$ to $C_{12}$ alkyl, substituted or unsubstituted $C_6$ to $C_{19}$ aryl, substituted or unsubstituted $C_2$ to $C_{20}$ heteroaryl, substituted or unsubstituted 6-membered heteroaryl, electron-withdrawing group, $NO_2$, partially fluorinated or perfluorinated $C_1$ to $C_6$ alkyl, $CH_2F$, $CHF_2$, CHDF, $CD_2F$, $CDF_2$, $CF_3$, substituted or unsubstituted $C_1$ to $C_6$ alkoxy, partially fluorinated or perfluorinated $C_1$ to $C_6$ alkoxy, $OCH_2F$, $OCHF_2$, OCHDF, $OCD_2F$, $OCDF_2$, $OCF_3$, F or CN,
wherein the one or more substituents on $R^a$, and $R^b$ is independently selected from D, electron-withdrawing group, F, CN, $NO_2$, substituted or unsubstituted $C_1$ to $C_6$ alkyl, partially fluorinated or perfluorinated $C_1$ to $C_6$ alkyl, $CH_2F$, $CHF_2$, CHDF, $CD_2F$, $CDF_2$, $CF_3$, substituted or unsubstituted $C_1$ to $C_6$ alkoxy, partially fluorinated or perfluorinated $C_1$ to $C_6$ alkoxy, $OCH_2F$, $OCHF_2$, OCHDF, $OCD_2F$, $OCDF_2$, $OCF_3$;
or
$R^a$, and $R^b$ form together, irrespective of the previous definition, a substituted or unsubstituted aryl ring, substituted or unsubstituted aryl ring system, substituted or unsubstituted heteroaryl ring or substituted or unsubstituted heteroaryl ring system;
wherein the one or more substituents on the aryl ring, the aryl ring system, the heteroaryl ring and heteroaryl ring system is independently selected from D, electron-withdrawing group, F, CN, $NO_2$, substituted or unsubstituted $C_1$ to $C_6$ alkyl, partially fluorinated, or perfluorinated $C_1$ to $C_6$ alkyl, $CH_2F$, $CHF_2$, CHDF, $CD_2F$, $CDF_2$, $CF_3$, substituted or unsubstituted $C_1$ to $C_6$ alkoxy, partially fluorinated or perfluorinated $C_1$ to $C_6$ alkoxy, $OCH_2F$, $OCHF_2$, OCHDF, $OCD_2F$, $OCDF_2$, $OCF_3$;
wherein U is selected from formula (VI)

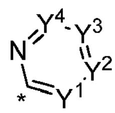 (VI)

wherein the asterisks "*" denotes the binding position;
$Y^1$ is selected from $CR^1$ or N;
$Y^2$ is selected from $CR^2$ or N;
$Y^3$ is selected from $CR^3$ or N;
$Y^4$ is selected from $CR^4$ or N;
wherein 0, 1, or 2 of the group consisting of $Y^1$ ,$Y^2$,$Y^3$, $Y^4$ are selected from N;
$R^1$, $R^2$, $R^3$, and $R^4$ are independently selected from H, D, substituted or unsubstituted $C_1$ to $C_{12}$ alkyl, substituted or unsubstituted $C_6$ to $C_{19}$ aryl, substituted or unsubstituted $C_2$ to $C_{20}$ heteroaryl, substituted or unsubstituted 6-membered heteroaryl, electron-withdrawing group, $NO_2$, partially fluorinated or perfluorinated $C_1$ to $C_6$ alkyl, $CH_2F$, $CHF_2$, CHDF, $CD_2F$, $CDF_2$, $CF_3$, substituted or unsubstituted $C_1$ to $C_6$ alkoxy, partially fluorinated or perfluorinated $C_1$ to $C_6$ alkoxy, $OCH_2F$, $OCHF_2$, OCHDF, $OCD_2F$, $OCDF_2$, $OCF_3$, F or CN,
wherein the one or more substituents on $R^1$, $R^2$, $R^3$, and $R^4$ is independently selected from D, electron-withdrawing group, F, CN, $NO_2$, substituted or unsubstituted $C_1$ to $C_6$ alkyl, partially fluorinated or perfluorinated $C_1$ to $C_6$ alkyl, $CH_2F$, $CHF_2$, CHDF, $CD_2F$, $CDF_2$, $CF_3$, substituted or unsubstituted $C_1$ to $C_6$ alkoxy, partially fluorinated or perfluorinated $C_1$ to $C_6$ alkoxy, $OCH_2F$, $OCHF_2$, OCHDF, $OCD_2F$, $OCDF_2$, $OCF_3$;
and wherein L comprises at least one F and/or CN.

It should be noted that throughout the application and the claims any $R^k$ etc. always refer to the same moieties, unless otherwise noted.

It should be noted that throughout the application and the claims the term "substituents", when referring to Formula (I) and/or ligand L always are meant to be selected from H, D, halogen, Cl, F, CN, $NO_2$, $C_1$ to $C_{12}$ alkyl, $C_1$ to $C_{12}$ alkoxy, partially or perfluorinated $C_1$ to $C_{12}$ alkyl, $CF_3$, $CF_2H$, partially or perfluorinated $C_1$ to $C_{12}$ alkoxy, or a combination thereof.

Throughout the applications and the claims the term "ring system" shall especially mean that adjacent rings share two common atoms.

In the present specification, when a definition is not otherwise provided, "partially fluorinated" refers to an alkyl group or an alkoxy group in which only part of the hydrogen atoms are replaced by fluorine atoms.

In the present specification, when a definition is not otherwise provided, "perfluorinated" refers to an alkyl group or an alkoxy group in which all hydrogen atoms are replaced by fluorine atoms.

In the present specification, when a definition is not otherwise provided, "substituted" refers to one substituted with a deuterium, $C_1$ to $C_{12}$ alkyl and $C_1$ to $C_{12}$ alkoxy.

However, in the present specification "aryl substituted" refers to a substitution with one or more aryl groups, which themselves may be substituted with one or more aryl and/or heteroaryl groups.

Correspondingly, in the present specification "heteroaryl substituted" refers to a substitution with one or more heteroaryl groups, which themselves may be substituted with one or more aryl and/or heteroaryl groups.

In the present specification, when a definition is not otherwise provided, an "alkyl group" refers to a saturated aliphatic hydrocarbyl group. The alkyl group may be a $C_1$ to $C_{12}$ alkyl group. More specifically, the alkyl group may be a $C_1$ to $C_{10}$ alkyl group or a $C_1$ to $C_6$ alkyl group. For example, a $C_1$ to $C_4$ alkyl group includes 1 to 4 carbons in alkyl chain, and may be selected from methyl, ethyl, propyl, iso-propyl, n-butyl, iso-butyl, sec-butyl, and tert-butyl.

Specific examples of the alkyl group may be a methyl group, an ethyl group, a propyl group, an iso-propyl group, a butyl group, an iso-butyl group, a tert-butyl group, a pentyl group, a hexyl group.

In the context of the present invention, "$^iC_nH_{(2n+1)}$" denotes an iso-alkyl group and "$^iC_nF_{(2n+1)}$" denotes a perfluorinated iso-alkyl group.

The term "cycloalkyl" refers to saturated hydrocarbyl groups derived from a cycloalkane by formal abstraction of one hydrogen atom from a ring atom comprised in the corresponding cycloalkane. Examples of the cycloalkyl group may be a cyclopropyl group, a cyclobutyl group, a cyclopentyl group, a cyclohexyl group, a methylcyclohexyl group, an adamantly group and the like.

The term "hetero" is understood the way that at least one carbon atom, in a structure which may be formed by covalently bound carbon atoms, is replaced by another polyvalent atom. Preferably, the heteroatoms are selected from B, Si, N, P, O, S; more preferably from N, P, O, S.

In the present specification, "aryl group" refers to a hydrocarbyl group which can be created by formal abstraction of one hydrogen atom from an aromatic ring in the corresponding aromatic hydrocarbon. Aromatic hydrocarbon refers to a hydrocarbon which contains at least one aromatic ring or aromatic ring system. Aromatic ring or aromatic ring system refers to a planar ring or ring system of covalently bound carbon atoms, wherein the planar ring or ring system comprises a conjugated system of delocalized electrons fulfilling Hückel's rule. Examples of aryl groups include monocyclic groups like phenyl or tolyl, polycyclic groups which comprise more aromatic rings linked by single bonds, like biphenyl, and polycyclic groups comprising fused rings, like naphthyl or fluorenyl.

Analogously, under heteroaryl, it is especially where suitable understood a group derived by formal abstraction of one ring hydrogen from a heterocyclic aromatic ring in a compound comprising at least one such ring.

Under heterocycloalkyl, it is especially where suitable understood a group derived by formal abstraction of one ring hydrogen from a saturated cycloalkyl ring in a compound comprising at least one such ring.

The term "fused aryl rings" or "condensed aryl rings" is understood the way that two aryl rings are considered fused or condensed when they share at least two common $sp^2$-hybridized carbon atoms

In the present specification, the single bond refers to a direct bond.

In the present specification, an electron-withdrawing group (EWG) is a group that reduces electron density in a molecule through the carbon atom it is bonded to. Typical examples of electron withdrawing groups are halogen, in

particular F and Cl, -COR, -COH, -COOR, -COOH, partially fluorinated or perfluorinated alkyl, partially fluorinated or perfluorinated aryl, partially fluorinated or perfluorinated heteroaryl, partially fluorinated or perfluorinated carbocyclyl, partially fluorinated or perfluorinated $C_2$ to $C_{20}$ heterocyclyl, $-NO_2$, and -CN.

In the context of the present invention, "different" means that the compounds do not have an identical chemical structure.

The term "free of', "does not contain", "does not comprise" does not exclude impurities which may be present in the compounds prior to deposition. Impurities have no technical effect with respect to the object achieved by the present invention.

The term "contacting sandwiched" refers to an arrangement of three layers whereby the layer in the middle is in direct contact with the two adjacent layers.

The terms "light-absorbing layer" and "light absorption layer" are used synonymously.

The terms "light-emitting layer", "light emission layer" and "emission layer" are used synonymously.

The terms "OLED", "organic light-emitting diode" and "organic light-emitting device" are used synonymously.

The terms anode, anode layer and anode electrode are used synonymously.

The terms cathode, cathode layer and cathode electrode are used synonymously.

In the specification, hole characteristics refer to an ability to donate an electron to form a hole when an electric field is applied and that a hole formed in the anode may be easily injected into the emission layer and transported in the emission layer due to conductive characteristics according to a highest occupied molecular orbital (HOMO) level.

In addition, electron characteristics refer to an ability to accept an electron when an electric field is applied and that electrons formed in the cathode may be easily injected into the emission layer and transported in the emission layer due to conductive characteristics according to a lowest unoccupied molecular orbital (LUMO) level.

The term "LUMO level" is understood to mean the lowest unoccupied molecular orbital and is determined in eV (electron volt). The LUMO may also be named "LUMO" or "LUMO energy level".

The term "LUMO level further away from vacuum level" is understood to mean that the absolute value of the LUMO level is higher than the absolute value of the LUMO level of the reference compound.

The term "HOMO level" is understood to mean the highest occupied molecular orbital and is determined in eV (electron volt).

The term "HOMO level further away from vacuum level" is understood to mean that the absolute value of the HOMO level is higher than the absolute value of the HOMO level of the reference compound. For example, the term "further away from vacuum level than the HOMO level of N2,N2,N2',N2',N7,N7,N7',N7'-octakis(4-methoxyphenyl)-9,9'-spirobi[fluorene]-2,2',7,7'-tetraamine is understood to mean that the absolute value of the HOMO level of the matrix compound of the hole injection layer is higher than the HOMO level of N2,N2,N2',N2', N7,N7, N7',N7'-octakis(4-methoxyphenyl)-9,9'-spirobi[fluorene]-2,2',7,7'-tetraamine.

The term "absolute value" is understood to mean the value without the "- "symbol. According to one embodiment of the present invention, the HOMO level of the matrix compound of the hole injection layer may be calculated by quantum mechanical methods.

**Advantageous Effects**

Surprisingly, it was found that the compounds according to formula (I) show an unexpected thermal stability and/or lead to devices with improved operating voltage and/or improved voltage stability over time.

Without going into much detail and without being bound by any theory the inventors believe that especially the incorporation ofF and/or CN is advantageous. Fluorinated compounds often show a better volatility whereas compounds with cyano groups often show a better melting point.

According to one embodiment, the molecular mass of L is selected in the range of $\leq$ 700 Da and $\geq$ 150 Da, preferably $\leq$ 650 Da Da and $\geq$ 180 Da, more preferred $\leq$ 600 Da and $\geq$ 200 Da and most preferred $\leq$ 560 Da and $\geq$ 210 Da.

According to one embodiment of the invention, the compound of Formula (I) comprises 10 fluorine atoms or less, preferably 8 fluorine atoms or less.

According to one embodiment of the invention, ligand L comprises 6 fluorine atoms or less, preferably 4 fluorine atoms or less.

According to one embodiment of the invention, the fluorine in the compound of Formula (I) is only contained in the groups selected from $CF_2R$, aryl-F, heteroraryl-F and $CF_3$.

According to one embodiment of the invention, the compound of formula (I) comprises either fluorine or cyano groups, but not both.

According to one embodiment of the invention, in ligand L either the moiety E or the moiety U comprises at least one fluorine or cyano group, but not both.

According to one embodiment of the present invention, the ligand of formula (II) is free of alkoxy groups.

According to an embodiment, the compound of Formula (I) has an difference between the LUMO energy level

and HOMO energy level, i.e. an Egap of ≥ 3.3 eV.

According to an embodiment, the compound of Formula (I) does not emit light in the wave length range ≥400 to ≤780 nm.

According to an embodiment, wherein G is selected from O.

According to a preferred embodiment, A is bound to U via a single bond.

According to an embodiment, W is selected from Q=O, wherein Q is selected from C, S, SO, PR'; preferably from C, SO, PR'; more preferably C, PR'; and most preferably C.

According to an embodiment of the present invention, $R^a$, and $R^b$ of formula (V) form together a substituted or unsubstituted aryl ring, substituted or unsubstituted aryl ring system, substituted or unsubstituted heteroaryl ring or substituted or unsubstituted heteroaryl ring system;

wherein the one or more substituents on the aryl ring, the aryl ring system, the heteroaryl ring and heteroaryl ring system is independently selected from D, electron-withdrawing group, F, CN, $NO_2$, substituted or unsubstituted $C_1$ to $C_6$ alkyl, partially fluorinated or perfluorinated $C_1$ to $C_6$ alkyl, $CH_2F$, $CHF_2$, CHDF, $CD_2F$, $CDF_2$, $CF_3$, substituted or unsubstituted $C_1$ to $C_6$ alkoxy, partially fluorinated or perfluorinated $C_1$ to $C_6$ alkoxy, $OCH_2F$, $OCHF_2$, OCHDF, $OCD_2F$, $OCDF_2$, $OCF_3$;

According to an embodiment, $R^a$, and $R^b$ are independently selected from H, D, substituted or unsubstituted $C_1$ to $C_4$ alkyl, substituted or unsubstituted $C_6$ to $C_{12}$ aryl, substituted or unsubstituted $C_2$ to $C_{12}$ heteroaryl, substituted or unsubstituted 6-membered heteroaryl, electron-withdrawing group, $NO_2$, partially fluorinated or perfluorinated $C_1$ to $C_4$ alkyl, $CH_2F$, $CHF_2$, CHDF, $CD_2F$, $CDF_2$, $CF_3$, substituted or unsubstituted $C_1$ to $C_4$ alkoxy, partially fluorinated or perfluorinated $C_1$ to $C_4$ alkoxy, $OCH_2F$, $OCHF_2$, OCHDF, $OCD_2F$, $OCDF_2$, $OCF_3$, F or CN,

wherein the one or more substituents on $R^a$, and $R^b$ is independently selected from D, electron-withdrawing group, F, CN, $NO_2$, substituted or unsubstituted $C_1$ to $C_6$ alkyl, partially fluorinated or perfluorinated $C_1$ to $C_6$ alkyl, $CH_2F$, $CHF_2$, CHDF, $CD_2F$, $CDF_2$, $CF_3$, substituted or unsubstituted $C_1$ to $C_6$ alkoxy, partially fluorinated or perfluorinated $C_1$ to $C_6$ alkoxy, $OCH_2F$, $OCHF_2$, OCHDF, $OCD_2F$, $OCDF_2$, $OCF_3$;

or $R^a$, and $R^b$ form together, irrespective of the previous definition, substituted or unsubstituted aryl ring, substituted or unsubstituted aryl ring system, substituted or unsubstituted heteroaryl ring or substituted or unsubstituted heteroaryl ring system;

wherein the one or more substituents on the aryl ring, the aryl ring system, the heteroaryl ring and heteroaryl ring system is independently selected from D, electron-withdrawing group, F, CN, $NO_2$, substituted or unsubstituted $C_1$ to $C_4$ alkyl, partially fluorinated or perfluorinated $C_1$ to $C_4$ alkyl, $CH_2F$, $CHF_2$, CHDF, $CD_2F$, $CDF_2$, $CF_3$, substituted or unsubstituted $C_1$ to $C_4$ alkoxy, partially fluorinated or perfluorinated $C_1$ to $C_4$ alkoxy, $OCH_2F$, $OCHF_2$, OCHDF, $OCD_2F$, $OCDF_2$, $OCF_3$.

According to an embodiment, wherein $R^a$, and $R^b$ are independently selected from H, D, substituted or unsubstituted $C_1$ to $C_{12}$ alkyl, substituted or unsubstituted $C_6$ to $C_{19}$ aryl, substituted or unsubstituted $C_2$ to $C_{20}$ heteroaryl, substituted or unsubstituted 6-membered heteroaryl, electron-withdrawing group, $NO_2$, partially fluorinated or perfluorinated $C_1$ to $C_6$ alkyl, $CH_2F$, $CHF_2$, CHDF, $CD_2F$, $CDF_2$, $CF_3$, substituted or unsubstituted $C_1$ to $C_6$ alkoxy, partially fluorinated or perfluorinated $C_1$ to $C_6$ alkoxy, $OCH_2F$, $OCHF_2$, OCHDF, $OCD_2F$, $OCDF_2$, $OCF_3$, F or CN,

wherein the one or more substituents on $R^a$, and $R^b$ is independently selected from D, electron-withdrawing group, F, CN, $NO_2$, substituted or unsubstituted $C_1$ to $C_6$ alkyl, partially fluorinated or perfluorinated $C_1$ to $C_6$ alkyl, $CH_2F$, $CHF_2$, CHDF, $CD_2F$, $CDF_2$, $CF_3$, substituted or unsubstituted $C_1$ to $C_6$ alkoxy, partially fluorinated or perfluorinated $C_1$ to $C_6$ alkoxy, $OCH_2F$, $OCHF_2$, OCHDF, $OCD_2F$, $OCDF_2$, $OCF_3$;

or $R^a$, and $R^b$ form together, irrespective of the previous definition, substituted or unsubstituted 6-member aryl ring, substituted or unsubstituted 6-member heteroaryl ring,

wherein the one or more substituents on the aryl ring, the aryl ring system, the heteroaryl ring and heteroaryl ring system is independently selected from D, electron-withdrawing group, F, CN, $NO_2$, substituted or unsubstituted $C_1$ to $C_6$ alkyl, partially fluorinated or perfluorinated $C_1$ to $C_6$ alkyl, $CH_2F$, $CHF_2$, CHDF, $CD_2F$, $CDF_2$, $CF_3$, substituted or unsubstituted $C_1$ to $C_6$ alkoxy, partially fluorinated or perfluorinated $C_1$ to $C_6$ alkoxy, $OCH_2F$, $OCHF_2$, OCHDF, $OCD_2F$, $OCDF_2$, $OCF_3$.

According to an embodiment, U is selected from formula (VIb), (VIc), (VId), (VIe), and (VIf)

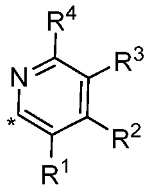 (VIb); 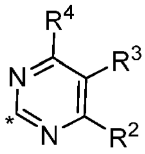 (VIc); 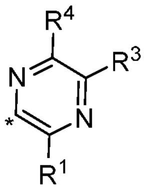 (VId); 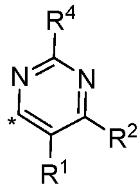 (VIe); 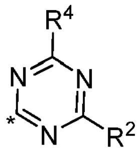 (VIf);

wherein the asterisks "*" denotes the binding position; preferably U is selected from formulae (VIb), (VIc), (VId), and (VIf), more preferably (VIb), (VIc), and (VId), and most preferably (VIb).

According to an embodiment $R^1$, $R^2$, $R^3$, and $R^4$ are independently selected from H, D, substituted or unsubstituted $C_1$ to $C_4$ alkyl, substituted or unsubstituted $C_6$ to $C_{12}$ aryl, substituted or unsubstituted $C_2$ to $C_{12}$ heteroaryl, substituted or unsubstituted 6-membered heteroaryl, electron-withdrawing group, $NO_2$, partially fluorinated or perfluorinated $C_1$ to $C_4$ alkyl, $CH_2F$, $CHF_2$, CHDF, $CD_2F$, $CDF_2$, $CF_3$, substituted or unsubstituted $C_1$ to $C_4$ alkoxy, partially fluorinated or perfluorinated $C_1$ to $C_4$ alkoxy, $OCH_2F$, $OCHF_2$, OCHDF, $OCD_2F$, $OCDF_2$, $OCF_3$, F or CN,
wherein the one or more substituents on $R^1$, $R^2$, $R^3$, and $R^4$ is independently selected from D, electron-withdrawing group, F, CN, $NO_2$, substituted or unsubstituted $C_1$ to $C_4$ alkyl, partially fluorinated or perfluorinated $C_1$ to $C_4$ alkyl, $CH_2F$, $CHF_2$, CHDF, $CD_2F$, $CDF_2$, $CF_3$, substituted or unsubstituted $C_1$ to $C_4$ alkoxy, partially fluorinated or perfluorinated $C_1$ to $C_4$ alkoxy, $OCH_2F$, $OCHF_2$, OCHDF, $OCD_2F$, $OCDF_2$, $OCF_3$.

According to an embodiment, $R^1$, $R^2$, $R^3$, and $R^4$ in formula (VI), (VIb), (VIc), (VId), (VIe), (VIf), (VIII-1a), (VIII-2a), (VIII-1b), (VIII-2b), (VIII-1c), (VIII-2c), (VIII-1d), (VIII-2d), (VIII-2e), (VIII-2f), (VIII-2g), (VIII-1e), (VIII-If), (VIII-1g), (VIII-1h), (VIII-Ij), and (VIII-1k), (VIII-2h), (VIII-2j),(VIII-2K), (X-1), (X-2), (X-1b), (X-2b), (X-1c), (X-2c), (X-1d), (X-1e), (X-1f), (X-2d), (X-2e), (X-2f), (XI-1), (XI-2), (X-1b), (XI-2b), (XI-1c), (XI-1d), (XI-1e), (XI-1f), (XI-2c), (XI-2d), (XI-2e), (XI-2f), (XII-1), (XII-2), (XII-1b), (XII-2b), (XII-1c), (XII-2c), (XII-1d), (XII-1e), (XII-1f) (XId), (XIe), and (XIf) are independently selected from H, D, substituted or unsubstituted $C_1$ to $C_4$ alkyl, substituted or unsubstituted $C_6$ to $C_{12}$ aryl, substituted or unsubstituted $C_2$ to $C_{12}$ heteroaryl, substituted or unsubstituted 6-membered heteroaryl, electron-withdrawing group, $NO_2$, partially fluorinated or perfluorinated $C_1$ to $C_4$ alkyl, $CH_2F$, $CHF_2$, CHDF, $CD_2F$, $CDF_2$, $CF_3$, substituted or unsubstituted $C_1$ to $C_4$ alkoxy, partially fluorinated or perfluorinated $C_1$ to $C_4$ alkoxy, $OCH_2F$, $OCHF_2$, OCHDF, $OCD_2F$, $OCDF_2$, $OCF_3$, F or CN, wherein the one or more substituents on $R^1$, $R^2$, $R^3$, and $R^4$ is independently selected from D, electron-withdrawing group, F, CN, $NO_2$, substituted or unsubstituted $C_1$ to $C_4$ alkyl, partially fluorinated or perfluorinated $C_1$ to $C_4$ alkyl, $CH_2F$, $CHF_2$, CHDF, $CD_2F$, $CDF_2$, $CF_3$, substituted or unsubstituted $C_1$ to $C_4$ alkoxy, partially fluorinated or perfluorinated $C_1$ to $C_4$ alkoxy, $OCH_2F$, $OCHF_2$, OCHDF, $OCD_2F$, $OCDF_2$, $OCF_3$.

According to an embodiment, A and/ or formula (IV) is selected from formula (IVb)

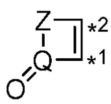 (IVb);

wherein the asterisks "*1" denotes the binding position to U; and the asterisks "*2" denotes the binding position to G; wherein Q is selected from C, S, S=O, P-R'; preferably C, S=O, P-R'; more preferably C, S=O; and most preferably C.

According to an embodiment, A, formula (IV) and/or (IVb) are selected from formulae (IVc), (IVd), and (IVe)

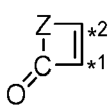 (IVc), 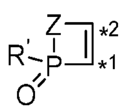 (IVd), 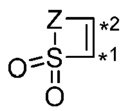 (IVe);

wherein the asterisks "*1" denotes the binding position to U and the asterisks "*2" denotes the binding position to G; preferably from formula (IVc), and (IVd); and more preferably from formula (IVc).
wherein the asterisks "*1" denotes the binding position to U; and the asterisks "*2" denotes the binding position to G.

According to an embodiment, E and/or formula (III) are selected from formula (VII)

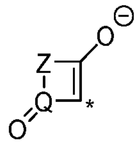
(VII);

wherein the asterisks "*" denotes the binding position;
wherein Q is selected from C, S, S=O, P-R'; preferably C, S=O, P-R'; more preferably C, S=O; and most preferably C.

According to an embodiment, E, formulae (III), and/or (VII) are selected from formula (VIIb), (VIIc), and (VIId).

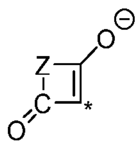
(VIIb),
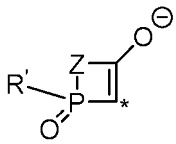
(VIIc),
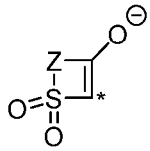
(VIId);

wherein the asterisks "*" denotes the binding position;
preferably from formula (VIIb), and (VIIc); and more preferably from formula (VIIb).

According to an embodiment, L and/or formula (II) are selected from formula (VIII-1a)

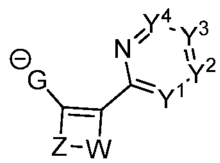
(VIII -1a).

According to an embodiment, L, formula (II) and/or (VIII-1a) are selected from formula (VIII-2a)

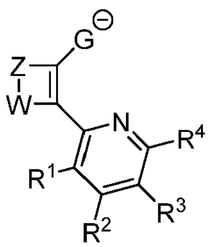
(VIII-2a).

According to an embodiment, L, formula (II) and/or (VIII-1a) are selected from formula (VIII-1b)

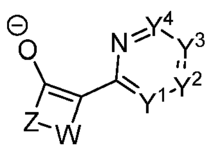
(VIII-1b).

According to an embodiment, L, formula (II), (VIII-1a), (VIII-1b), and/or (VIII-2a) are selected from formula (VIII-2b)

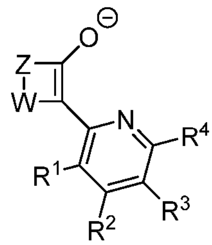

(VIII-2b).

According to an embodiment, L, formula (II) and/or (VIII-1a) are selected from formula (VIII-1c)

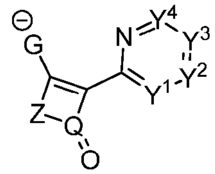

(VIII-1c);

wherein Q is selected from C, S, S=O, P-R'; preferably C, S=O, P-R'; more preferably C, S=O; and most preferably C.

According to an embodiment, L, formula (II), (VIII-1a), (VIII-2a), and/or (VIII-1c) are selected from (VIII-2c)

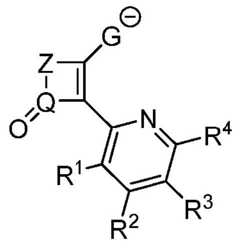

(VIII-2c);

wherein Q is selected from C, S, S=O, P-R'; preferably C, S=O, P-R'; more preferably C, S=O; and most preferably C.

According to an embodiment, L, formula (II), (VIII-1a), (VIII-1b), and/or (VIII-1c) are selected from (VIII-1d)

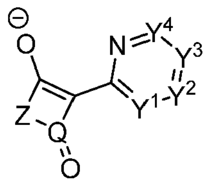

(VIII-1d);

wherein Q is selected from C, S, S=O, P-R'; preferably C, S=O, P-R'; more preferably C, S=O; and most preferably C.

According to an embodiment, wherein L, formula (II), (VIII-1a), (VIII-2a), (VIII-1b), (VIII-2b), (VIII-1c), (VIII-2c) and/or (VIII-1d) are selected from (VIII-2d)

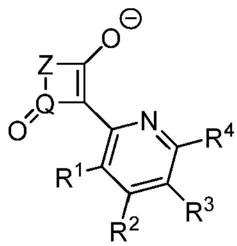

(VIII-2d);

wherein Q is selected from C, S, S=O, P-R'; preferably C, S=O, P-R'; more preferably C, S=O; and most preferably C.

According to an embodiment, wherein L and/or formula (II), (VIII-1a) and (VIII-1c) are selected from (VIII-1e), (II-1f), and (II-1g)

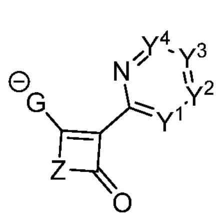 (VIII-1e); 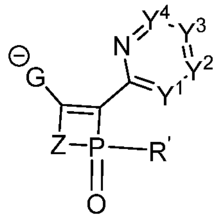 (VIII-1f), 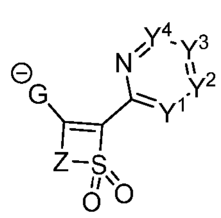 (VIII-1g),

preferably from formulae (VIII-1e), and (VIII-If), and more preferably from (VIII-1e).

According to an embodiment, L, formula (II), (VIII-1a), (VIII-2a), (VIII-1c), (VIII-2c), (VIII-1e), (VIII-If), and/or (VIII-1g) are selected from formula (VIII-2e), (VIII-2f) and (VIII-2g)

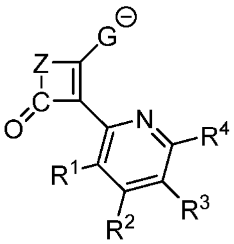 (VIII-2e); 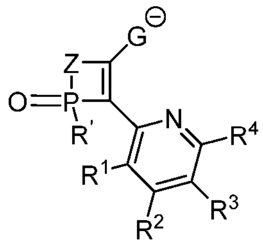 (VIII-2f), 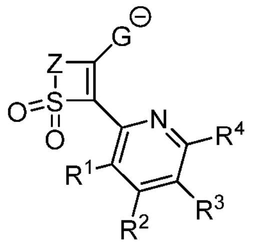 (VIII-2g);

preferably from formula (VIII-2e), and (VIII-2f), and more preferably from (VIII-2e).

According to an embodiment, L, formula (II), (VIII-1a), (VIII-1b), (VIII-1c), (VIII-1d), (VIII-1e), (VIII-If), and/or (VIII-1g) are selected from formula (VIII-1h), (VIII-1j) and (VIII-1k)

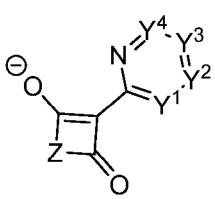 (VIII-1h); 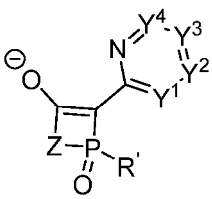 (VIII-1j), 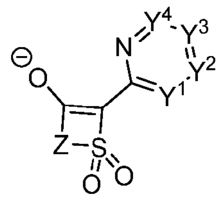 (VIII-1k),

preferably from formula (VIII-1h), and (VIII-1j); and more preferably from (VIII-1h).

According to an embodiment, L, formula (II), (VIII-1a), (VIII-2a), (VIII-1b), (VIII-2b), (VIII-1c), (VIII-2c), (VIII-1d), (VIII-2d), (VIII-2e), (VIII-2f), (VIII-2g), (VIII-1e), (VIII-If), (VIII-1g), (VIII-1h), (VIII-1j), and/or (VIII-1k) are selected from formula (VIII-2h), (VIII-2j), and (VIII-2K)

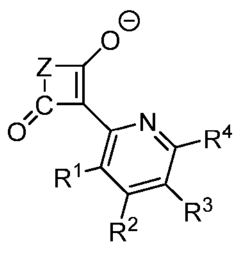 (VIII-2h); 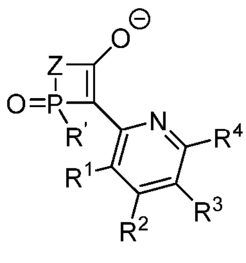 (VIII-2j), 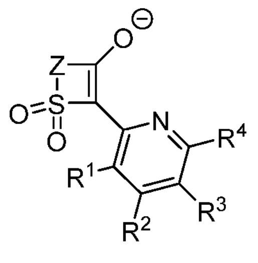 (VIII-2k),

preferably from formula (VIII-2h), and (VIII-2j); and more preferably from (VIII-2h).

According to an embodiment, Z and /or formula (V) are selected from formula (Vb)

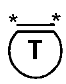 (Vb);

wherein the asterisk "*" denotes the binding position;

wherein T is substituted or unsubstituted aryl ring, substituted or unsubstituted aryl ring system, substituted or unsubstituted heteroaryl ring or substituted or unsubstituted heteroaryl ring system;

wherein the one or more substituents on T is independently selected from D, electron-withdrawing group, F, CN, $NO_2$, substituted or unsubstituted $C_1$ to $C_6$ alkyl, partially fluorinated or perfluorinated $C_1$ to $C_6$ alkyl, $CH_2F$, $CHF_2$, CHDF, $CD_2F$, $CDF_2$, $CF_3$, substituted or unsubstituted $C_1$ to $C_6$ alkoxy, partially fluorinated or perfluorinated $C_1$ to $C_6$ alkoxy, $OCH_2F$, $OCHF_2$, OCHDF, $OCD_2F$, $OCDF_2$, $OCF_3$.

According to an embodiment, T in formulae (Vb), (IX), (IXb), (IXg), (IXh), (IXj), (X-1), (X-2), (X-1b), (X-2b), (X-1c), (X-2c), (X-1d), (X-1e), (X-1f), (X-2d), (X-2e), and (X-2f) is independently selected from substituted or unsubstituted $C_6$ to $C_{12}$ aryl, substituted or unsubstituted $C_2$ to $C_{12}$ heteroaryl, or substituted or unsubstituted 6-membered heteroaryl, wherein the one or more substituents on $C_6$ to $C_{12}$ aryl, $C_2$ to $C_{12}$ heteroaryl, and 6-membered heteroaryl is independently selected from D, electron-withdrawing group, F, CN, $NO_2$, substituted or unsubstituted $C_1$ to $C_4$ alkyl, partially fluorinated or perfluorinated $C_1$ to $C_4$ alkyl, $CH_2F$, $CHF_2$, CHDF, $CD_2F$, $CDF_2$, $CF_3$, substituted or unsubstituted $C_1$ to $C_4$ alkoxy, partially fluorinated or perfluorinated $C_1$ to $C_4$ alkoxy, $OCH_2F$, $OCHF_2$, OCHDF, $OCD_2F$, $OCDF_2$, $OCF_3$.

According to an embodiment, T in formulae (Vb), (IX), (IXb), (IXg), (IXh), (IXj), (X-1), (X-2), (X-1b), (X-2b), (X-1c), (X-2c), (X-1d), (X-1e), (X-1f), (X-2d), (X-2e), and (X-2f) is independently selected from substituted or unsubstituted $C_6$ to $C_{12}$ aryl, substituted or unsubstituted $C_2$ to $C_{12}$ heteroaryl, or substituted or unsubstituted 6-membered heteroaryl, wherein the one or more substituents on $C_6$ to $C_{12}$ aryl, $C_2$ to $C_{12}$ heteroaryl, and 6-membered heteroaryl is independently selected from D, electron-withdrawing group, F, CN, $NO_2$, substituted or unsubstituted $C_1$ to $C_4$ alkyl, partially fluorinated or perfluorinated $C_1$ to $C_4$ alkyl, $CH_2F$, $CHF_2$, CHDF, $CD_2F$, $CDF_2$, $CF_3$, substituted or unsubstituted $C_1$ to $C_4$ alkoxy, partially fluorinated or perfluorinated $C_1$ to $C_4$ alkoxy, $OCH_2F$, $OCHF_2$, OCHDF, $OCD_2F$, $OCDF_2$, $OCF_3$.

According to an embodiment, Z, formula (V), and/or (Vb) are selected from formula (Vc)

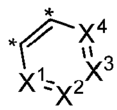
(Vc);

wherein the asterisk "*" denotes the binding position;
wherein

- $X^1$ is selected from $CR^5$ or N;
- $X^2$ is selected from $CR^6$ or N;
- $X^3$ is selected from $CR^7$ or N;
- $X^4$ is selected from $CR^8$ or N;

wherein $R^5$, $R^6$, $R^7$, and $R^8$ are independently selected from H, D, substituted or unsubstituted $C_1$ to $C_{12}$ alkyl, substituted or unsubstituted $C_6$ to $C_{19}$ aryl, substituted or unsubstituted $C_2$ to $C_{20}$ heteroaryl, substituted or unsubstituted 6-membered heteroaryl, F or CN,
wherein the one or more substituents on $R^5$, $R^6$, $R^7$, and $R^8$ is independently selected from D, electron-withdrawing group, F, CN, $NO_2$, substituted or unsubstituted $C_1$ to $C_6$ alkyl, partially fluorinated or perfluorinated $C_1$ to $C_6$ alkyl, $CH_2F$, $CHF_2$, CHDF, $CD_2F$, $CDF_2$, $CF_3$, substituted or unsubstituted $C_1$ to $C_6$ alkoxy, partially fluorinated or perfluorinated $C_1$ to $C_6$ alkoxy, $OCH_2F$, $OCHF_2$, OCHDF, $OCD_2F$, $OCDF_2$, $OCF_3$.

According to an embodiment, Z, formula (V), (Vb), and/or formula (Vc) are selected from formula (Vd)

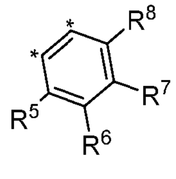
(Vd);

wherein the asterisk "*" denotes the binding position;
wherein $R^5$, $R^6$, $R^7$, and $R^8$ are independently selected from H, D, substituted or unsubstituted $C_1$ to $C_{12}$ alkyl, substituted or unsubstituted $C_6$ to $C_{19}$ aryl, substituted or unsubstituted $C_2$ to $C_{20}$ heteroaryl, substituted or unsubstituted 6-membered heteroaryl, F or CN,
wherein the one or more substituents on $R^1$, $R^2$, $R^3$, and $R^4$ is independently selected from D, electron-withdrawing group, F, CN, $NO_2$, substituted or unsubstituted $C_1$ to $C_6$ alkyl, partially fluorinated or perfluorinated $C_1$ to $C_6$ alkyl, $CH_2F$, $CHF_2$, CHDF, $CD_2F$, $CDF_2$, $CF_3$, substituted or unsubstituted $C_1$ to $C_6$ alkoxy, partially fluorinated or

perfluorinated $C_1$ to $C_6$ alkoxy, $OCH_2F$, $OCHF_2$, OCHDF, $OCD_2F$, $OCDF_2$, $OCF_3$.

According to an embodiment, $R^5$, $R^6$, $R^7$, and $R^8$ are independently selected from H, D, substituted or unsubstituted $C_1$ to $C_4$ alkyl, substituted or unsubstituted $C_6$ to $C_{12}$ aryl, substituted or unsubstituted $C_2$ to $C_{12}$ heteroaryl, substituted or unsubstituted 6-membered heteroaryl, electron-withdrawing group, $NO_2$, partially fluorinated or perfluorinated $C_1$ to $C_4$ alkyl, $CH_2F$, $CHF_2$, CHDF, $CD_2F$, $CDF_2$, $CF_3$, substituted or unsubstituted $C_1$ to $C_4$ alkoxy, partially fluorinated or perfluorinated $C_1$ to $C_4$ alkoxy, $OCH_2F$, $OCHF_2$, OCHDF, $OCD_2F$, $OCDF_2$, $OCF_3$, F or CN,
wherein the one or more substituents on $R^5$, $R^6$, $R^7$, and $R^8$ is independently selected from D, electron-withdrawing group, F, CN, $NO_2$, substituted or unsubstituted $C_1$ to $C_4$ alkyl, partially fluorinated or perfluorinated $C_1$ to $C_4$ alkyl, $CH_2F$, $CHF_2$, CHDF, $CD_2F$, $CDF_2$, $CF_3$, substituted or unsubstituted $C_1$ to $C_4$ alkoxy, partially fluorinated or perfluorinated $C_1$ to $C_4$ alkoxy, $OCH_2F$, $OCHF_2$, OCHDF, $OCD_2F$, $OCDF_2$, $OCF_3$.

According to an embodiment, $R^5$, $R^6$, $R^7$, and $R^8$ in formulae (Vc), (Vd), (IXc), (IXd), (IXe), (IXf), (IXk), (IXm), (IXn), (IXo), (IXp), (IXq), (XI-1), (XI-2), (XI-1b), (XI-2b), (XI-1c), (XI-2c), (XI-1d), (XI-1e), and (XI-1f), (XI-2d), (XI-2e), (XI-2f), (XII-1), (XII-2), (XII-1b), (XII-2b), (XII-1c), (XII-2c), (XII-1d), (XII-1e), (XII-1f), (XId), (XIe), and (XIf) are independently selected from H, D, substituted or unsubstituted $C_1$ to $C_4$ alkyl, substituted or unsubstituted $C_6$ to $C_{12}$ aryl, substituted or unsubstituted $C_2$ to $C_{12}$ heteroaryl, substituted or unsubstituted 6-membered heteroaryl, electron-withdrawing group, $NO_2$, partially fluorinated or perfluorinated $C_1$ to $C_4$ alkyl, $CH_2F$, $CHF_2$, CHDF, $CD_2F$, $CDF_2$, $CF_3$, substituted or unsubstituted $C_1$ to $C_4$ alkoxy, partially fluorinated or perfluorinated $C_1$ to $C_4$ alkoxy, $OCH_2F$, $OCHF_2$, OCHDF, $OCD_2F$, $OCDF_2$, $OCF_3$, F or CN,
wherein the one or more substituents on $R^5$, $R^6$, $R^7$, and $R^8$ is independently selected from D, electron-withdrawing group, F, CN, $NO_2$, substituted or unsubstituted $C_1$ to $C_4$ alkyl, partially fluorinated or perfluorinated $C_1$ to $C_4$ alkyl, $CH_2F$, $CHF_2$, CHDF, $CD_2F$, $CDF_2$, $CF_3$, substituted or unsubstituted $C_1$ to $C_4$ alkoxy, partially fluorinated or perfluorinated $C_1$ to $C_4$ alkoxy, $OCH_2F$, $OCHF_2$, OCHDF, $OCD_2F$, $OCDF_2$, $OCF_3$.

According to an embodiment, E and/or formula (III) are selected from formula (IX)

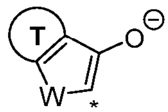 (IX);

wherein the asterisk "*" denotes the binding position.

According to an embodiment, E, formula (III), and/or (IX) are selected from formula (IXb)

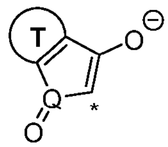 (IXb);

wherein the asterisk "*" denotes the binding position,
wherein Q is selected from C, S, S=O, P-R'; preferably C, S=O, P-R'; more preferably C, P-R'; and most preferably C.

According to an embodiment, E, formula (III) and/or (IX) are selected from formula (IXc)

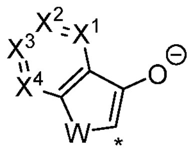 (IXc);

wherein the asterisk "*" denotes the binding position.

According to an embodiment, E, formula (III), (IX), (IXb), and/or (IXc) are selected from formula (IXd)

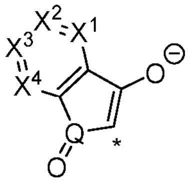

(IXd);

wherein the asterisk "*" denotes the binding position;
wherein Q is selected from C, S, S=O, P-R'; preferably C, S=O, P-R'; more preferably C, P-R'; and most preferably C.

According to an embodiment, E, formula (III), (IX), and/or (IXc) are selected from formula (IXe)

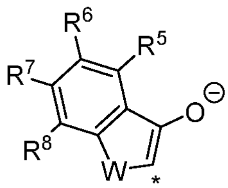

(IXe);

wherein the asterisk "*" denotes the binding position.

According to an embodiment, E, formula (III), (IX), (IXb), (IXc), (IXd), and/or (IXe) are selected from formula (IXf)

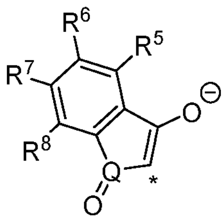

(IXf);

wherein the asterisk "*" denotes the binding position;
wherein Q is selected from C, S, S=O, P-R'; preferably C, S=O, P-R'; more preferably C, P-R'; and most preferably C.

According to an embodiment, wherein E, formula (III), (IX), and/or (IXb) are selected from formula (IXg), (IXh), and (IXj)

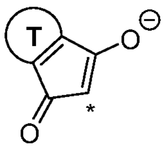

(IXg);

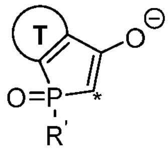

(IXh);

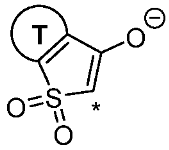

(IXj);

wherein the asterisk "*" denotes the binding position;
preferably from formula (IXg), and (IXh); and more preferably from (IXj).

According to an embodiment, E, formula (III), (IX), (IXb), (IXc), (IXd), (IXe), (IXf), (IXg), (IXh) and/or (IXj) are selected from formula (IXk), (IXm), and (IXn)

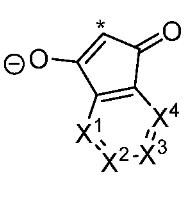

(IXk);

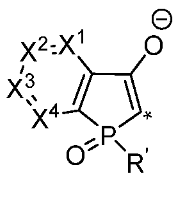

(IXm);

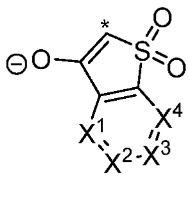

(IXn);

wherein the asterisk "*" denotes the binding position;
preferably from formula (IXk), and (IXm); and more preferably from (IXk).

According to an embodiment, E, formula (III), (IX), (IXb), (IXc), (IXd), (IXe), (IXf), (IXg), (IXh), (IXj), (IXk), (IXm), and/or (IXn) are selected from formulae (IXo), (IXp), and (IXq)

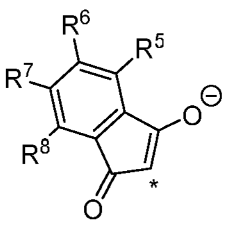 (IXo); 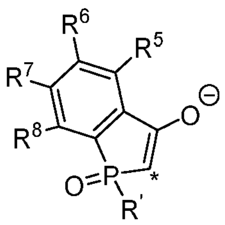 (IXp); 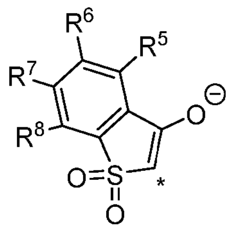 (IXq);

wherein the asterisk "*" denotes the binding position;
preferably from formula (IXo), and (IXp); and more preferably from formula (IXo).

According to an embodiment, L and/or formula (II) are selected from formula (X-1)

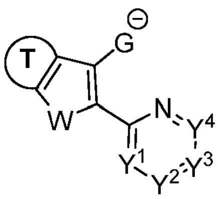 (X-1).

According to an embodiment, L, and/or formula (II) are selected from formula (X-2)

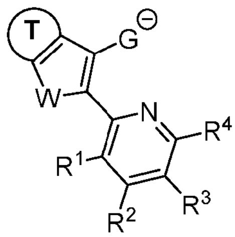 (X-2).

According to an embodiment, L, formula (II), and/or (X-1) are selected from formula (X-1b)

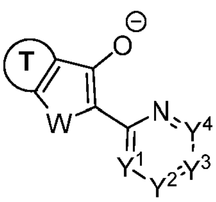 (X-1b).

According to an embodiment, L, formula (II), (X-1), (X-2), and/or (X-1b) are selected from formula (X-2b)

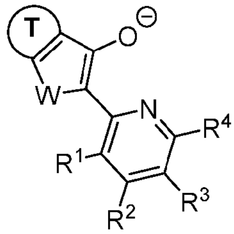

(X-2b).

According to an embodiment, L, formula (II), (X-1) (X-1b), and/or (X-2b) are selected from formula (X-1c)

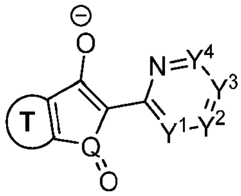

(X-1c);

wherein Q is selected from C, S, S=O, P-R'; preferably C, S=O, P-R'; more preferably C, P-R'; and most preferably C.

According to an embodiment, L, formula (II), (X-1), (X-2), (X-1b), (X-2b) and/or (X-1c) are selected from formula (X-2c)

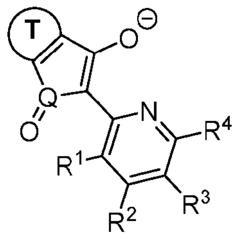

(X-2c);

wherein Q is selected from C, S, S=O, P-R'; preferably C, S=O, P-R'; more preferably C, P-R'; and most preferably C.

According to an embodiment, L, formula (II), (X-1), (X-1b), (X-2b), (X-1c) and (X-2c) are selected from formula (X-1d), (X-1e), and (X-1f)

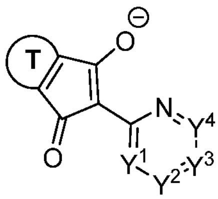

(X-1d);

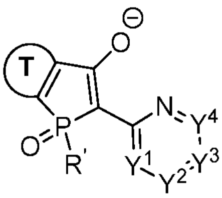

(X-1e);

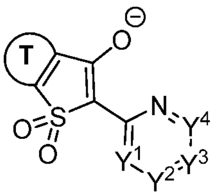

(X-1f)

preferably from (X-1d), and (X-1e), and more preferably from (X-1d).

According to an embodiment, L, formulae (II), (X-1), (X-2), (X-1b), (X-2b), (X-1c), (X-2c), (X-1d), (X-1e) and/or (X-1f) are selected from formula (X-2d), (X-2e), and (X-2f)

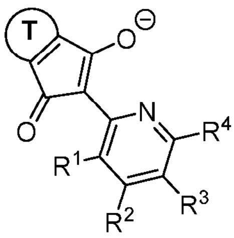

(X-2d);

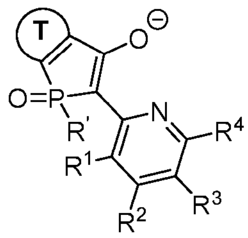

(X-2e);

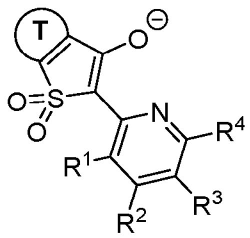

(X-2f),

preferably selected from formula (X-2d), and.(X-2e), and more preferably from formula (X-2f).

According to an embodiment, L, formulae (II), and/or (X-1) are selected from formula (XI-1)

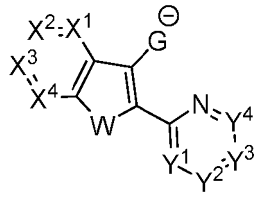
(XI-1).

According to an embodiment, L, formula (II), (X-1), (X-2), and/or (XI-1) are selected from formula (XI-2)

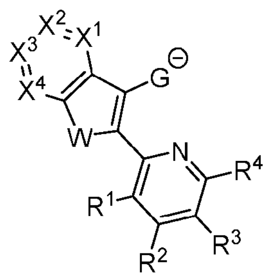
(XI-2).

According to an embodiment, L, formula (II), (X-1), (X-1b), and/or (XI-1) are selected from formula (XI-1b)

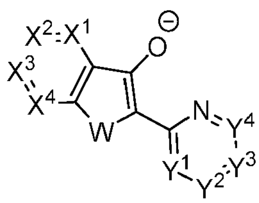
(XI-1b).

According to an embodiment, L, formula (II), (X-1), (X-2), (X-1b), (X-2b), (XI-1), (XI-2) and/or (XI-1b) are selected from formula (XI-2b)

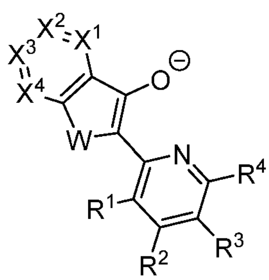
(XI-2b).

According to an embodiment, L, formula (II), (X-1), (X-1b), (XI-1) and/or (XI-1b) are selected from formula (XI-1c)

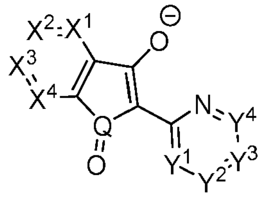
(XI-1c);

wherein Q is selected from C, S, S=O, P-R'; preferably C, S=O, P-R'; more preferably C, P-R'; and most preferably C.

According to an embodiment, L, formula (II), (X-1), (X-2), (X-1b), (X-2b), (X-1c), (X-2c), (XI-1), (XI-2), (X-1b), (XI-2b) and/or (XI-1c) are selected from formula (X-2c)

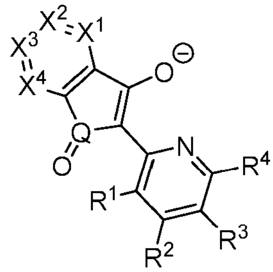

(X-2c);

wherein Q is selected from C, S, S=O, P-R'; preferably C, S=O, P-R'; more preferably C, P-R'; and most preferably C.

According to an embodiment, L, formula (II), (X-1), (X-1b), (XI-1), (XI-1b) and/or (XI-1c) are selected from formula (XI-1d), (XI-1e), and (XI-1f)

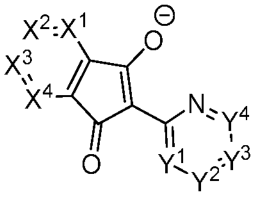

(XI-1d);

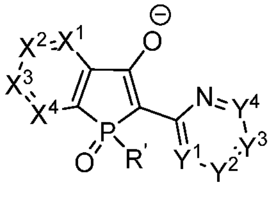

(XI-1e);

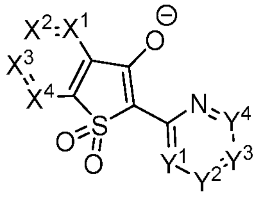

(XI-1f),

preferably selected from formula (XI-1d), and (XI-1e), more preferably selected from (XI-1d).

According to an embodiment, L, formula (II), (X-1), (X-2), (X-1b), (X-2b), (X-1c), (X-2c), (XI-1), (XI-2), (XI-1b), (XI-2b), (XI-1c), (XI-1d), (XI-1e) and/or (XI-1f) are selected from formula (XI-2d), (XI-2e), and (XI-2f)

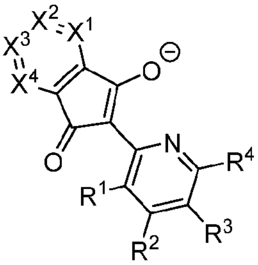

(XI-2d);

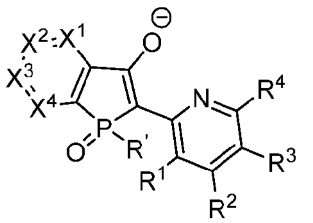

(XI-2e);

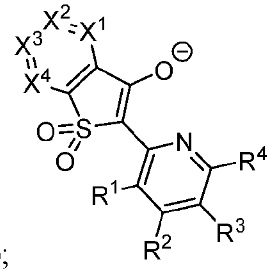

(XI-2f),

preferably from formula (XI-2d), and (XI-2e), and more preferably from (XI-2d).

According to an embodiment, L, formula (II), (X-1), and/or (XI-1) are selected from formula (XII-1)

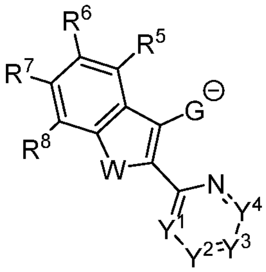

(XII-1).

According to an embodiment, L, formula (II), (X-1), (X-2), (XI-1), (XI-2) and/or (XII-1) are selected from formula (XII-2)

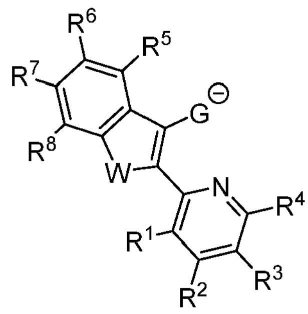
(XII-2).

According to an embodiment, L, formula (II), (X-1), (X-1b), (XI-1), (XI-1b), (XII-1) and/or (XII-2) are selected from formula (XII-1b)

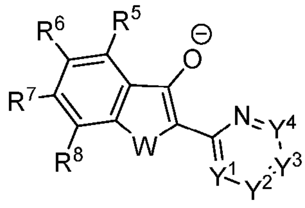
(XII-1b).

According to an embodiment, L, formula (II), (X-1), (X-2), (X-1b), (X-2b), (XI-1), (XI-2), (XI-1b), (XI-2b), (XII-1), (XII-2) and/or (XII-1b) are selected from formula (XII-2b)

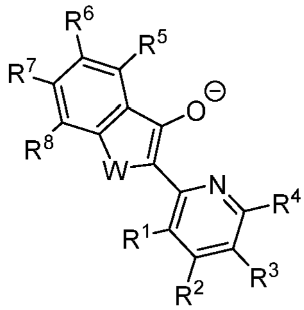
(XII-2b).

According to an embodiment, L, formula (II), (X-1), (X-1b), (X-1c), (X-1b), (X-2b), (XI-1), (XI-1b), (XI-1c), (XII-1) and/or (XII-1b) are selected from formula (XII-1c)

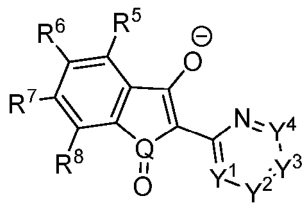
(XII-1c);

wherein Q is selected from C, S, S=O, P-R'; preferably C, S=O, P-R'; more preferably C, P-R'; and most preferably C.

According to an embodiment, L, formula (II), (X-1), (X-2), (X-1b), (X-2b), (X-1c), (X-2c), (XI-1), (XI-2), (XI-1b), (XI-2b), (XI-1c), (XI-2c), (XII-1), (XII-2), (XII-1b), (XII-2b) and/or (XII-1c) are selected from formula (XII-2c)

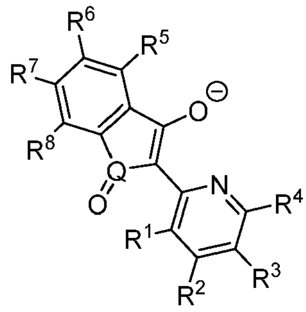

(XII-2c);

wherein Q is selected from C, S, S=O, P-R'; preferably C, S=O, P-R'; more preferably C, P-R'; and most preferably C.

According to an embodiment, L, formulae (II), (X-1), (X-1b), (X-1c), (X-1b), (X-2b), (XI-1), (XI-1b), (XI-1c), (XII-1), (XII-1b) and/or (XII-1c) are selected from formula (XII-1d), (XII-1e), and (XII-1f)

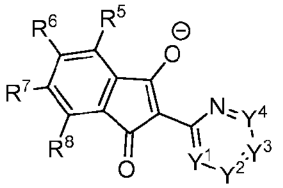

(XI-1d);

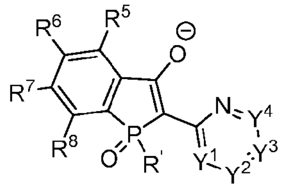

(XI-1e);

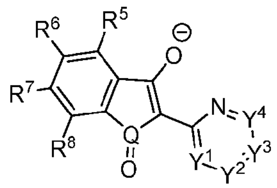

(XI-1f);

preferably from formula (XII-1d), and (XII-1e), and more preferably from (XII-1d).

According to an embodiment, L, formula (II), (X-1), (X-2), (X-1b), (X-2b), (X-1c), (X-2c), (XI-1), (XI-2), (XI-1b), (XI-2b), (XI-1c), (XI-2c), (XII-1), (XII-2), (XII-1b), (XII-2b), (XII-1c), (XII-2c), (XII-1d), (XII-1e) and/or (XII-1f) are selected from formula (XId), (XIe), and (XIf)

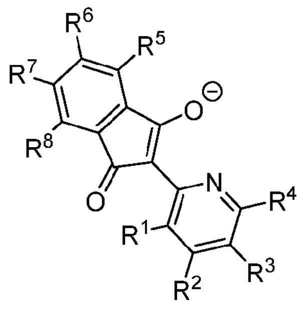

(XII-2d);

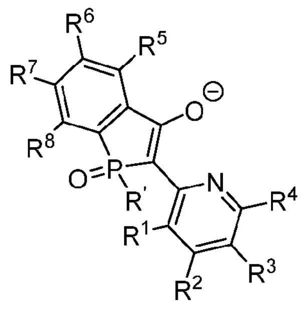

(XII-2e);

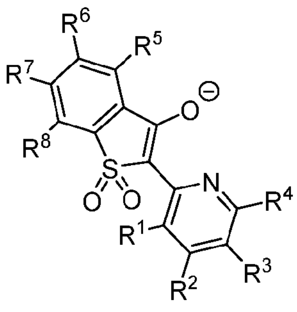

(XII-2f);

preferably from formula (XII-2d), and (XII-2e), and more preferably from (XII-2d).

Ancillary Ligand "AL"

According to one embodiment of the application, AL (the ancillary ligand) is selected from the group comprising $H_2O$, $C_2$ to $C_{40}$ mono- or multi-dentate ethers and $C_2$ to $C_{40}$ thioethers, $C_2$ to $C_{40}$ amines, $C_2$ to $C_{40}$ phosphine, $C_2$ to $C_{20}$ alkyl nitrile or $C_2$ to $C_{40}$ aryl nitrile, or a compound according to Formula (AL-I);

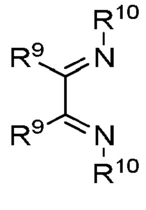

(AL-I),

wherein

$R^9$ and $R^{10}$ are independently selected from $C_1$ to $C_{20}$ alkyl, $C_1$ to $C_{20}$ heteroalkyl, $C_6$ to $C_{20}$ aryl, heteroaryl with 5 to 20 ring-forming atoms, halogenated or perhalogenated $C_1$ to $C_{20}$ alkyl, halogenated or perhalogenated $C_1$ to $C_{20}$ heteroalkyl, halogenated or perhalogenated $C_6$ to $C_{20}$ aryl, halogenated or perhalogenated heteroaryl with 5 to 20 ring-forming atoms, or at least one $R^6$ and $R^7$ are bridged and form a 5 to 20 member ring, or the two $R^6$ and/or the two $R^7$ are bridged and form a 5 to 40 member ring or form a 5 to 40 member ring comprising an unsubstituted or $C_1$ to $C_{12}$ substituted phenanthroline.

The term "m"

The term "m" is an integer selected from 0 to 2, which corresponds to the oxidation number of M. According to one embodiment "m" is an integer selected from 0 or 1. According to another embodiment "m" is an integer selected from 1. According to another embodiment "m" is an integer selected from 2. Preferably "m" is an integer and may be selected from 0.

According to another embodiment of compound of Formula (I), wherein n = 2 or 3; and/or m is an integer selected from 0 or 1, preferably 0.

M of the compound of Formula (I)

The term "M" represents a metal ion.

According to one embodiment, M of the compound of Formula (I) may be selected from a metal ion wherein the corresponding metal has an electronegativity value according to Allen of ≥ 0.65 and ≤ 1.9.

The term "electronegativity value according to Allen" especially refers to Allen, Leland C. (1989). "Electronegativity is the average one-electron energy of the valence-shell electrons in ground-state free atoms", Journal of the American Chemical Society 111 (25): 9003-9014.

According to one embodiment, M may be selected from a transition metal or group III or V metal.

According to one embodiment, the atomic mass of M is selected in the range of ≥ 54 Da and ≤ 200 Da, preferably in the range of ≥ 55 Da and ≤ 138 Da.

According to an embodiment, M is selected from an alkali metal, alkaline earth metal, metal of the group III to V, or rare earth metal;

According to an embodiment, wherein M is selected from Li(I), K(I), Rb(I), Cs(I), Mg(II), Ag(I), Cu(II), Zn(II), Pd(II), Ir(III), Al(III), Ga(III), Mn(II), Sn(II), Mn(III), Ru(III), In(III), Fe(II), Fe(III), Ce(IV).

According to a preferred embodiment, wherein M is selected from Mg(II), Cu(II), Mn(Π), Zn(II), Sn (II), Fe(II), Fe(III), In(III), Ce(IV)

According to a more preferred embodiment, M is selected from Mg(II), Cu(II), Mn(II), Zn(II), Fe(III), Ce(IV).

According to a most preferred embodiment, wherein M is selected from Cu(II), Mn(II), and Zn(II).

According to an embodiment, Z is selected from J1 to J34:

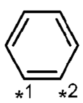 J1, 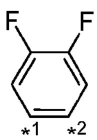 J2, 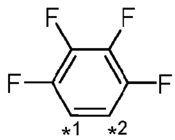 J3, 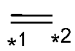 J4, 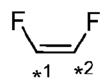 J5, 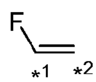 J6, 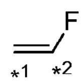 J7, 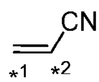 J8,

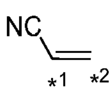 J9, 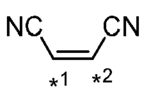 J10, 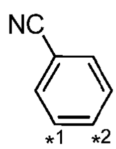 J11, 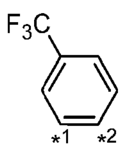 J12, 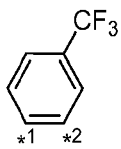 J13, 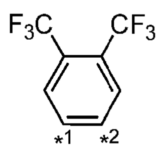 J14,

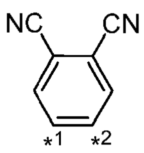 J15, 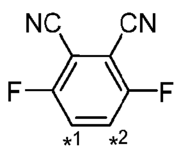 J16, 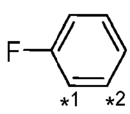 J17, 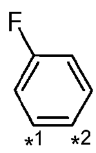 J18, 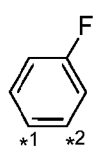 J19, 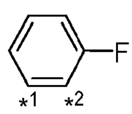 J20,

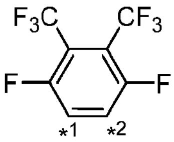 J21, 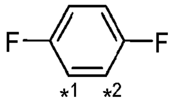 J22, 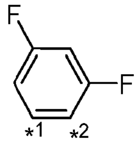 J23, 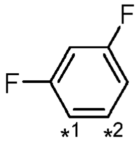 J24, 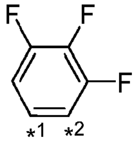 J25, 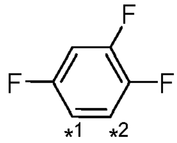

J26, 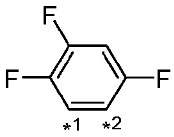 J27, 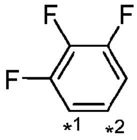 J28, 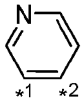 J29, 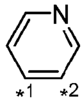 J30, 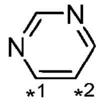 J31, 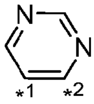 J32, 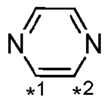

J33, 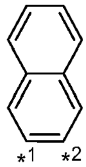 J34;

wherein the asterisks "*1" denotes the binding position to W; and the asterisks "*2" denotes the binding position to the ethylene moiety.

According to a preferred embodiment, Z is selected from J1 to J5.

According to an embodiment, A is selected from H1 to H49

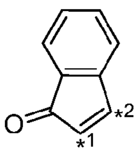 H1, 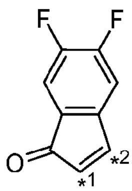 H2, 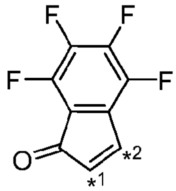 H3, 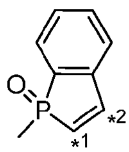 H4, 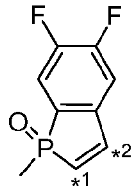 H5, 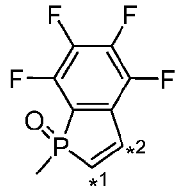 H6,

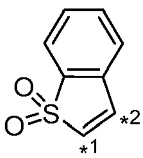 H7, 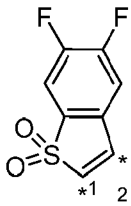 H8, 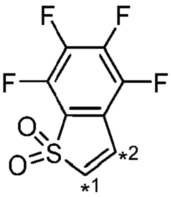 H9, 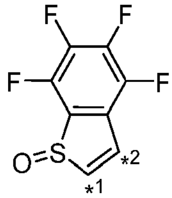 H10, 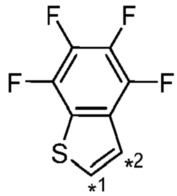 H11, 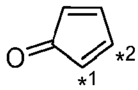

H12, 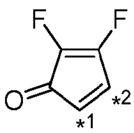 H13, 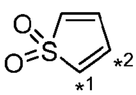 H14, 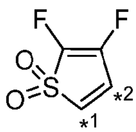 H15, 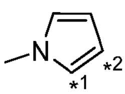 H16, 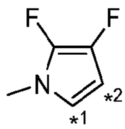 H17, 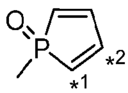

H18, 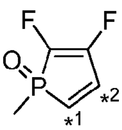 H19, 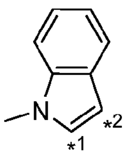 H20, 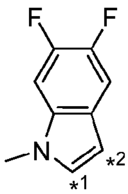 H21, 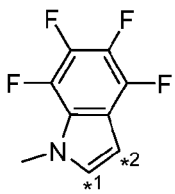 H22, 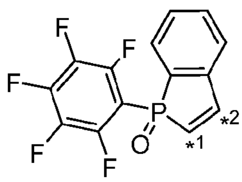 H23,

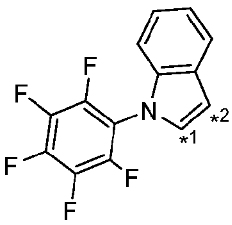 H24, 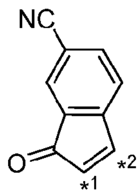 H25, 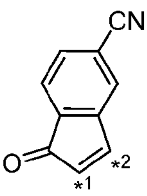 H26, 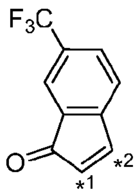 H27, 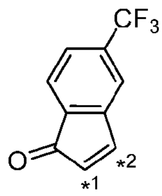 H28,

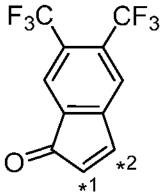 H29, 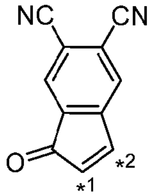 H30, 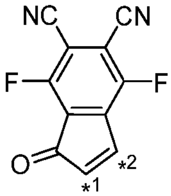 H31, 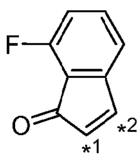 H32, 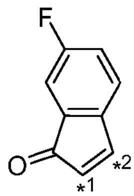 H33, 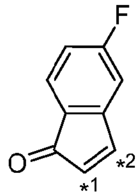

H34, 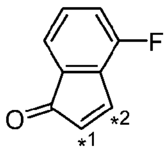 H35, 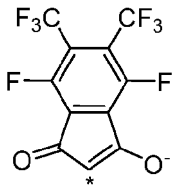 H36, 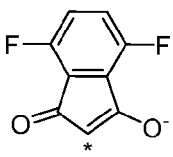 H37, 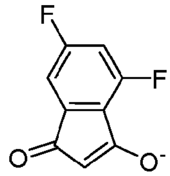 H 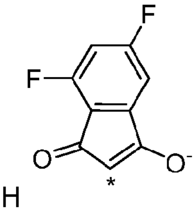 H39,

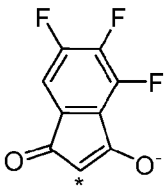 H40, 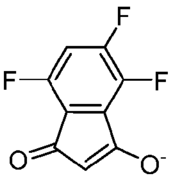 H41, 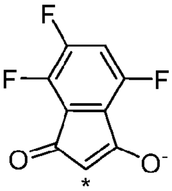 H42, 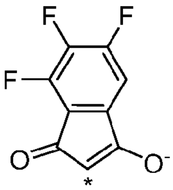 H43, 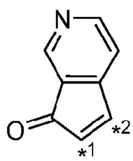 H44,

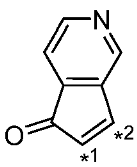 H45, 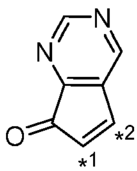 H46, 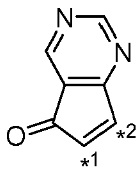 H47, 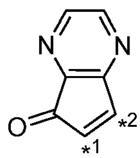 H48, 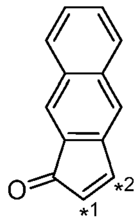 H49;

wherein the asterisks "*1" denotes the binding position to HA; and the asterisks "*2" denotes the binding position to G.

According to an embodiment, A is selected from H1 to H11, more preferred H1 to H9 and most preferred H1 to H4.

According to an embodiment, E is selected from F1 to F41:

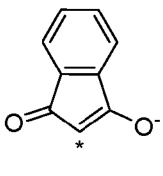 F1, 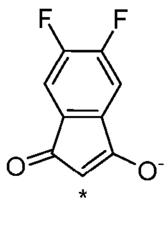 F2, 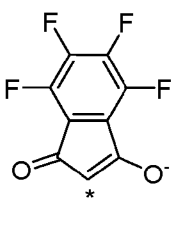 F3, 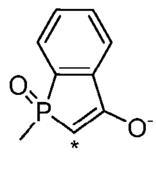 F4, 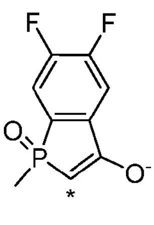 F5, 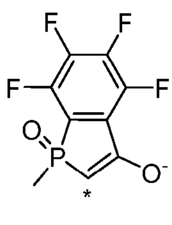

F6, 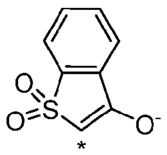 F7, 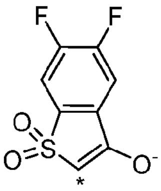 F8, 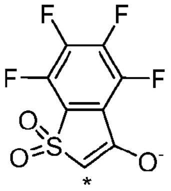 F9, 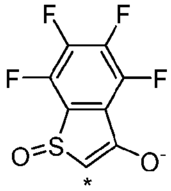 F10, 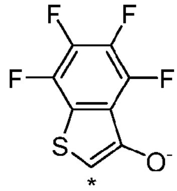 F11,

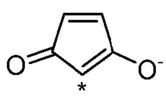 F12, 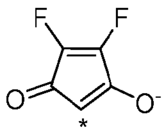 F13, 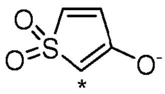 F14, 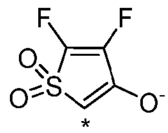 F15, 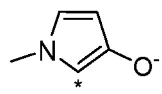 F16,

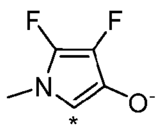 F17, 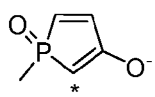 F18, 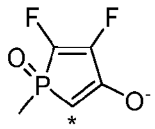 F19, 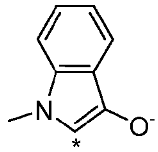 F20, 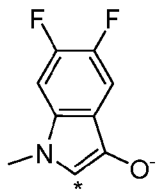 F21,

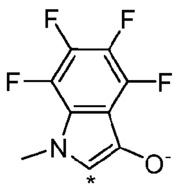 F22, 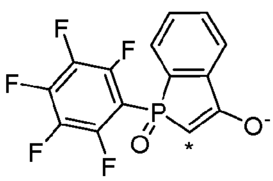 F23, 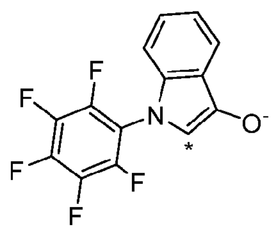 F24, 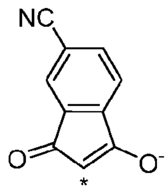 F25,

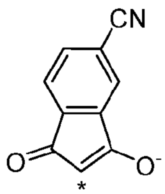 F26, 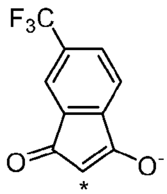 F27, 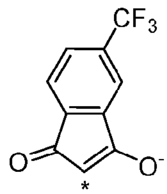 F28, 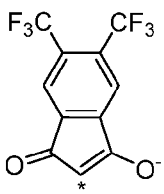 F29, 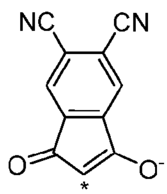 F30,

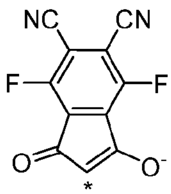 F31, 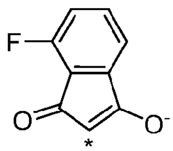 F32, 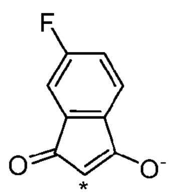 F33, 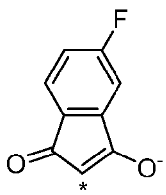 F34, 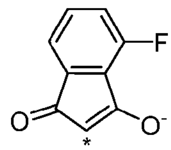 F35,

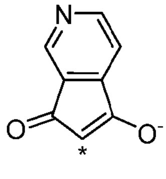 F36, 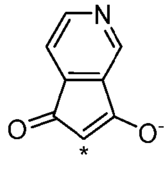 F37, 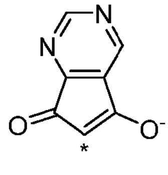 F38, 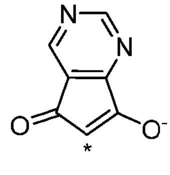 F39, 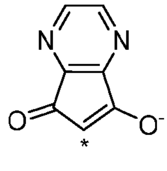 F40,

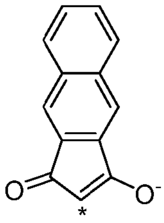 F41,

wherein the asterisk "*" denotes the binding position.

According to an embodiment, E is selected from F1 to F11, more preferred F1 to F9 and most preferred F1 to F6.

According to an embodiment, U is selected from D1 to D39

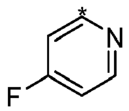 D1, 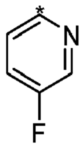 D2, 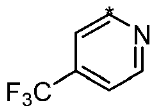 D3, 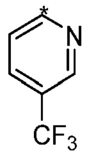 D4, 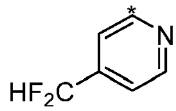 D5, 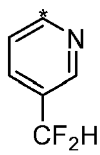 D6, 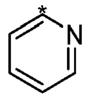 D7,

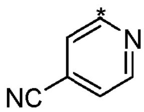 D8, 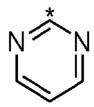 D9, 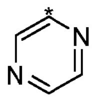 D10, 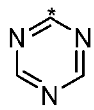 D11, 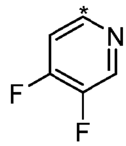 D12, 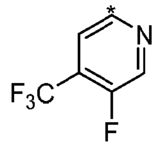 D13,

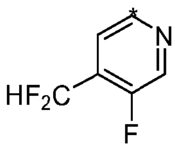 D14, 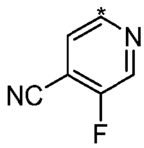 D15, 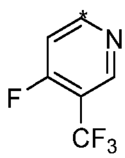 D16, 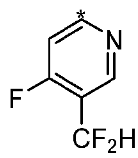 D17, 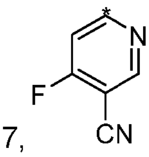 D18, 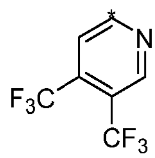

D19, 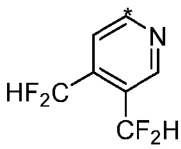 D20, 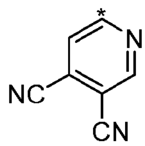 D21, 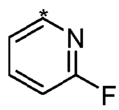 D22, 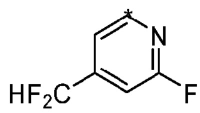 D23, 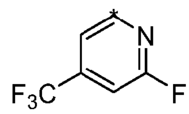

D24, 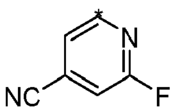 D25, 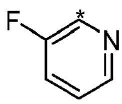 D26, 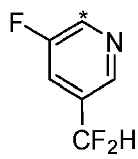 D27, 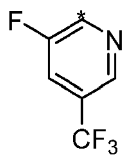 D28, 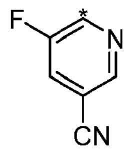 D29,

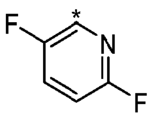 D30, 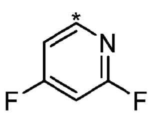 D31, 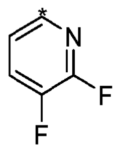 D32, 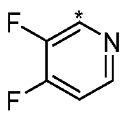 D33, 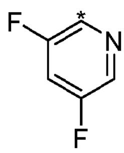 D34, 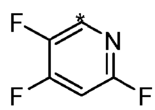 D35,

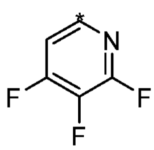 D36, 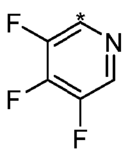 D37, 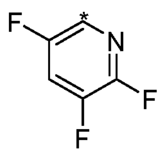 D38, 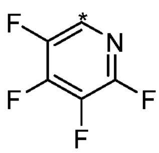 D39.

wherein the asterisk "*" denotes the binding position.

According to a preferred embodiment, U is selected from D1 to D11, more preferred D1 to D8 and most preferred D1 to D7.

According to an embodiment, L is selected from L1 to L144:

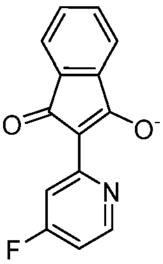 L1, 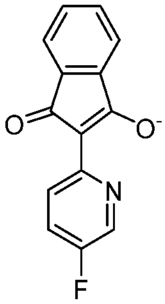 L2, 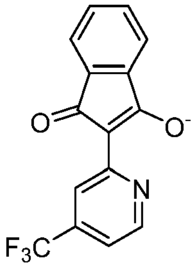 L3, 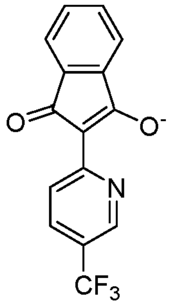 L4, 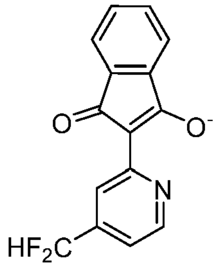 L5,

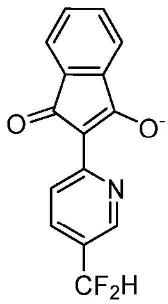 L6, 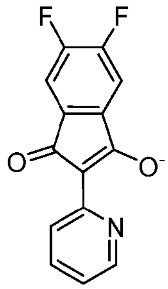 L7, 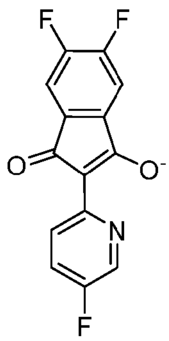 L8, 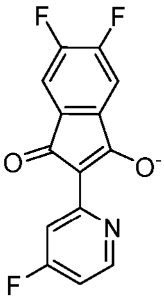 L9, 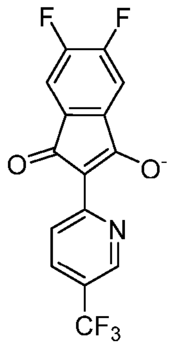 L10,

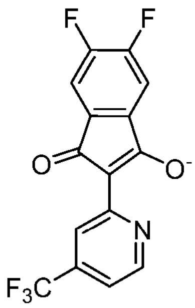 L11, 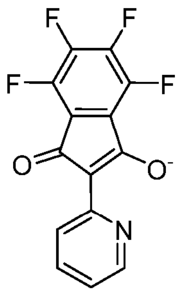 L12, 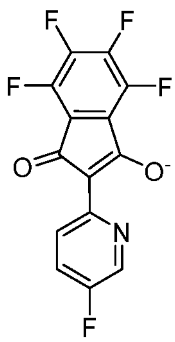 L13, 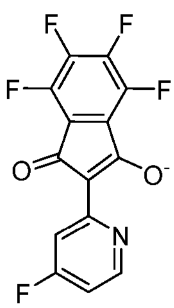 L14, 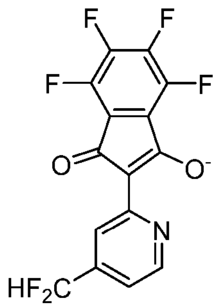 L15,

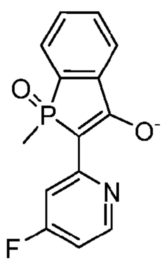 L16, 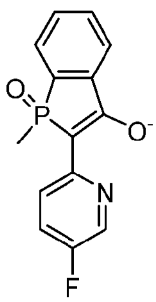 L17, 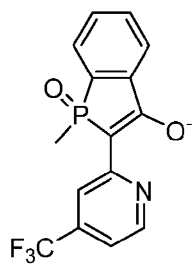 L18, 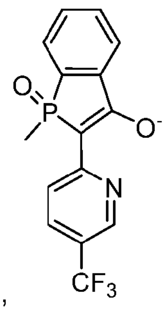 , 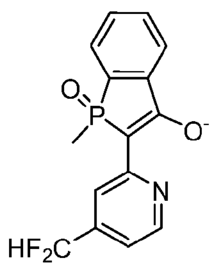 L19,

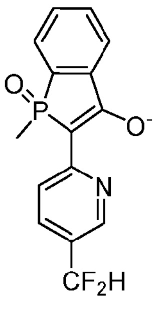 L20, 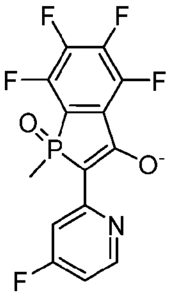 L21, 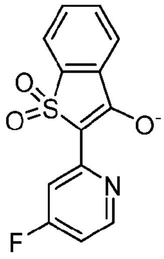 L22, 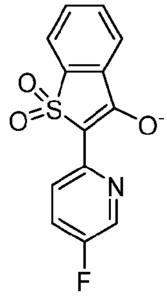 L23, 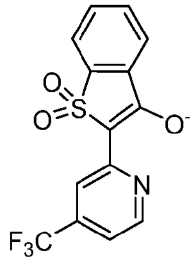 L24,

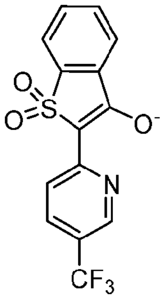 L25, 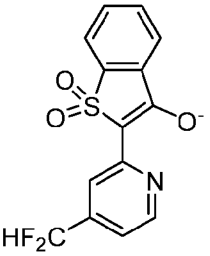 L26, 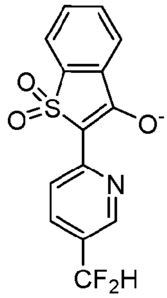 L27, 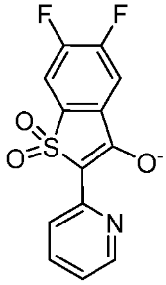 L28, 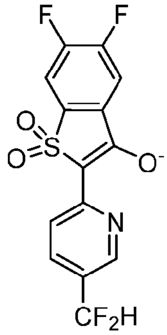 L29,

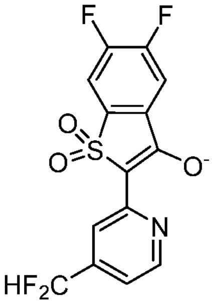 L30, 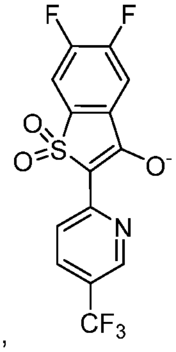 L31, 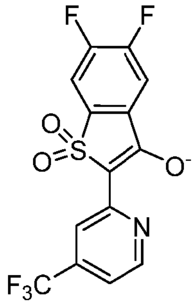 L32, 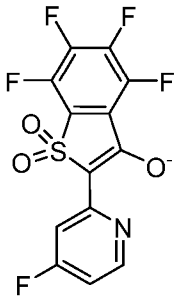 L33, 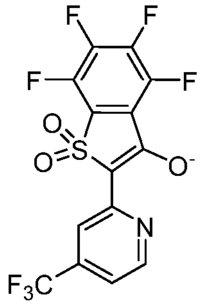 L34,

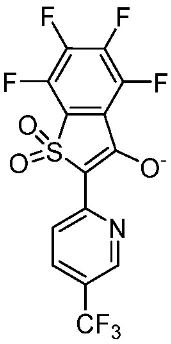 L35, 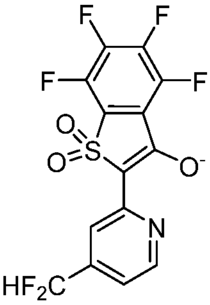 L36, 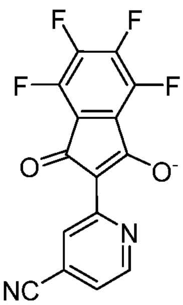 L37, 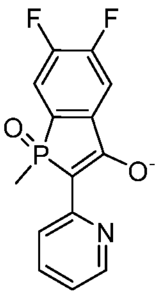 L38, 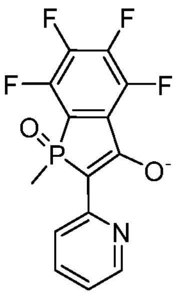 L39,

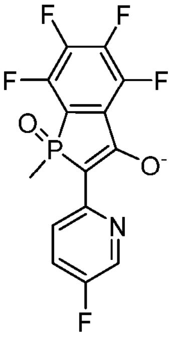 L40, 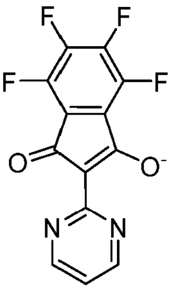 L41, 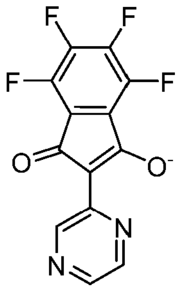 L42, 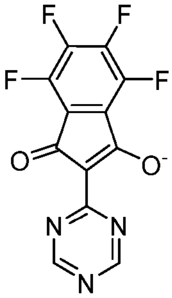 L43, 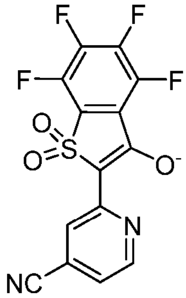 L44,

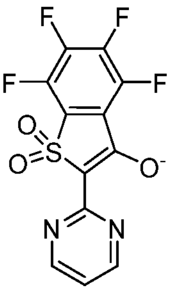 L45, 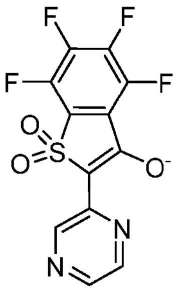 L46, 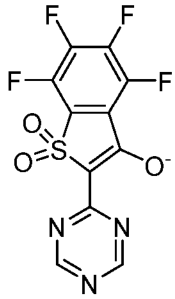 L47, 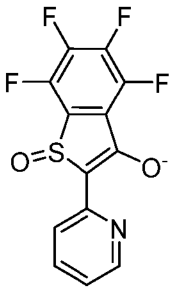 L48, 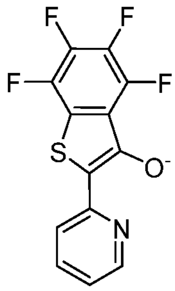 L49,

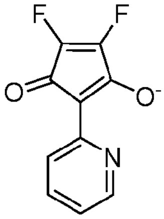 L50, 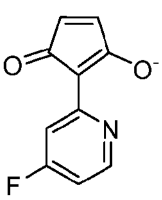 L51, 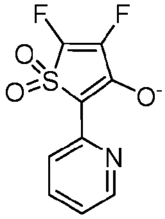 L52, 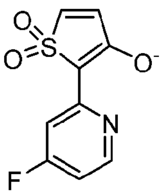 L53, 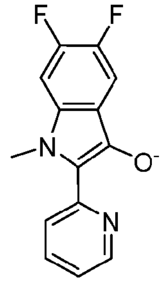 L54,

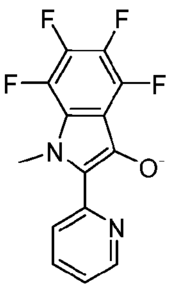 L55, 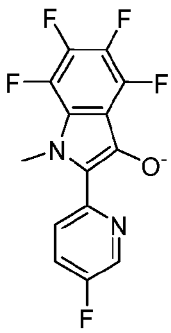 L56, 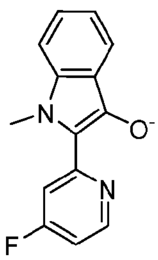 L57, 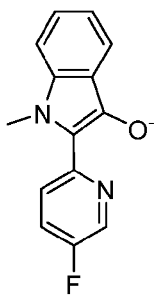 L58, 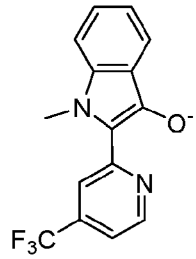 L59,

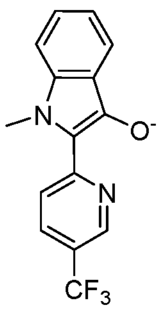 L60, 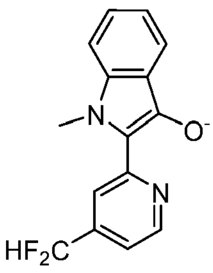 L61, 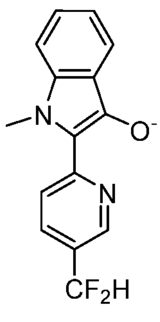 L62, 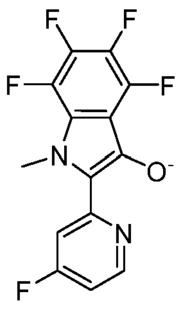 L63, 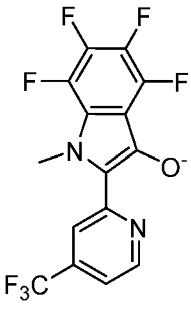 L64,

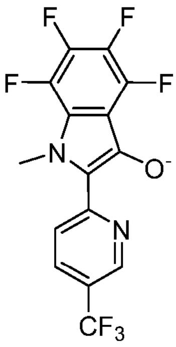 L65, 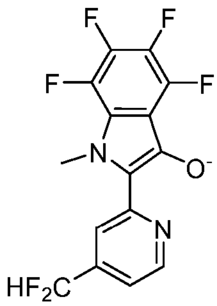 L66, 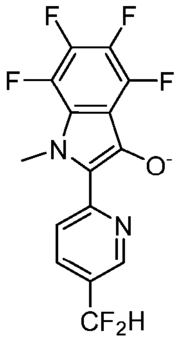 L67, 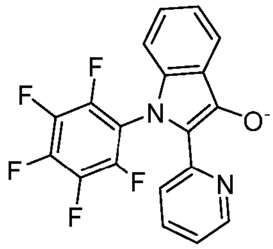 L68,

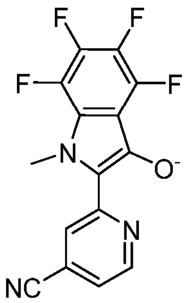 L69, 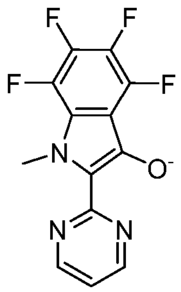 L70, 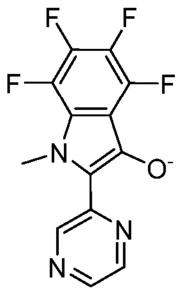 L71, 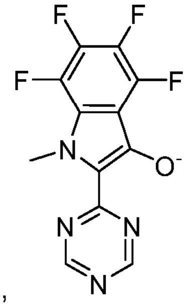 L72, 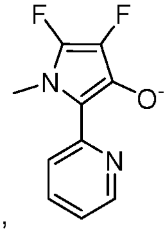 L73,

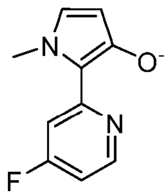 L74, 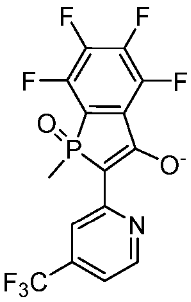 L75, 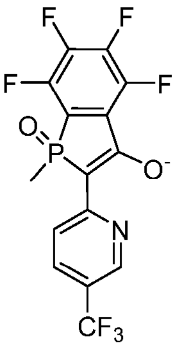 L76, 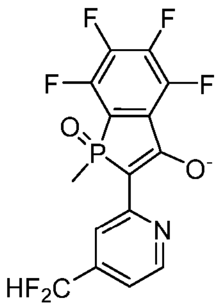 L77, 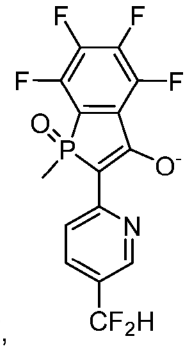 L78,

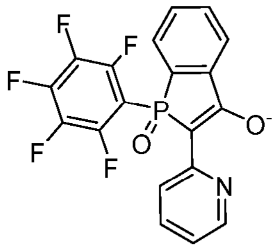 L79, 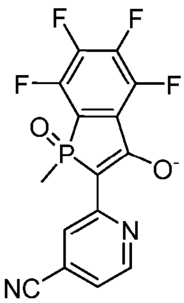 L80, 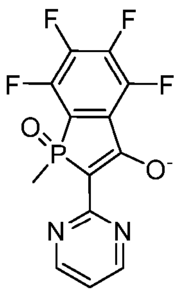 L81, 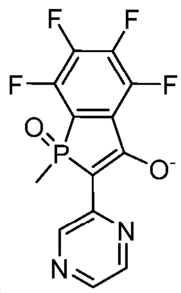 L82, 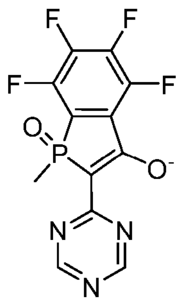

L83, 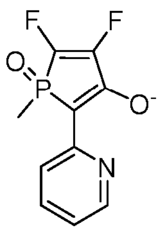 L84 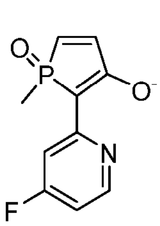 L85, 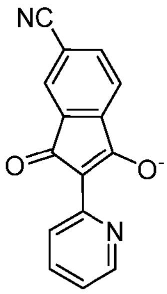 L86, 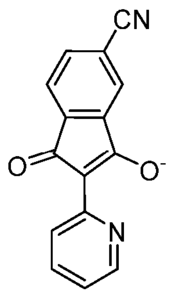 L87, 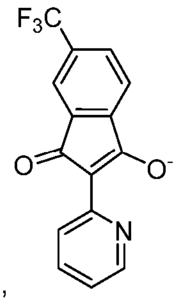 L88,

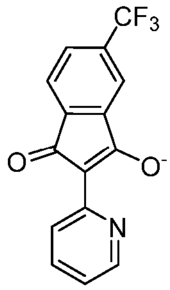 L89, 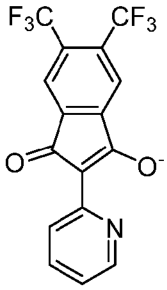 L90, 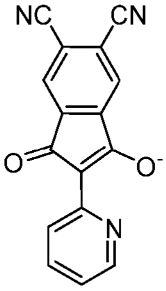 L91, 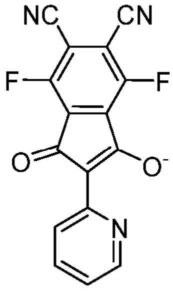 L92, 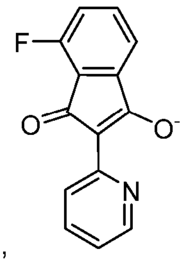 L93,

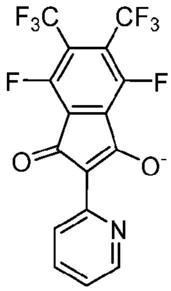 L94, 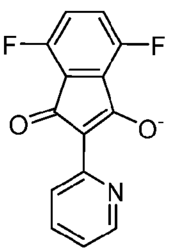 L95, 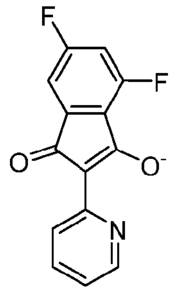 L96, 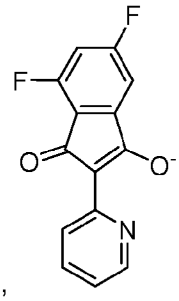 L97, 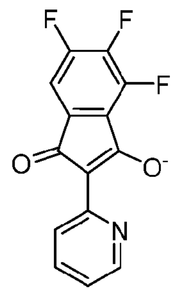 L98,

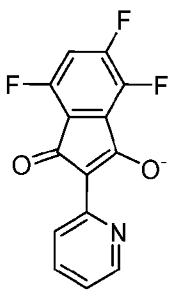 L99, 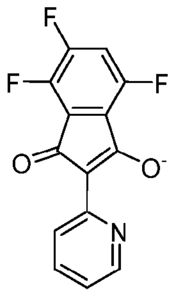 L100, 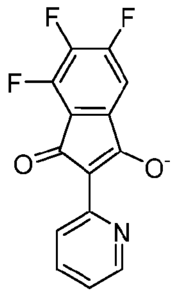 L101, 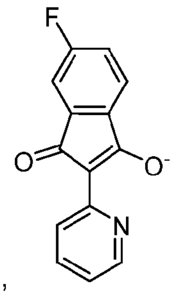 L102, 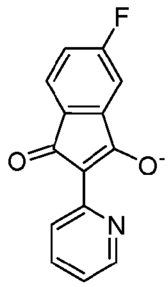 L103,

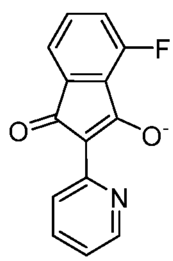 L104, 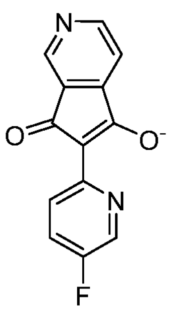 L105, 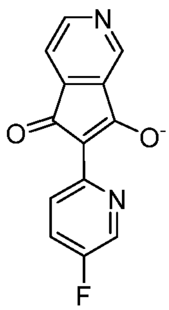 L106, 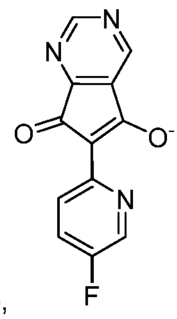 L107, 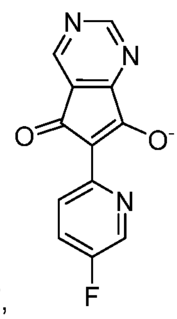 L108,

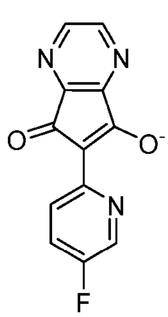 L109, 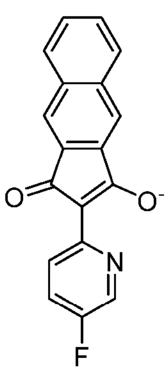 L110, 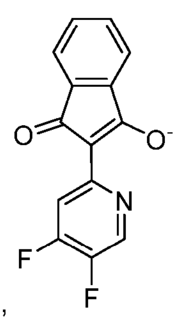 L111, 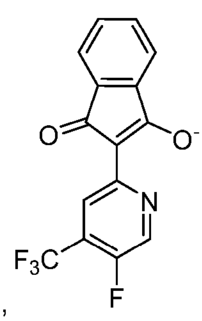 L112, 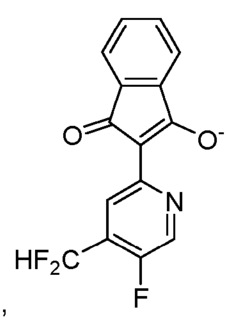

L113, 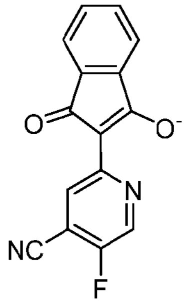 L114, 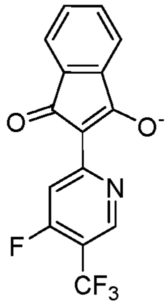 L115, 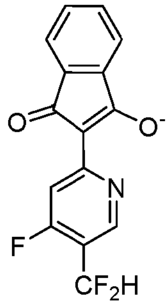 L116, 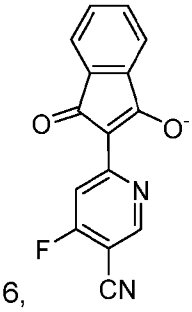 L117, 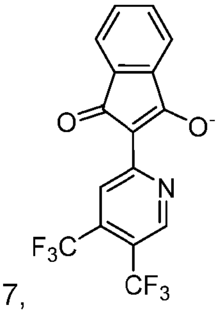

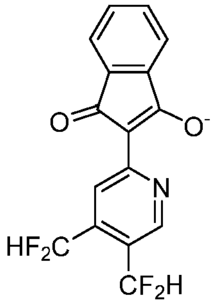 L118, 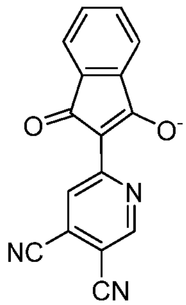 L119, L120,

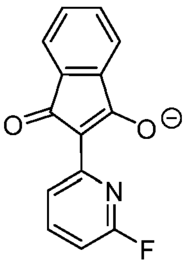 L121, 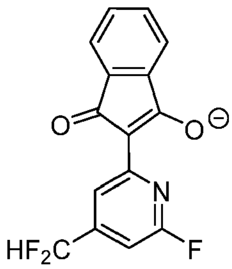 L122, 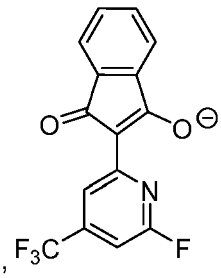 L123, 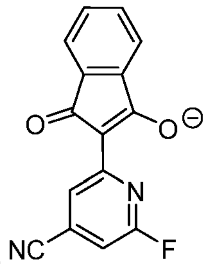 L124,

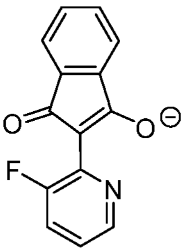 L125, 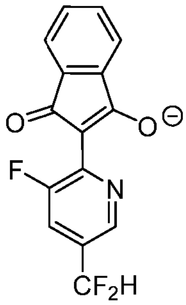 L126, 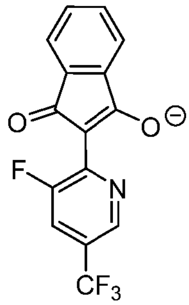 L127, 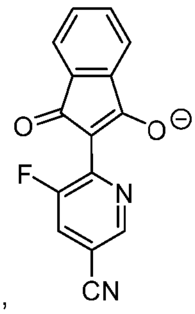 L128; 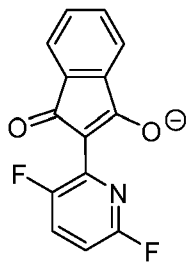

L129, 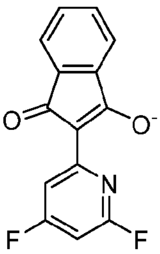 L130, 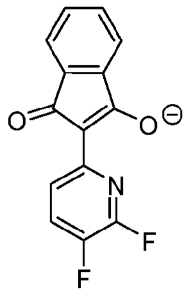 L131, 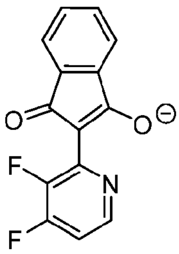 L132, 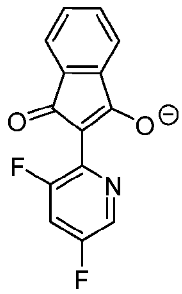 L133,

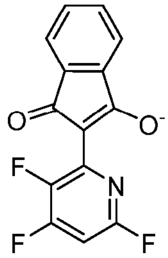 L134, 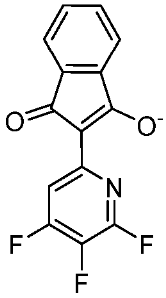 L135, 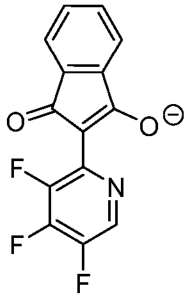 L136, 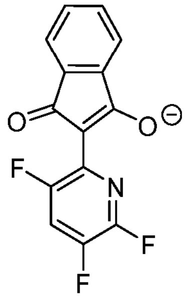 L137,

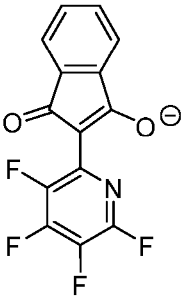 L138, 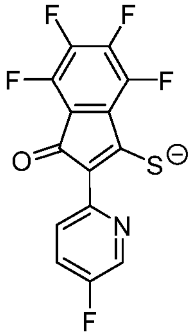 L139, 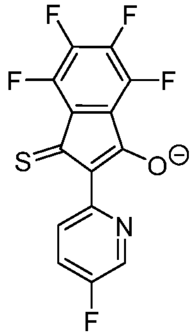 L140, 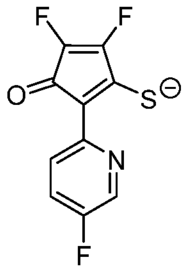 L141,

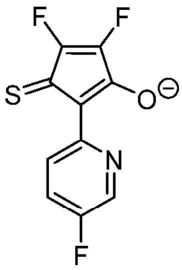 L142, 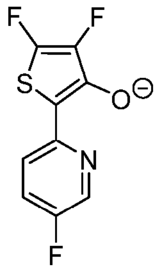 L143, 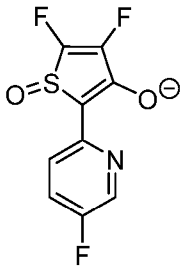 L144.

According to one embodiment, L is selected from L1 to L49, more preferred L1 to L40 and most preferred from L1 to L20.

According to an embodiment, the compound of formula (I) is selected from M1 to M252:

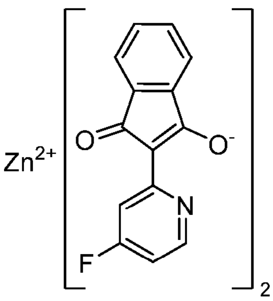 M1, 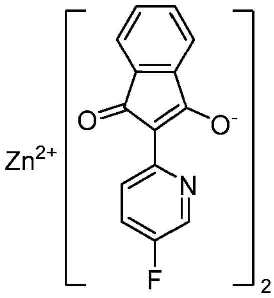 M2, 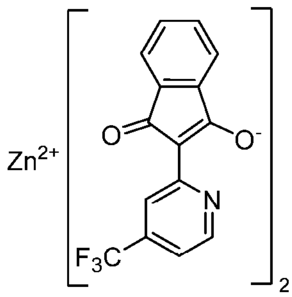 M3,

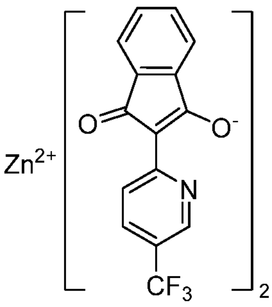 M4, 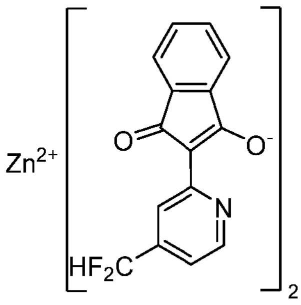 M5, 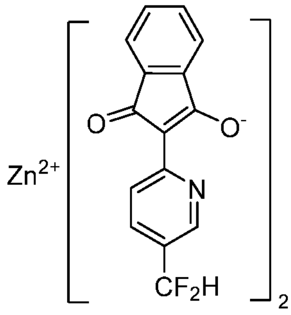 M6,

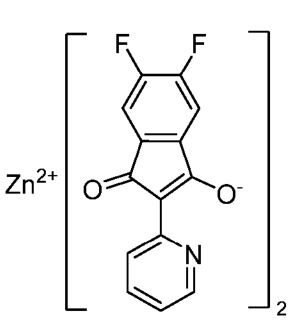 M7, 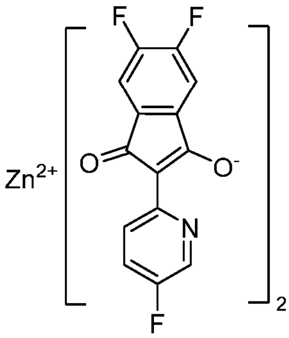 M8, 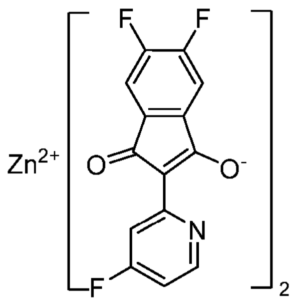 M9,

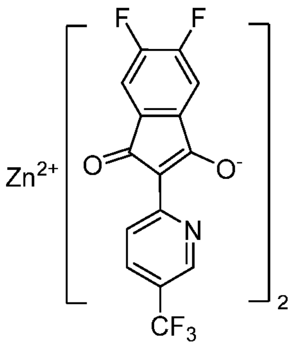 M10, 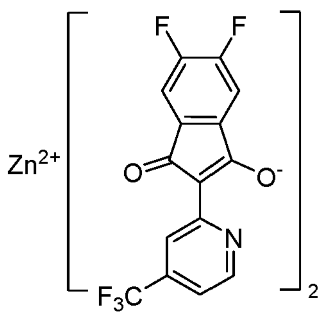 M11, 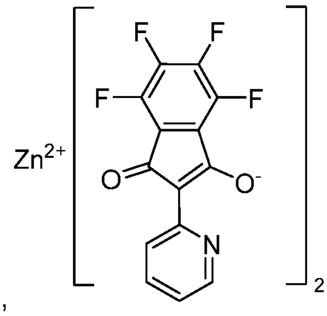 M12,

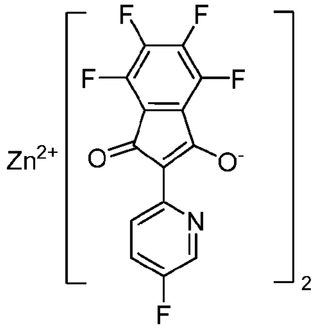 M13, 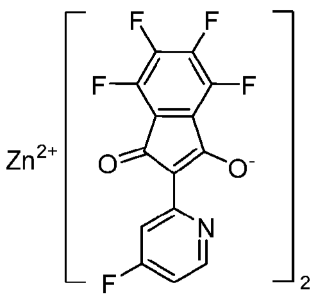 M14, 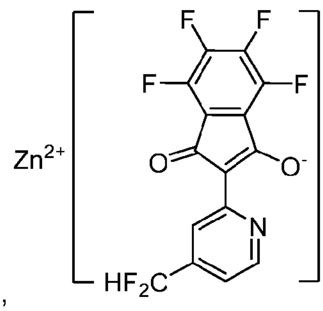 M15,

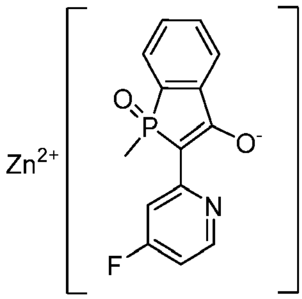 2 M16, 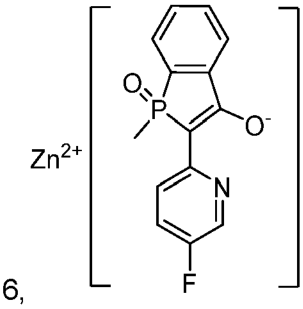 2 M17, 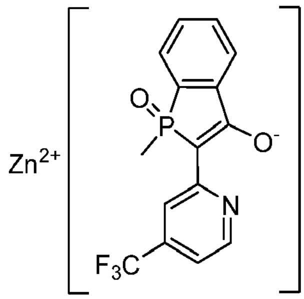 2 M18,

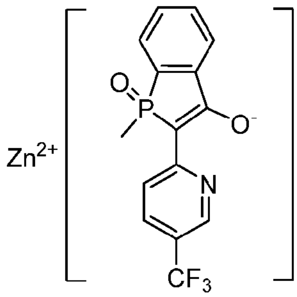 2 M19, 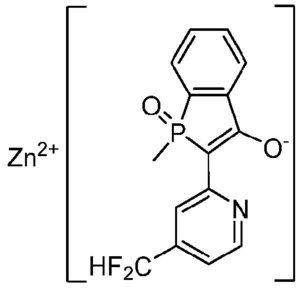 2 M20, 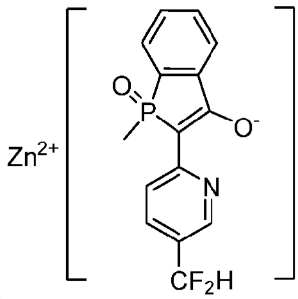 2 M21,

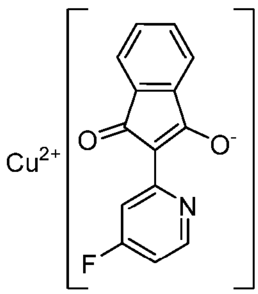 2 M22, 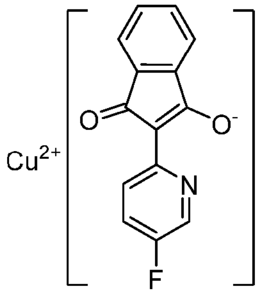 2 M23, 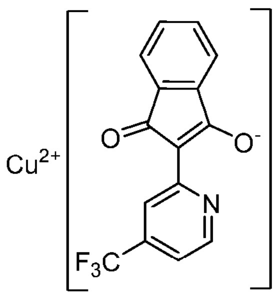 2 M24,

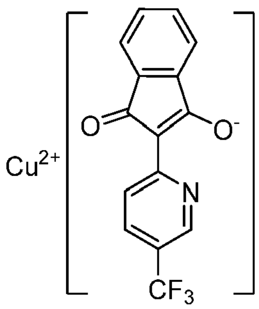 2 M25, 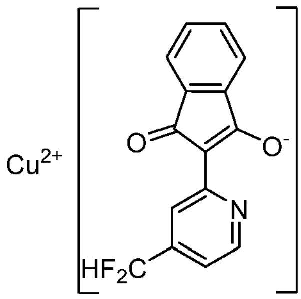 2 M26, 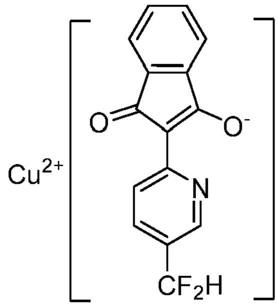 2 M27,

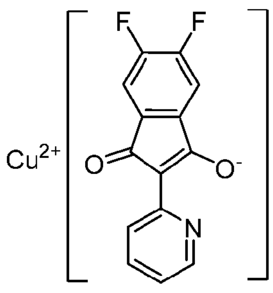 2 M28, 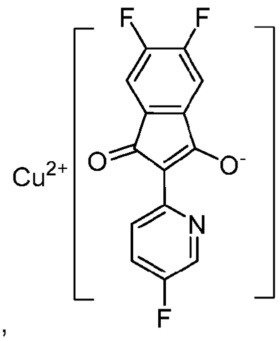 2 M29, 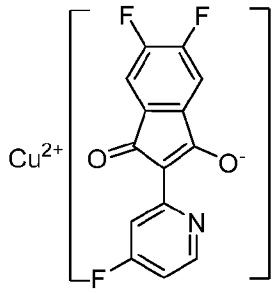 2 M30,

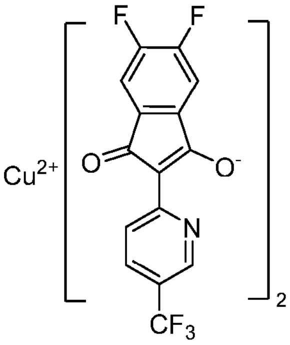 M31, 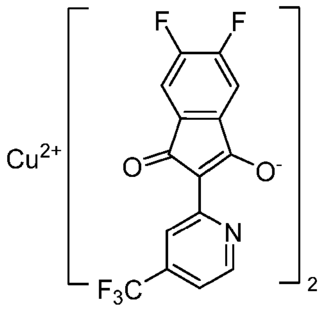 M32, 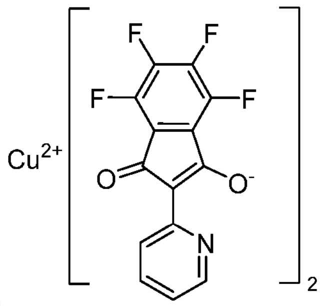 M33,

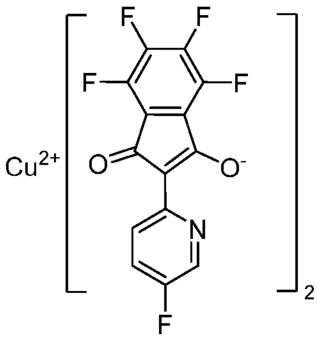 M34, 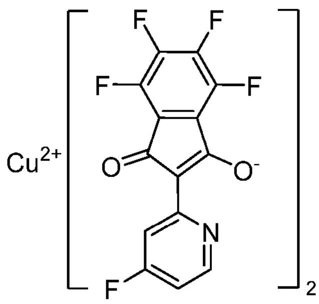 M35, 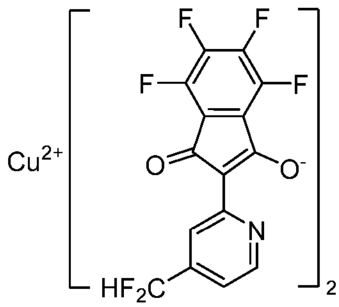 M36,

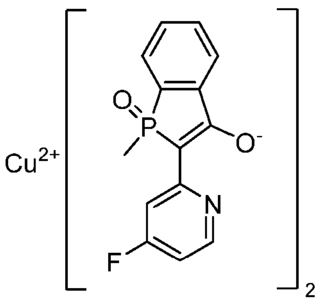 M37 , 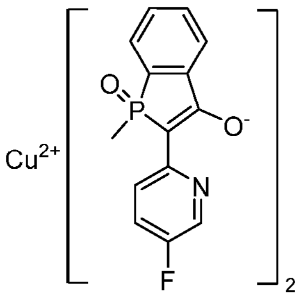 M38, 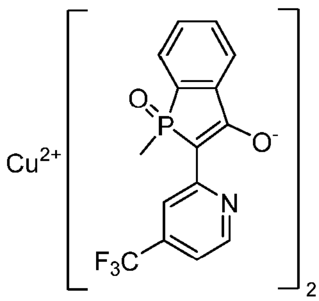 M39,

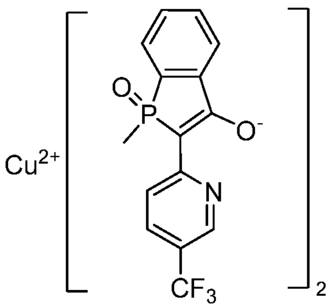 M40, 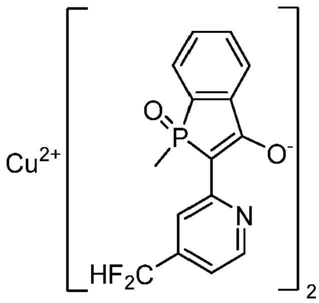 M41, 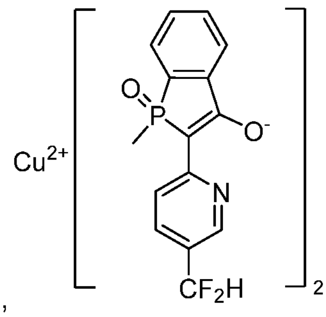 M42,

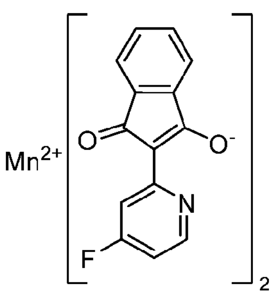 M43, 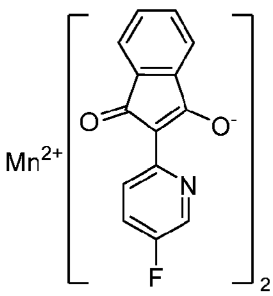 M44, 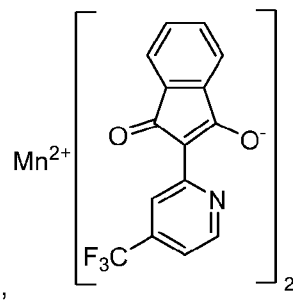 M45,

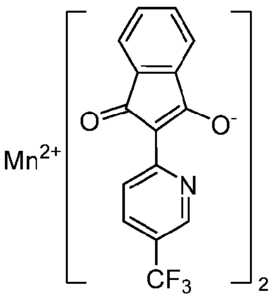 M46, 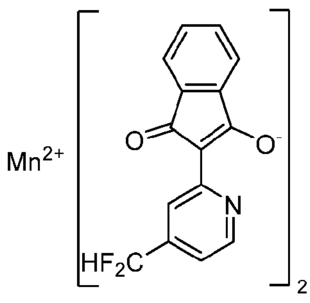 M47, 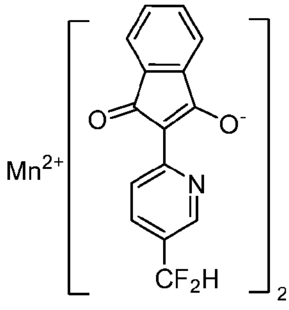 M48,

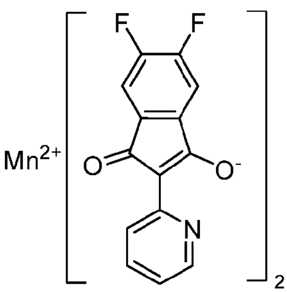 M49, 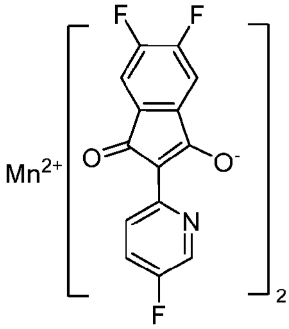 M50, 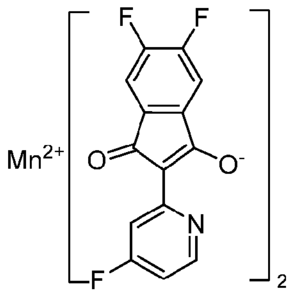 M51,

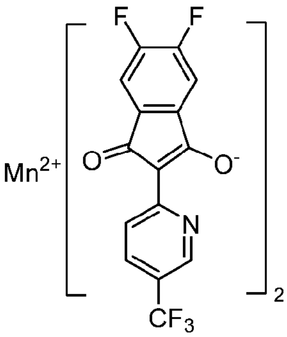 M52, 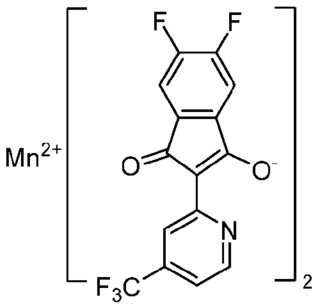 M53, 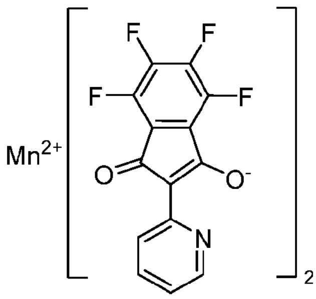 M54,

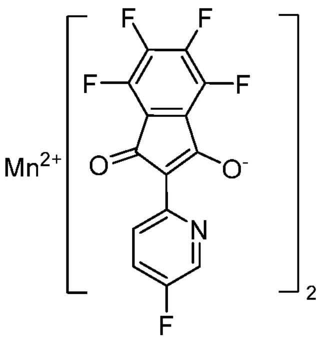 M55, 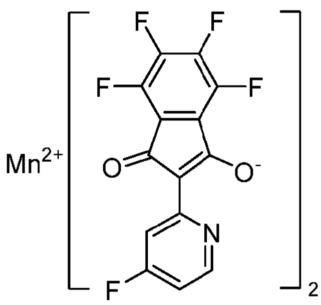 M56, 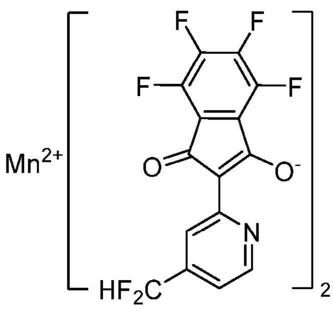 M57,

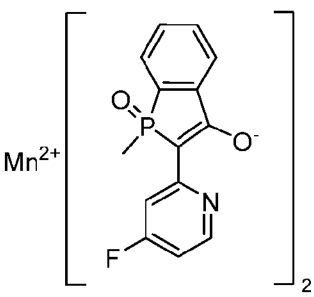 M58, 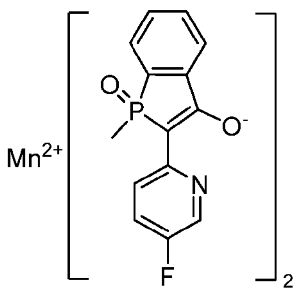 M59, 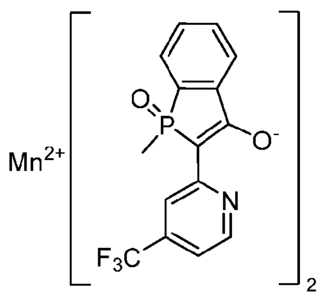 M60,

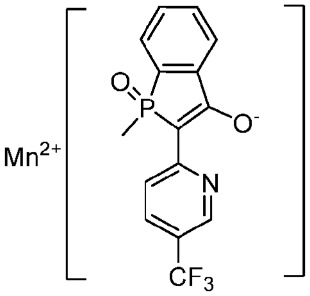 M61,
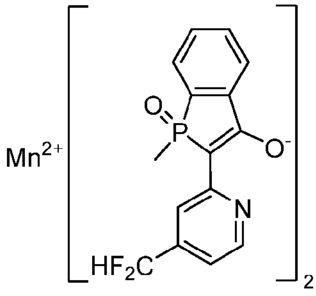 M62,
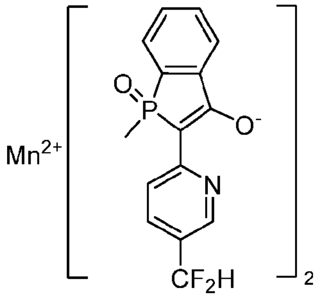 M63,

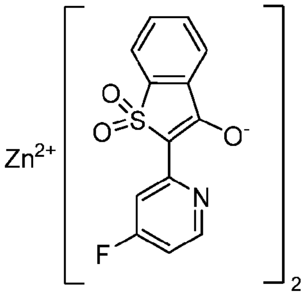 M64,
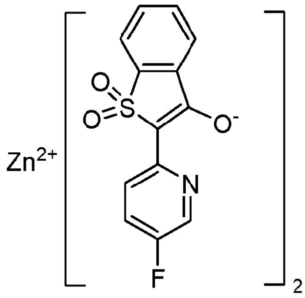 M65,
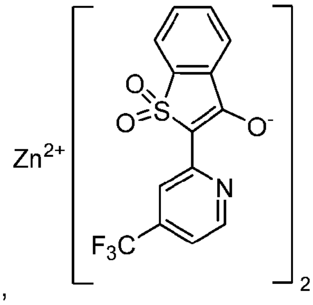 M66,

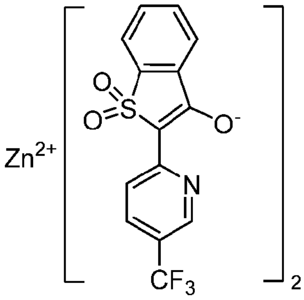 M67,
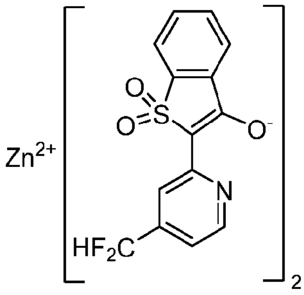 M68,
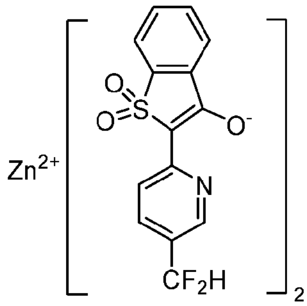 M69,

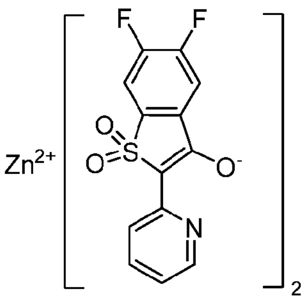 M70,
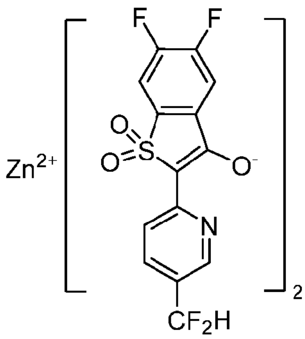 M71,
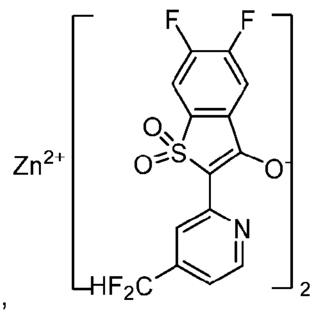 M72,

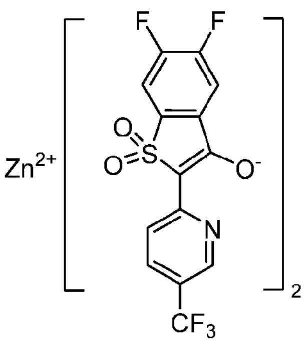 M73,
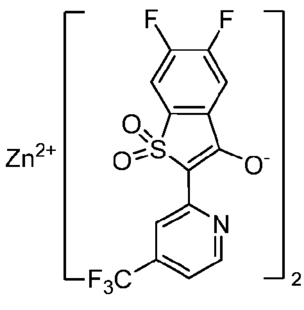 M74,
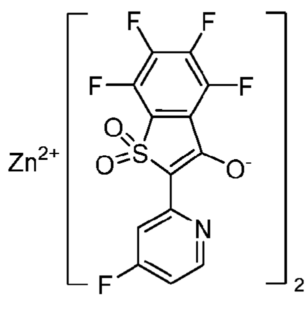 M75,

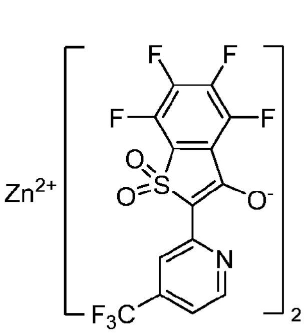
M76,
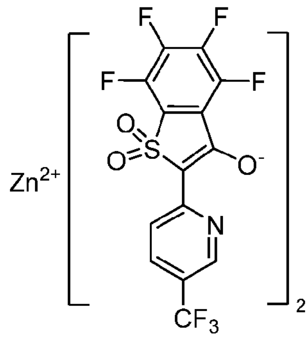
M77,
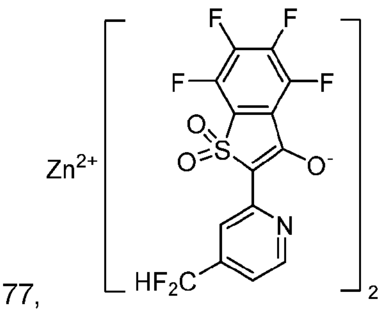
M78,
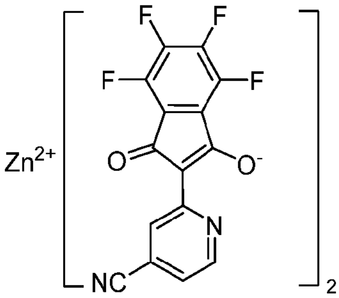
M79,
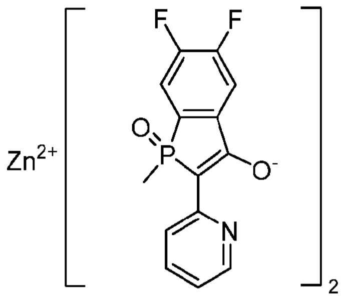
80,
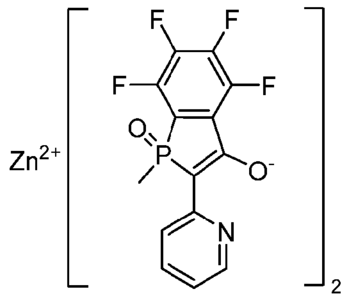
M81,
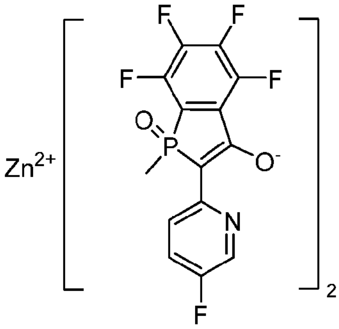
M82,
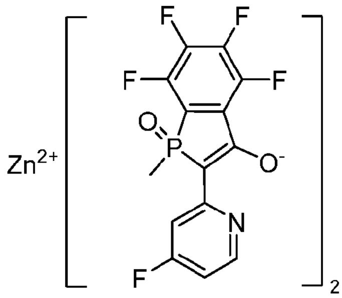
M83,
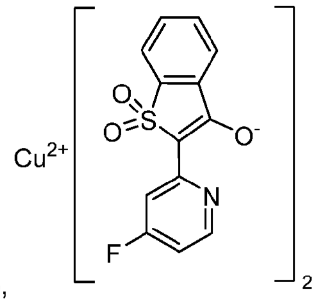
M84,
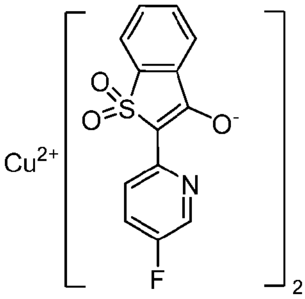
M85,
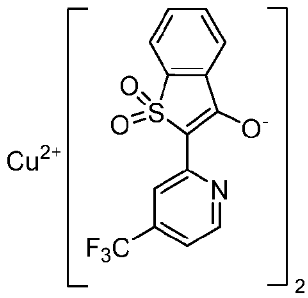
M86,
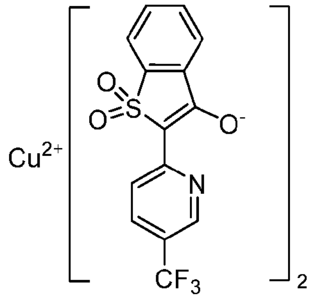
M87,
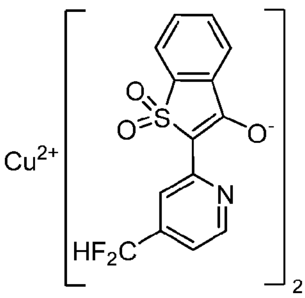
M88,
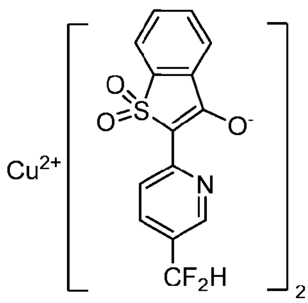
M89,
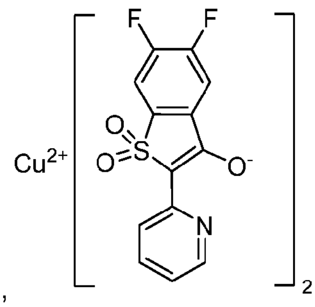
M90,

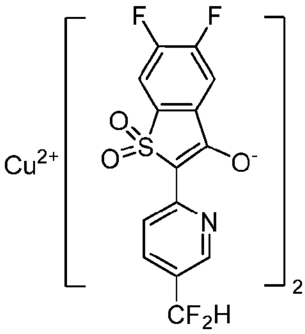 M91, 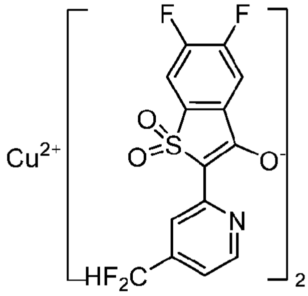 M92, 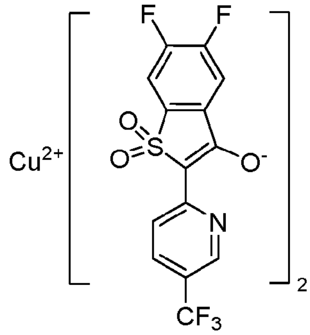 M93,

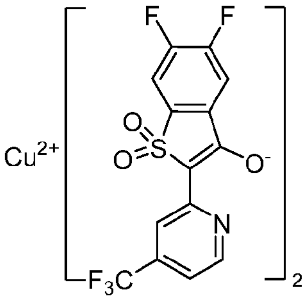 M94, 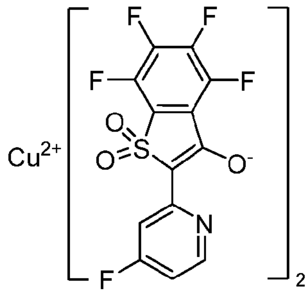 M95, 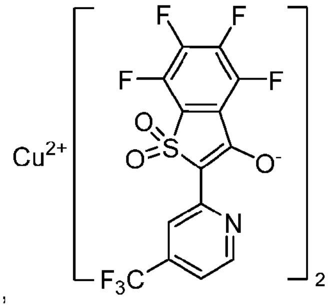 M96,

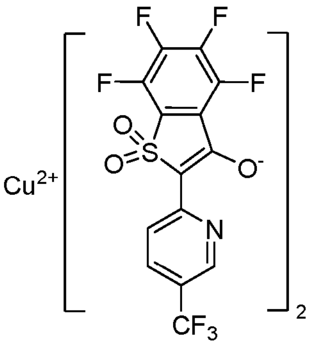 M97, 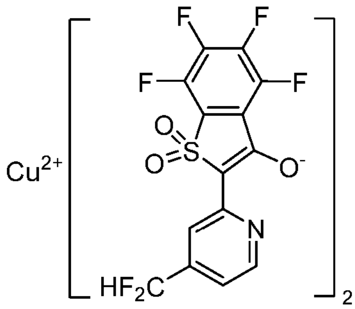 M98, 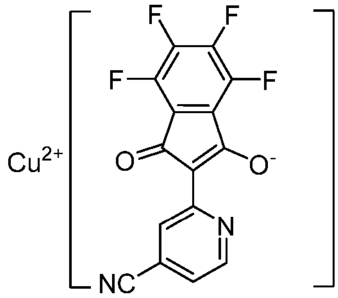 M99,

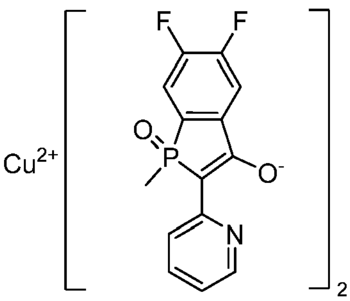 M100, 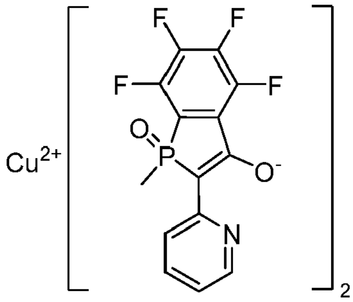 M101, 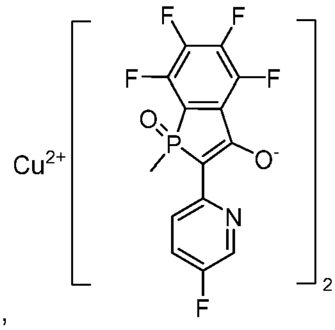

M102, 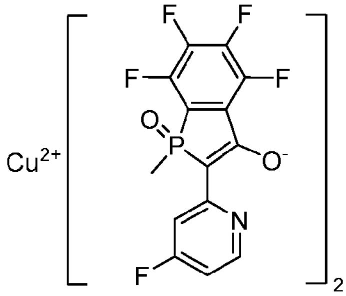 M103, 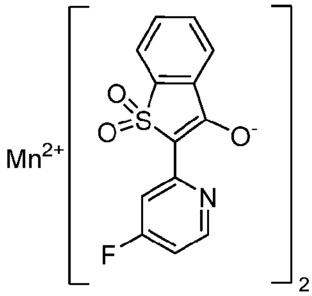 M104, 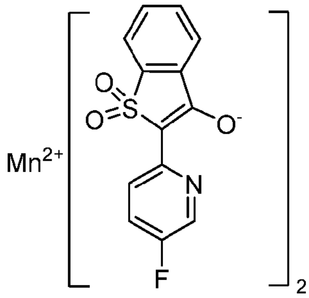

M105, 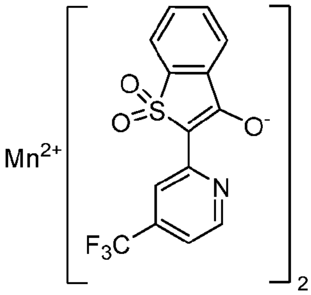 M106, 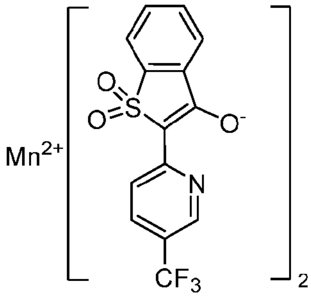 M107, 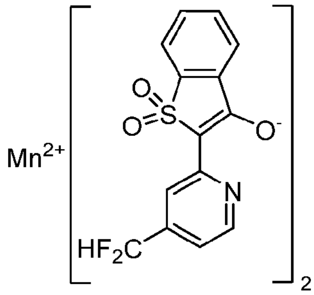

M108, 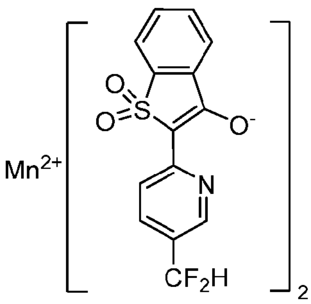 M109, 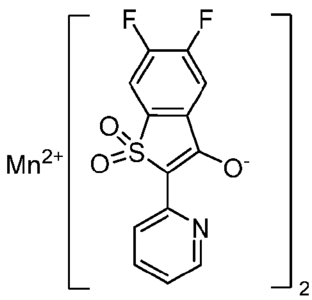 M110, 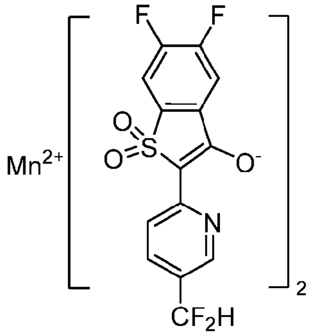

M111, 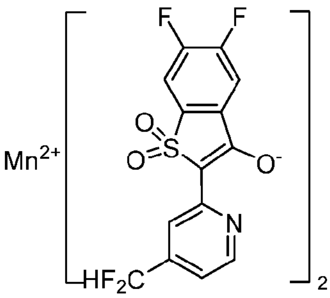 M112, 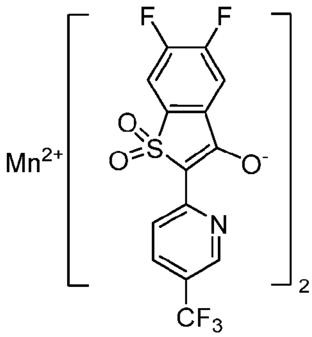 M113, 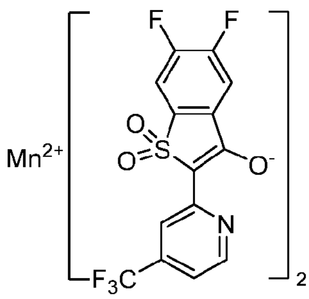

M114, 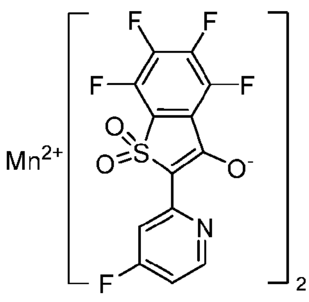 M115, 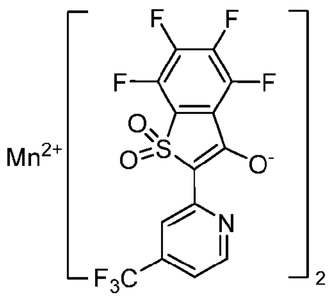 M116, 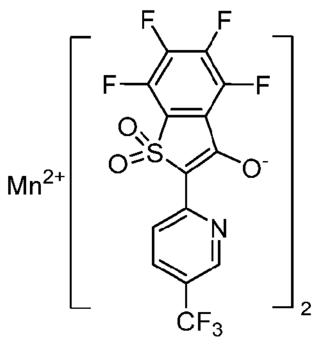

M117, 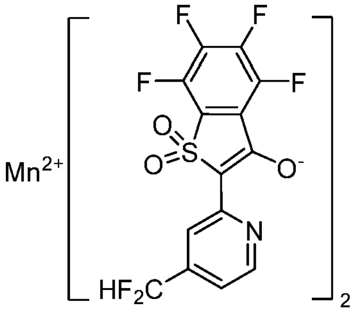 M118, 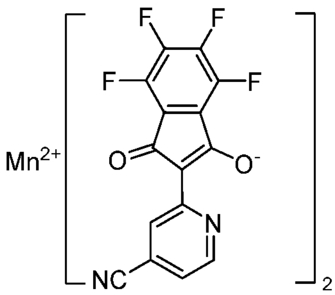 M119,

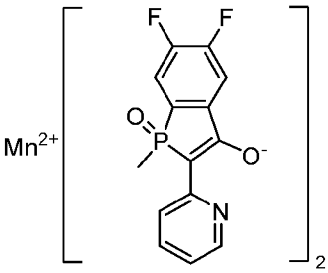 M120, 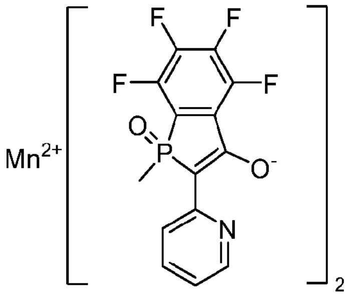 M121, 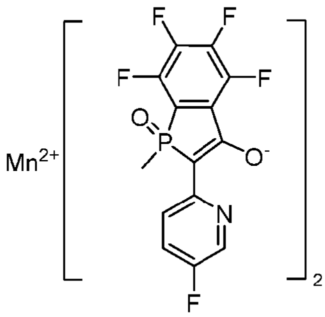

M122, 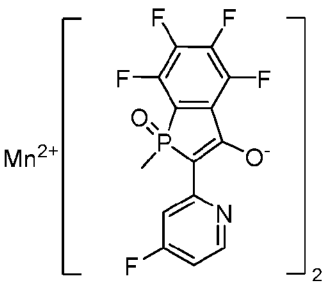 M123, 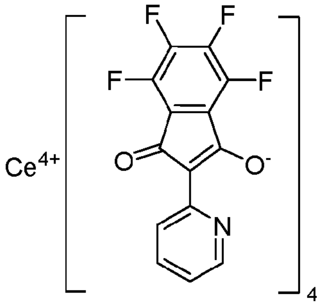 M124, 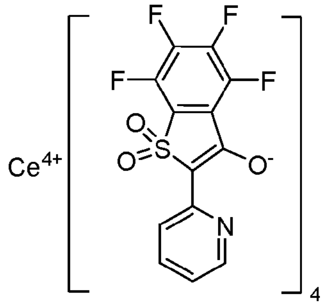

M125, 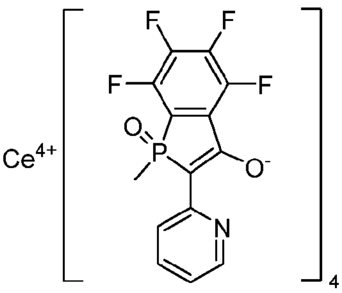 M126, 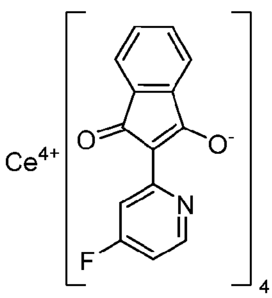 M127, 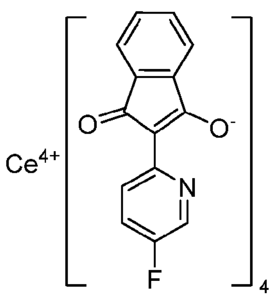 M128,

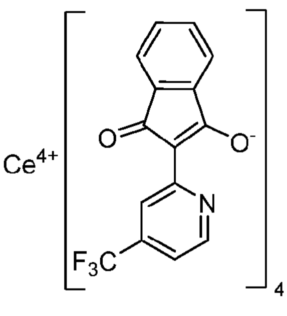 M129, 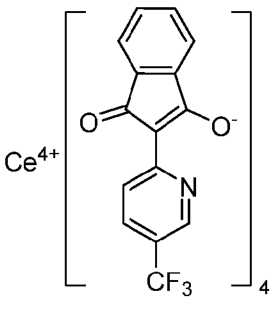 M130, 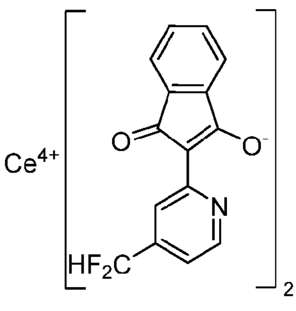 M131,

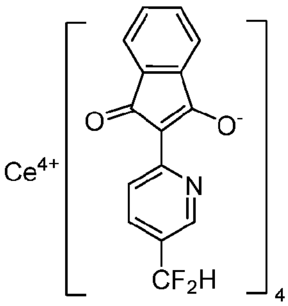 M132,

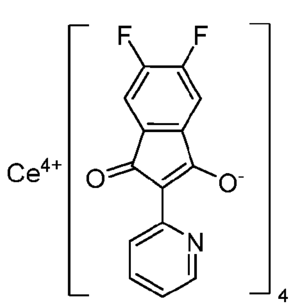 M133,

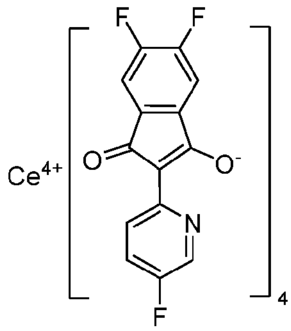 M134,

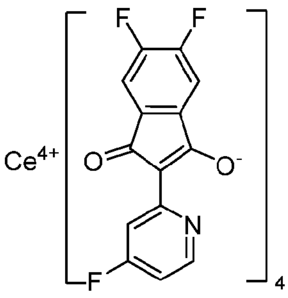 M135,

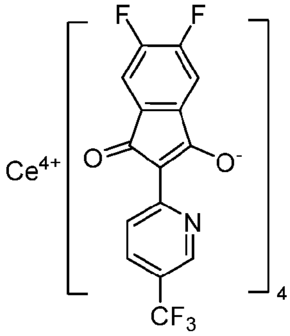 M136,

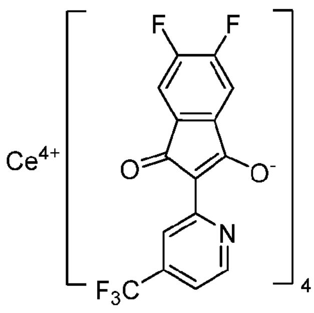 M137,

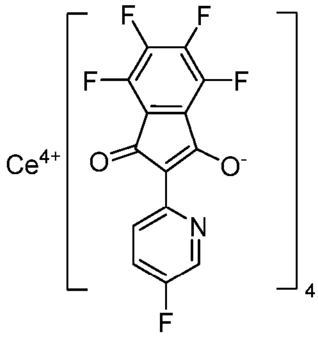 M138,

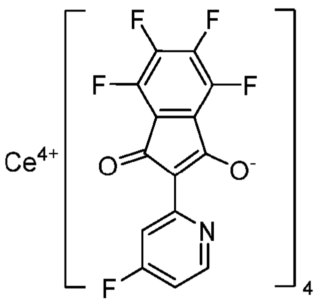 M139,

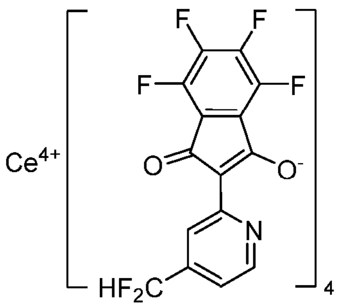 M140,

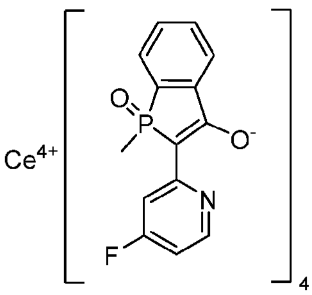 M141,

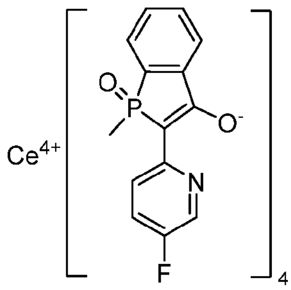 M142,

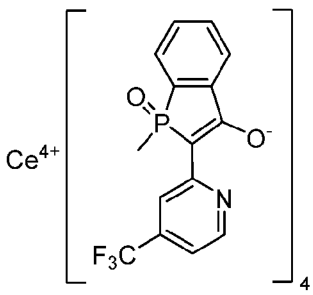 M143,

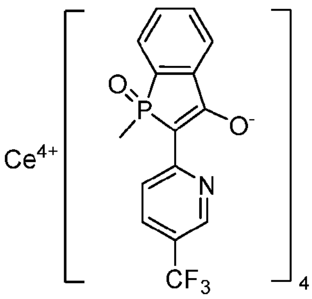 M144,

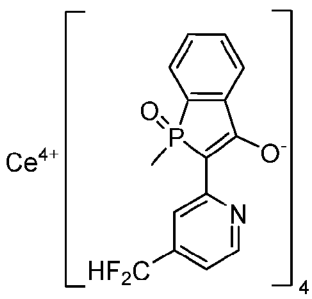 M145,

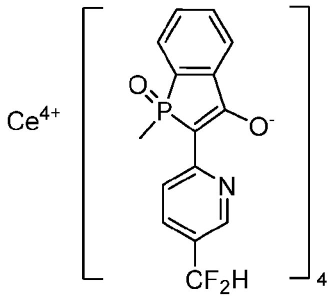 M146,

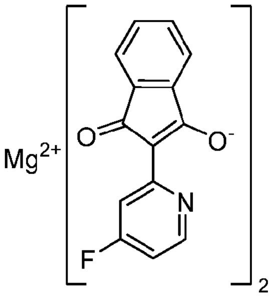 M147, 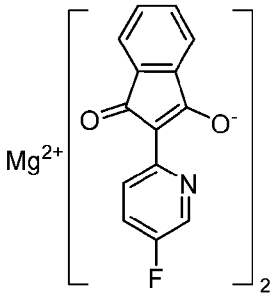 M148, 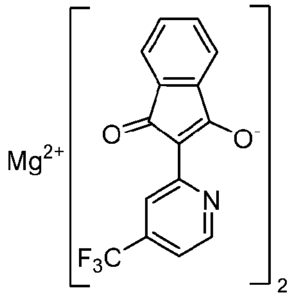 M149,

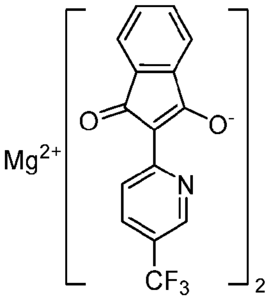 M150, 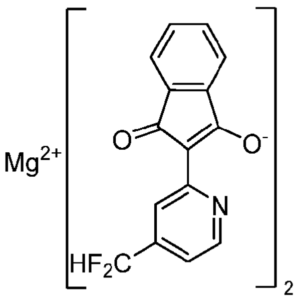 M151, 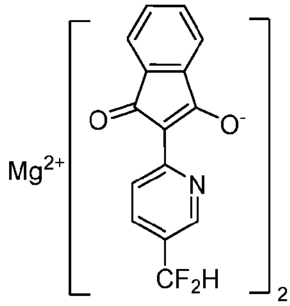 M152,

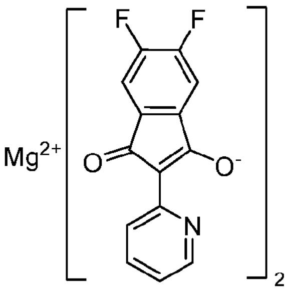 M153, 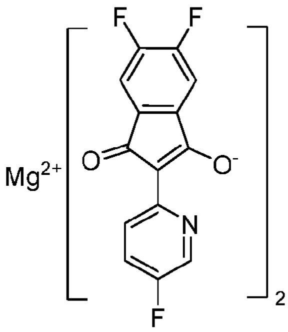 M154, 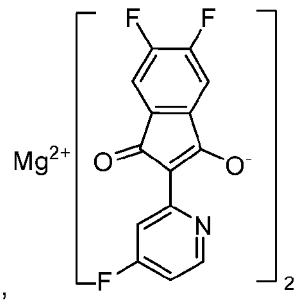 M155,

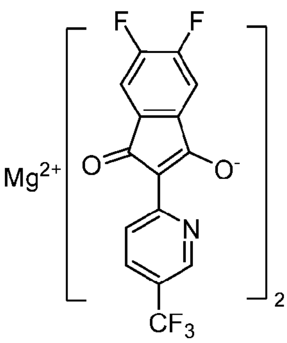 M156, 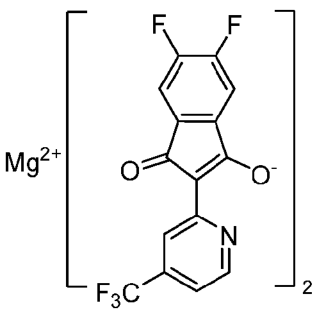 M157, 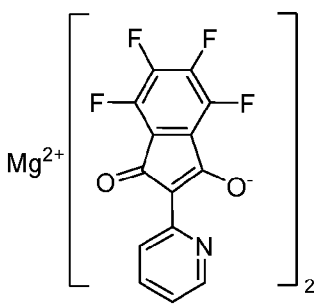 M158,

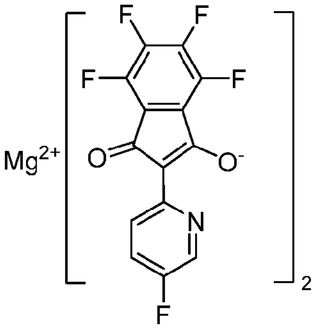 M159, 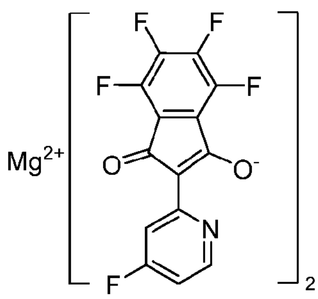 M160, 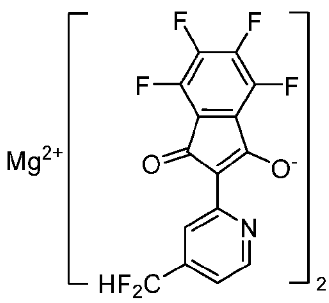 M161,

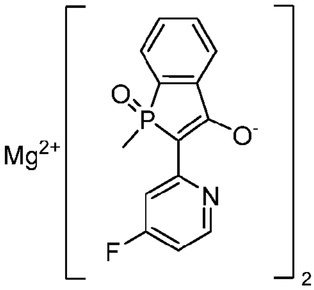 M162,

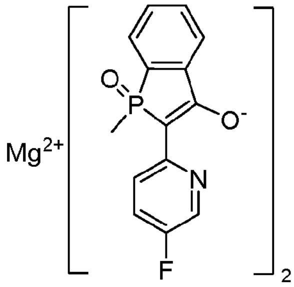 M163,

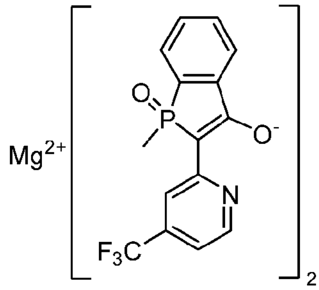 M164,

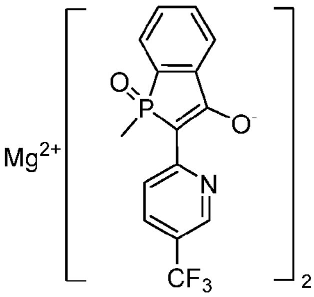 M165,

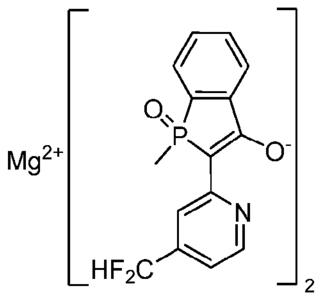 M166,

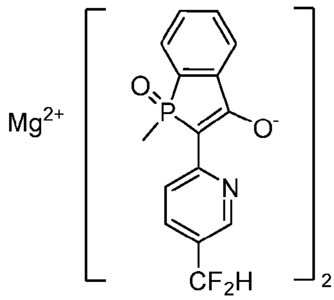 M167,

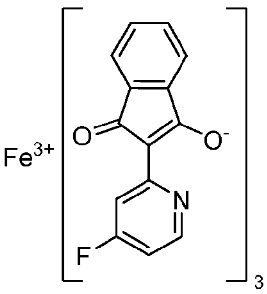 M168,

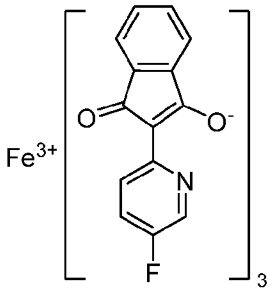 M169,

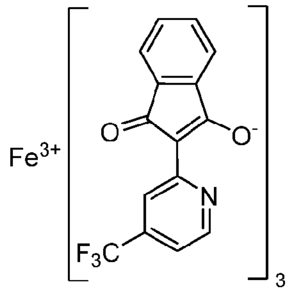 M170,

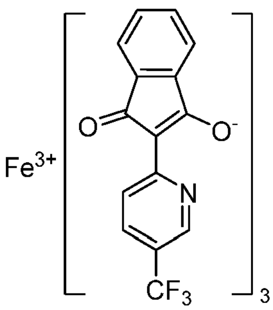 M171,

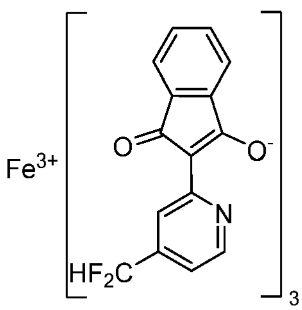 M172,

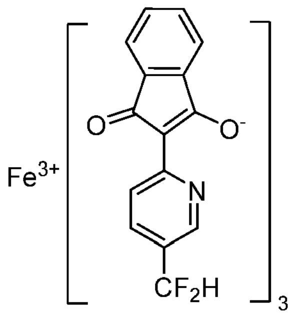 M173,

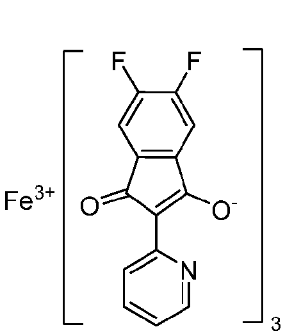 M174,

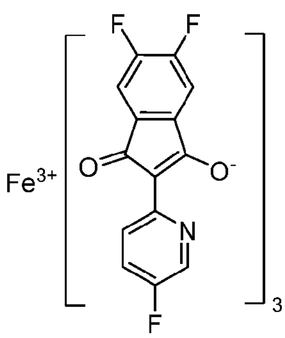 M175,

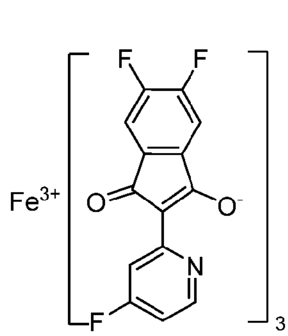 M176,

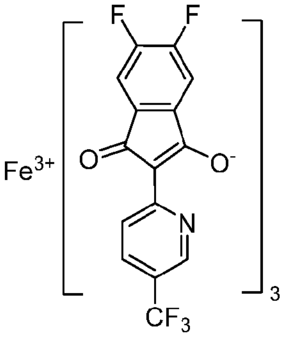 M177, 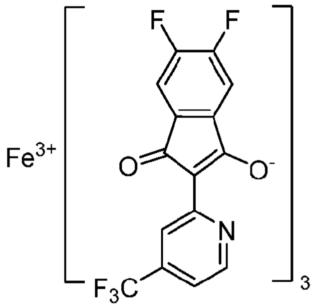 M178, 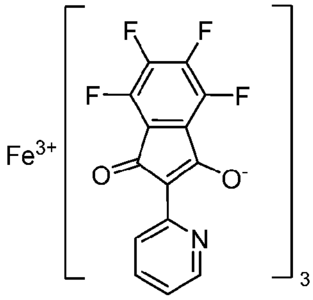 M179,

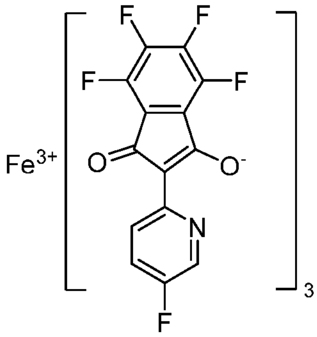 M180, 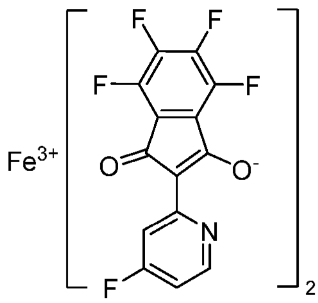 M181, 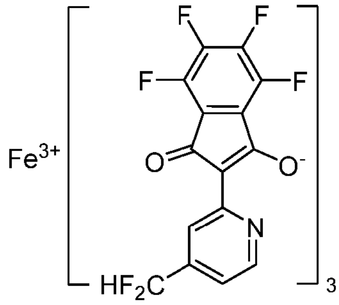 M182,

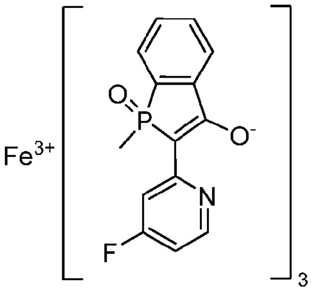 M183, 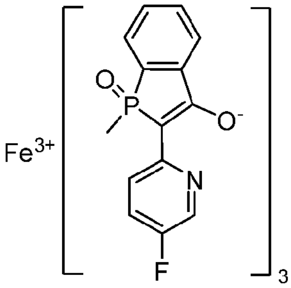 M184, 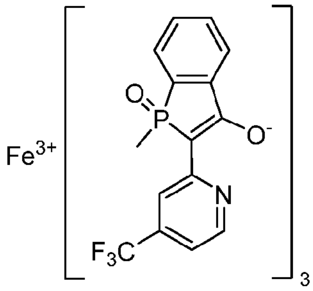 M185,

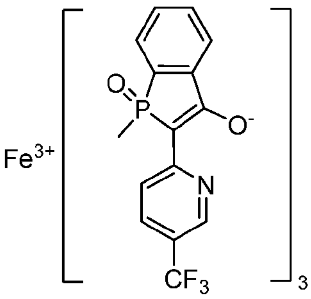 M186, 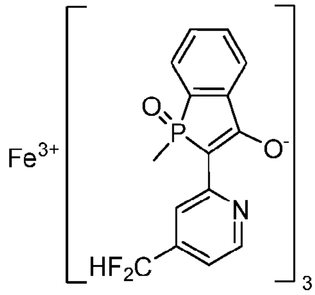 M187, 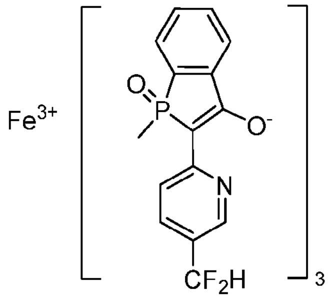 M188,

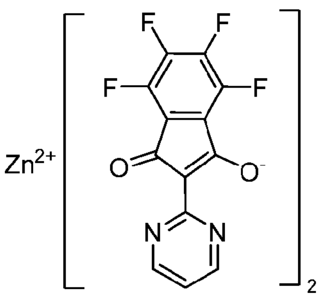 M189, 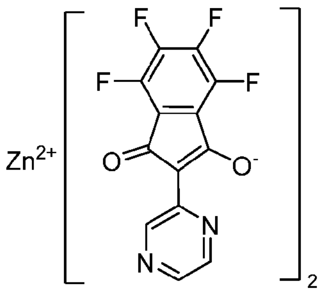 M190, 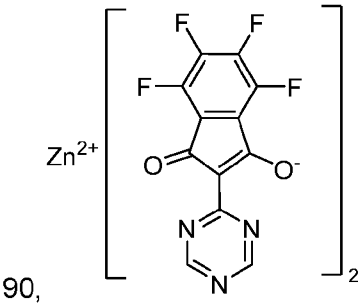 M191,

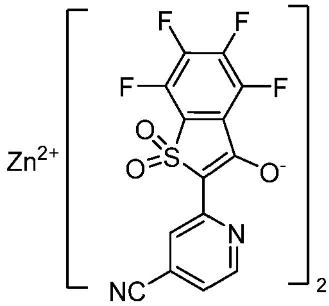 M192,

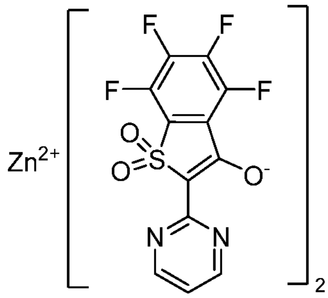 M193, 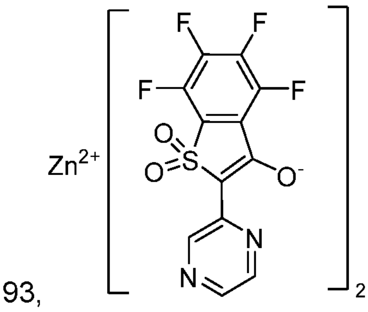 M194, 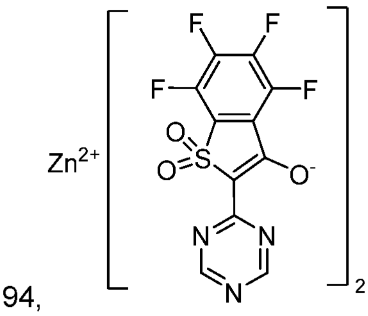 M195,

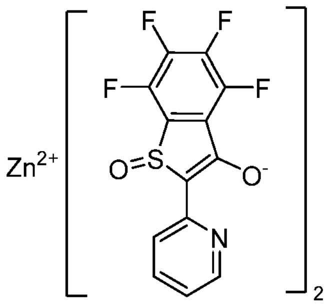 M196, 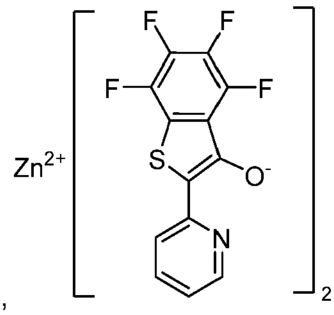 M197, 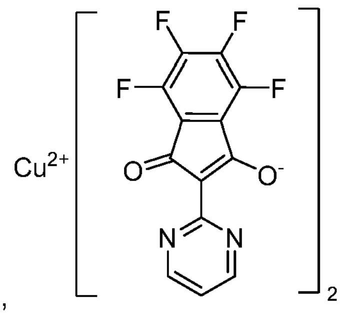 M198,

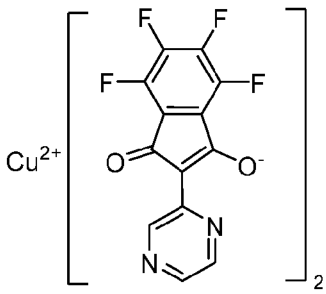 M199, 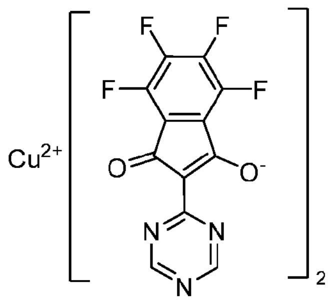 M200, 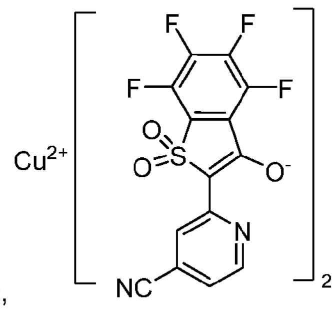 M201,

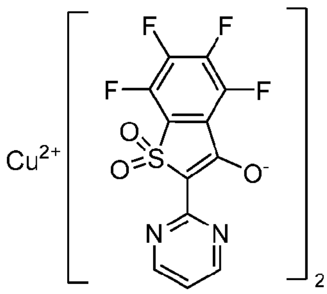 M202, 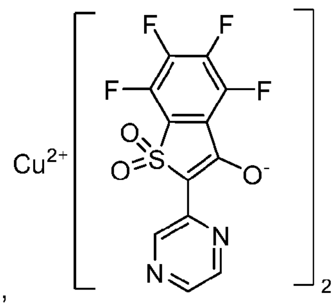 M203, 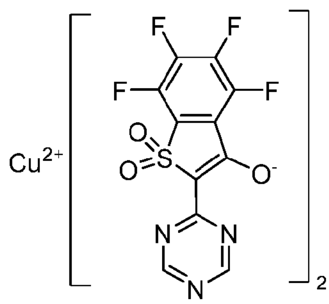 M204,

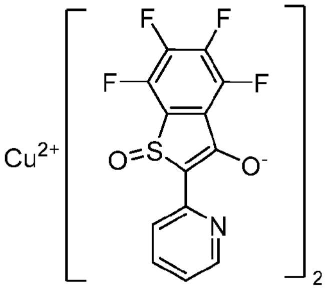 M205,

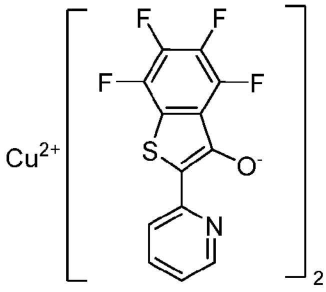 M206,

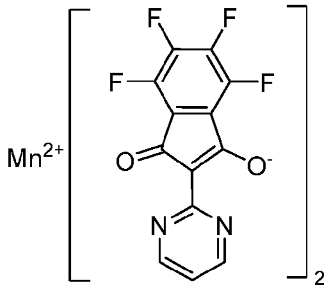 M207,

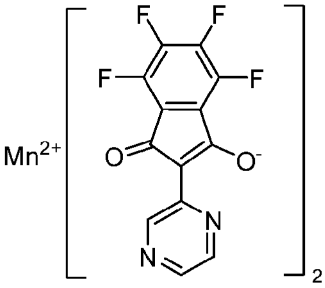 M208,

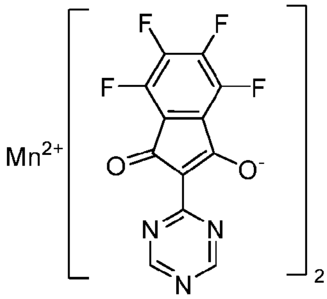 M209,

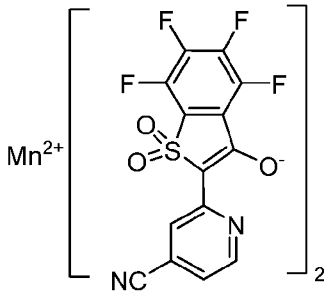 M210,

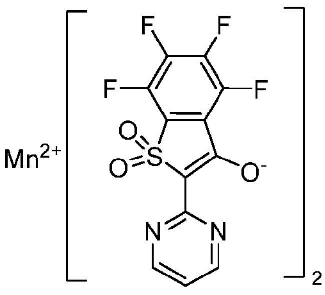 M211,

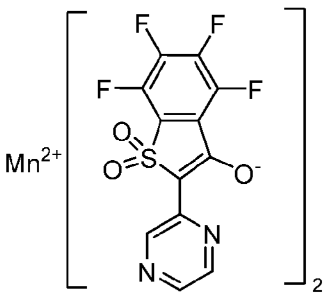 M212,

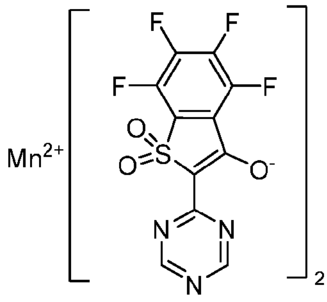 M213,

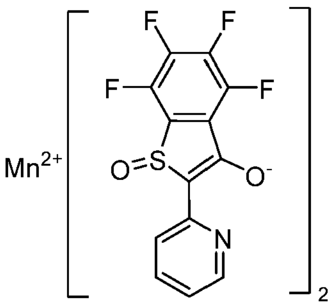 M214,

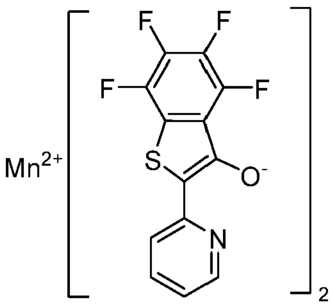 M215,

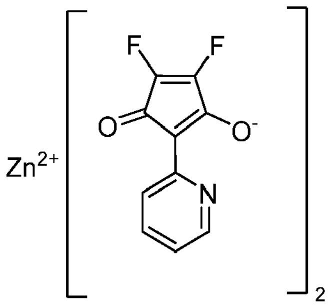 M216,

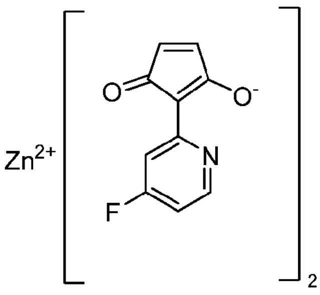 M217,

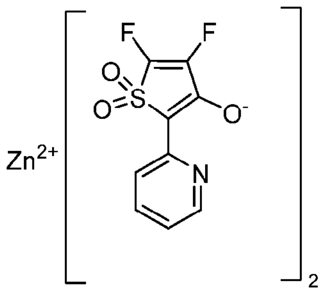 M218,

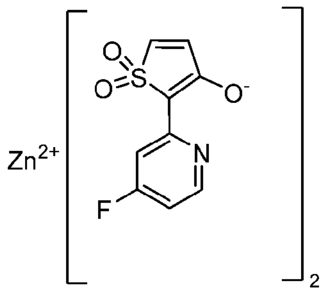 M219,

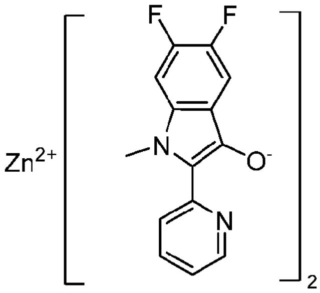 M220, 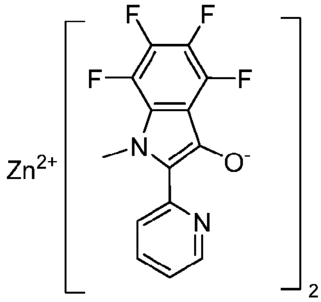 M221, 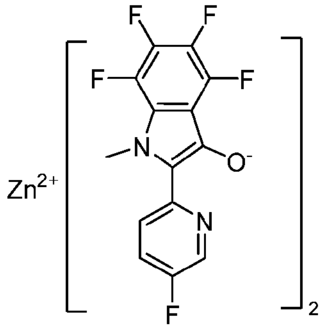 M222,

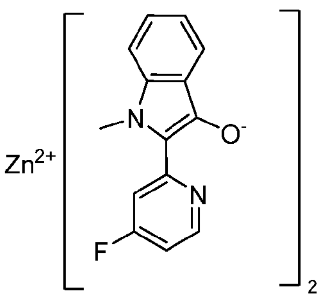 M223, 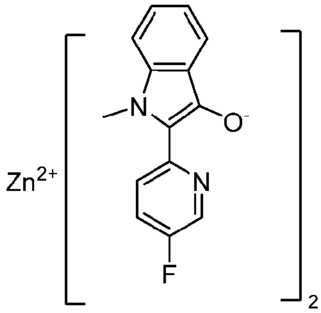 M224, 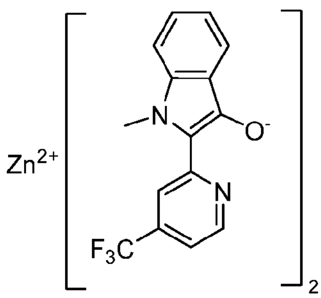 M225,

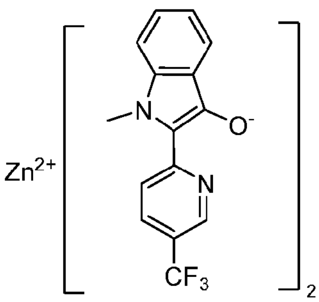 M226, 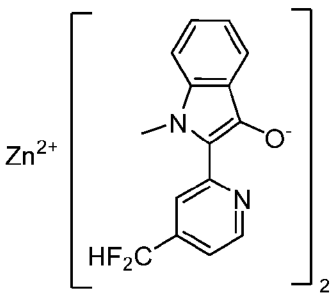 M227, 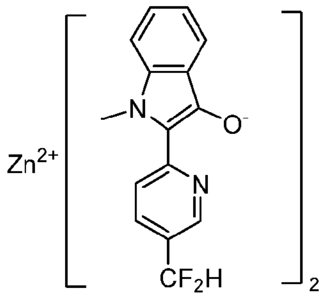 M228,

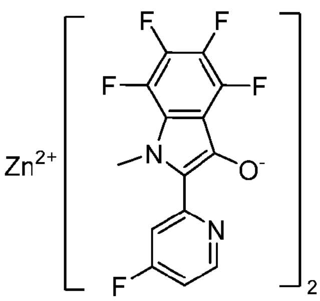 M229, 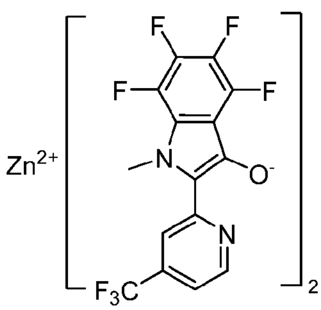 M230, 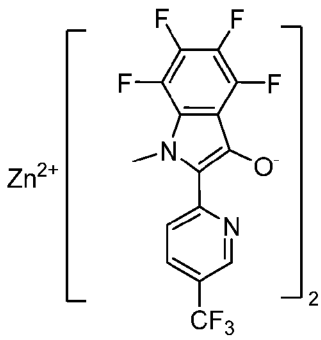 M231,

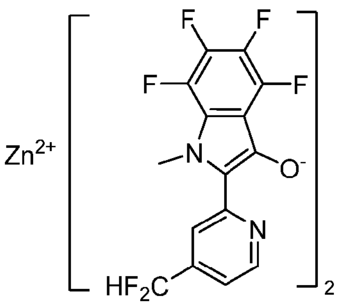 M232, 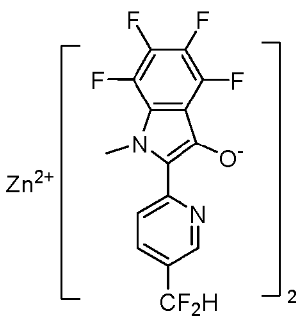 M233, 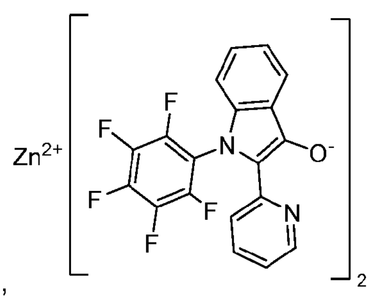 M234,

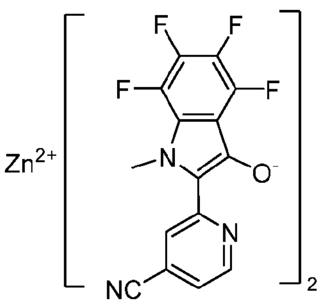 M235, 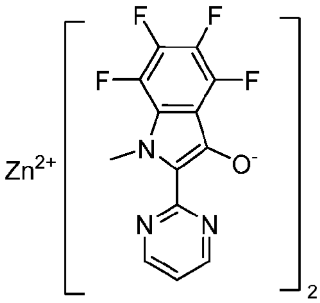 M236, 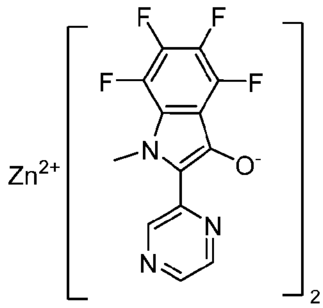 M237,

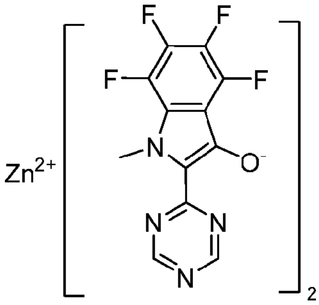 M238, 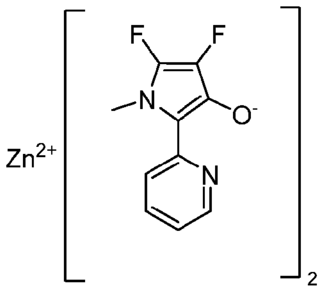 M239, 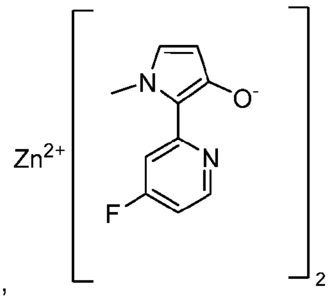 M240,

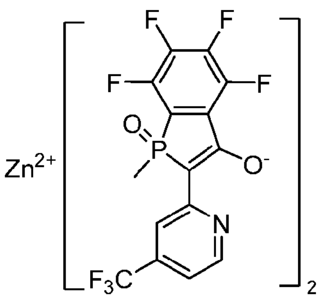 M241, 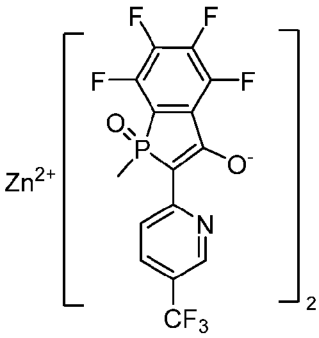 M242, 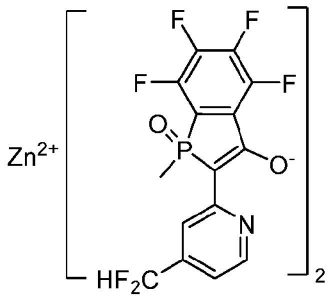 M243,

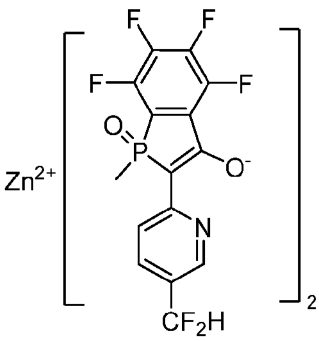 M244, 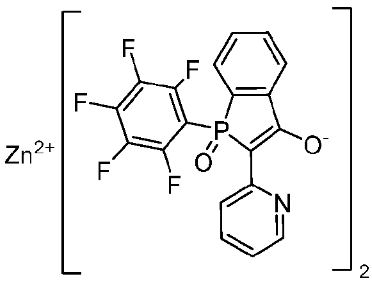 M245, 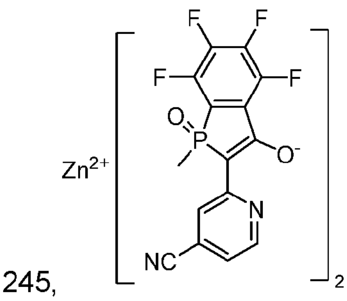 M246,

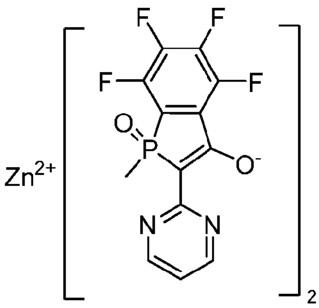 M247, 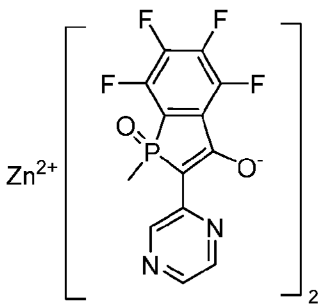 M248, 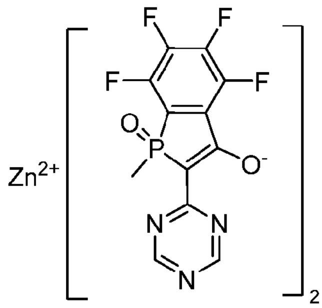 M249,

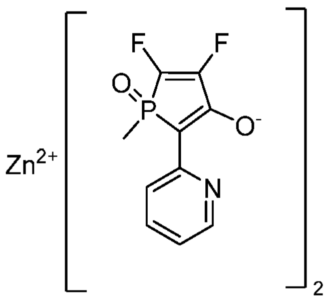 M250, 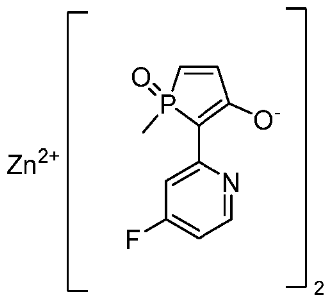 M251, 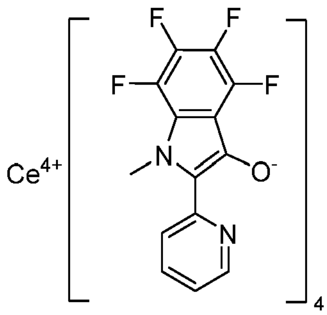 M252.

According to an embodiment, the compound of formula (I) is selected from M1 to M 215, more preferred M1 to M188 and most preferred M1 to M63.

The present invention furthermore relates to the use of a compound of formula (Ia)

$$M^{n\oplus}(L^{\ominus})_n\ (AL)_m \quad \text{(Ia)},$$

wherein:

- M is a metal ion,
- n is the valency of M and selected from 1 to 4;
- AL is an ancillary ligand which coordinates to the metal ion M;
- m is an integer selected from 0 to 2
- L is a ligand of formula (II)

E-U (II)

wherein E is selected from formula (III)

$$^{\ominus}G-A \quad \text{(III)},$$

wherein

G is selected from O and S;

A is is bound to U via a single or double bond and selected from formula (IV)

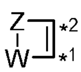 (IV)

wherein the asterisks "*1" denotes the binding position to U; and the asterisks "*2" denotes the binding position to G; whereby

W is selected from S, NR', C=S, and Q=O;

wherein Q is selected from C; S, SO, PR',
wherein R' is selected from substituted or unsubstituted $C_1$ to $C_{16}$ alkyl, substituted or unsubstituted $C_6$ to $C_{40}$ aryl, substituted or unsubstituted $C_3$ to $C_{40}$ heteroaryl;
wherein the one or more substituents on R' is independently selected from D, electron-withdrawing group, F, CN, $NO_2$, substituted or unsubstituted $C_1$ to $C_6$ alkyl, partially fluorinated or perfluorinated $C_1$ to $C_6$ alkyl, $CH_2F$, $CHF_2$, CHDF, $CD_2F$, $CDF_2$, $CF_3$, substituted or unsubstituted $C_1$ to $C_6$ alkoxy, partially fluorinated or perfluorinated $C_1$ to $C_6$ alkoxy, $OCH_2F$, $OCHF_2$, OCHDF, $OCD_2F$, $OCDF_2$, $OCF_3$;

Z is selected from formula (V);

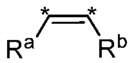 (V),

wherein the asterisks "*" denotes the binding positions to the other moieties of formula (IV);

$R^a$, and $R^b$ are independently selected from H, D, substituted or unsubstituted $C_1$ to $C_{12}$ alkyl, substituted or unsubstituted $C_6$ to $C_{19}$ aryl, substituted or unsubstituted $C_2$ to $C_{20}$ heteroaryl, substituted or unsubstituted 6-membered heteroaryl, electron-withdrawing group, $NO_2$, partially fluorinated or perfluorinated $C_1$ to $C_6$ alkyl, $CH_2F$, $CHF_2$, CHDF, $CD_2F$, $CDF_2$, $CF_3$, substituted or unsubstituted $C_1$ to $C_6$ alkoxy, partially fluorinated or perfluorinated $C_1$ to $C_6$ alkoxy, $OCH_2F$, $OCHF_2$, OCHDF, $OCD_2F$, $OCDF_2$, $OCF_3$, F or CN,

wherein the one or more substituents on $R^a$, and $R^b$ is independently selected from D, electron-withdrawing group, F, CN, $NO_2$, substituted or unsubstituted $C_1$ to $C_6$ alkyl, partially fluorinated or perfluorinated $C_1$ to $C_6$ alkyl, $CH_2F$, $CHF_2$, CHDF, $CD_2F$, $CDF_2$, $CF_3$, substituted or unsubstituted $C_1$ to $C_6$ alkoxy, partially fluorinated or perfluorinated $C_1$ to $C_6$ alkoxy, $OCH_2F$, $OCHF_2$, OCHDF, $OCD_2F$, $OCDF_2$, $OCF_3$;

or

$R^a$, and $R^b$ form together, irrespective of the previous definition, a substituted or unsubstituted aryl ring, substituted or unsubstituted aryl ring system, substituted or unsubstituted heteroaryl ring or substituted or unsubstituted heteroaryl ring system;

wherein the one or more substituents on the aryl ring, the aryl ring system, the heteroaryl ring and heteroaryl ring system is independently selected from D, electron-withdrawing group, F, CN, $NO_2$, substituted or unsubstituted $C_1$ to $C_6$ alkyl, partially fluorinated, or perfluorinated $C_1$ to $C_6$ alkyl, $CH_2F$, $CHF_2$, CHDF, $CD_2F$, $CDF_2$, $CF_3$, substituted or unsubstituted $C_1$ to $C_6$ alkoxy, partially fluorinated or perfluorinated $C_1$ to $C_6$ alkoxy, $OCH_2F$, $OCHF_2$, OCHDF, $OCD_2F$, $OCDF_2$, $OCF_3$;

wherein U is selected from formula (VI)

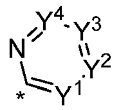 (VI)

wherein the asterisks "*" denotes the binding position;

$Y^1$ is selected from $CR^1$ or N;

$Y^2$ is selected from $CR^2$ or N;

$Y^3$ is selected from $CR^3$ or N;

$Y^4$ is selected from $CR^4$ or N;

wherein 0, 1, or 2 of the group consisting of $Y^1$ , $Y^2$,$Y^3$, $Y^4$ are selected from N;

$R^1$, $R^2$, $R^3$, and $R^4$ are independently selected from H, D, substituted or unsubstituted $C_1$ to $C_{12}$ alkyl, substituted or unsubstituted $C_6$ to $C_{19}$ aryl, substituted or unsubstituted $C_2$ to $C_{20}$ heteroaryl, substituted or unsubstituted 6-membered heteroaryl, electron-withdrawing group, $NO_2$, partially fluorinated or perfluorinated $C_1$ to $C_6$ alkyl, $CH_2F$, $CHF_2$, CHDF, $CD_2F$, $CDF_2$, $CF_3$, substituted or unsubstituted $C_1$ to $C_6$ alkoxy, partially fluorinated or perfluorinated $C_1$ to $C_6$ alkoxy, $OCH_2F$, $OCHF_2$, OCHDF, $OCD_2F$, $OCDF_2$, $OCF_3$, F or CN,

wherein the one or more substituents on $R^1$, $R^2$, $R^3$, and $R^4$ is independently selected from D, electron-withdrawing group, F, CN, $NO_2$, substituted or unsubstituted $C_1$ to $C_6$ alkyl, partially fluorinated or perfluorinated $C_1$ to $C_6$ alkyl, $CH_2F$, $CHF_2$, CHDF, $CD_2F$, $CDF_2$, $CF_3$, substituted or unsubstituted $C_1$ to $C_6$ alkoxy, partially fluorinated or perfluorinated $C_1$ to $C_6$ alkoxy, $OCH_2F$, $OCHF_2$, OCHDF, $OCD_2F$, $OCDF_2$, $OCF_3$;

for one or more of the following:

- hole injection material;
- charge generation material;
- dopant for doping an organic semiconductor;
- p-dopant for a hole transport material;
- organic semiconductor material;
- electron transport material; and/ or
- electron injection material.

The present invention furthermore relates to the use of a compound of formula (I) for one or more of the following:

- hole injection material;
- charge generation material;
- dopant for doping an organic semiconductor;
- p-dopant for a hole transport material;
- organic semiconductor material;
- electron transport material; and/ or
- electron injection material.

All restrictions and/or preferred embodiments for the compound formula (I) may apply *mutatis mutandis.*

Semiconductor material

According to another aspect, a semiconductor material is provided which comprises at least one compound of Formula (I) according to the present invention.

According to one embodiment, the semiconductor material comprises in addition at least one covalent matrix compound or at least one substantially covalent matrix compound.

According to another aspect, a semiconductor material comprises at least one compound of Formula (I) according to the present invention and in addition at least one covalent matrix compound or at least one substantially covalent matrix compound.

Organic semiconductor layer

According to another aspect, an organic semiconductor layer is provided which comprises at least one compound of Formula (I) according to the present invention.

The organic semiconductor layer may be formed on the anode layer or cathode layer by vacuum deposition, spin coating, printing, casting, slot-die coating, Langmuir-Blodgett (LB) deposition, or the like. When the organic semiconductor layer is formed using vacuum deposition, the deposition conditions may vary according to the compound(s) that are used to form the layer, and the desired structure and thermal properties of the layer. In general, however, conditions for vacuum deposition may include a deposition temperature of 100° C to 350° C, a pressure of $10^{-8}$ to $10^{-3}$ Torr (1 Torr equals 133.322 Pa), and a deposition rate of 0.1 to 10 nm/sec.

When the organic semiconductor layer is formed using spin coating or printing, coating conditions may vary according to the compound(s) that are used to form the layer, and the desired structure and thermal properties of the organic semiconductor layer. For example, the coating conditions may include a coating speed of about 2000 rpm to about 5000 rpm, and a thermal treatment temperature of about 80° C to about 200° C. Thermal treatment removes a solvent after the coating is performed.

The thickness of the organic semiconductor layer may be in the range from about 1 nm to about 20 nm, and for example, from about 2 nm to about 15 nm, alternatively about 2 nm to about 12 nm.

When the thickness of the organic semiconductor layer is within this range, the organic semiconductor layer may have excellent hole injecting and/or hole generation characteristics, without a substantial penalty in driving voltage.

According to one embodiment of the present invention, the organic semiconductor layer may comprise:

- at least about ≥ 0.5 wt.-% to about ≤ 30 wt.-%, preferably about ≥ 0.5 wt.-% to about ≤ 20 wt.-%, and more preferred about ≥ 1 wt.-% to about ≤ 15 wt.-% of a compound of Formula (I), and
- at least about ≥ 70 wt.-% to about ≤ 99.5 wt.-%, preferably about ≥ 80 wt.-% to about ≤ 99.5 wt.-%, and more preferred about ≥ 85 wt.-% to about ≤ 99 wt.-% of a substantially covalent matrix compound; preferably the wt.-% of the compound of Formula (I) is lower than the wt.-% of the substantially covalent matrix compound; wherein the weight-% of the components are based on the total weight of the organic semiconductor layer.

According to one embodiment of the present invention the organic semiconductor layer and/or the compound of Formula (I) are non-emissive.

In the context of the present specification the term "essentially non-emissive" or "non-emissive" means that the contribution of the compound or layer to the visible emission spectrum from the device is less than 10 %, preferably less than 5 % relative to the visible emission spectrum. The visible emission spectrum is an emission spectrum with a wavelength of about ≥ 380 nm to about ≤ 780 nm.

Substantially covalent matrix compound/ covalent matrix compound

According to another aspect of the present invention, the semiconductor material and/or the organic semiconductor layer may further comprise a substantially covalent matrix compound.

The substantially covalent matrix compound, also named matrix compound, may be an organic aromatic matrix compounds, which comprises organic aromatic covalent bonded carbon atoms. The substantially covalent matrix compound may be an organic compound, consisting substantially from covalently bound C, H, O, N, S, which may optionally comprise also covalently bound B, P or Si. The substantially covalent matrix compound may be an organic aromatic covalent bonded compound, which is free of metal atoms, and the majority of its skeletal atoms may be selected from C, O, S, N and preferably from C, O and N, wherein the majority of atoms are C-atoms. Alternatively, the covalent matrix compound is free of metal atoms and majority of its skeletal atoms may be selected from C and N, preferably the covalent matrix compound is free of metal atoms and majority of its skeletal atoms may be selected from C and the minority of its skeletal atoms may be N.

According to one embodiment, the substantially covalent matrix compound may have a molecular weight Mw of ≥ 400 and ≤ 2000 g/mol, preferably a molecular weight Mw of ≥ 450 and ≤ 1500 g/mol, further preferred a molecular weight Mw of ≥ 500 and ≤ 1000 g/mol, in addition preferred a molecular weight Mw of ≥ 550 and ≤ 900 g/mol, also preferred a molecular weight Mw of ≥ 600 and ≤ 800 g/mol.

In one embodiment, the HOMO level of the substantially covalent matrix compound may be more negative than the HOMO level of N2,N2,N2',N2',N7,N7,N7',N7'-octakis(4-methoxyphenyl)-9,9'-spirobi[fluorene]-2,2',7,7'-tetraamine (CAS 207739-72-8) when determined under the same conditions.

In one embodiment of the present invention, the substantially covalent matrix compound may be free of alkoxy groups.

Preferably, the substantially covalent matrix compound comprises at least one arylamine moiety, alternatively a diarylamine moiety, alternatively a triarylamine moiety.

Preferably, the substantially covalent matrix compound is free of TPD or NPB.

Compound of formula (IIIa) or a compound of formula (IIIb)

According to another aspect of the present invention, the substantially covalent matrix compound or covalent matrix compound, also referred to as matrix compound herein, may comprises at least one arylamine compound, diarylamine compound, triarylamine compound, a compound of formula (IIIa) or a compound of formula (IIIb):

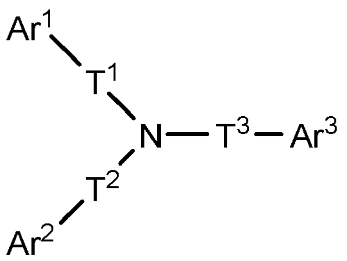
(IIIa),
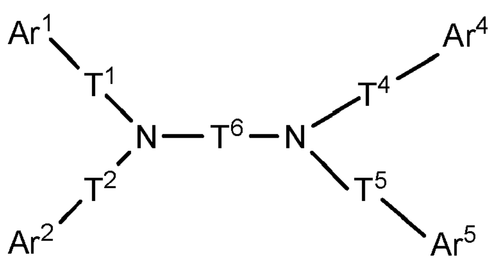
(IIIb),

wherein:

$T^1$, $T^2$, $T^3$, $T^4$ and $T^5$ are independently selected from a single bond, phenylene, biphenylene, terphenylene or naphthenylene, preferably a single bond or phenylene;

$T^6$ is phenylene, biphenylene, terphenylene or naphthenylene;

$Ar^1$, $Ar^2$, $Ar^3$, $Ar^4$ and $Ar^5$ are independently selected from substituted or unsubstituted $C_6$ to $C_{20}$ aryl, or substituted or unsubstituted $C_3$ to $C_{20}$ heteroarylene, substituted or unsubstituted biphenylene, substituted or unsubstituted fluorene, substituted 9-fluorene, substituted 9,9-fluorene, substituted or unsubstituted naphthalene, substituted or unsubstituted anthracene, substituted or unsubstituted phenanthrene, substituted or unsubstituted pyrene, substituted or unsubstituted perylene, substituted or unsubstituted triphenylene, substituted or unsubstituted tetracene, substituted or unsubstituted tetraphene, substituted or unsubstituted dibenzofurane, substituted or unsubstituted dibenzothiophene, substituted or unsubstituted xanthene, substituted or unsubstituted carbazole, substituted 9-phenylcarbazole, substituted or unsubstituted azepine, substituted or unsubstituted dibenzo[b,f]azepine, substituted or unsubstituted 9,9'-spirobi[fluorene], substituted or unsubstituted spiro[fluorene-9,9'-xanthene], or a substituted or unsubstituted aromatic fused ring system comprising at least three substituted or unsubstituted aromatic rings selected from the group comprising substituted or unsubstituted non-hetero, substituted or unsubstituted hetero 5-member rings, substituted or unsubstituted 6-member rings and/or substituted or unsubstituted 7-member rings, substituted or unsubstituted fluorene, or a fused ring system comprising 2 to 6 substituted or unsubstituted 5- to 7-member rings and the rings are selected from the group comprising (i) unsaturated 5- to 7-member ring of a

heterocycle, (ii) 5- to 6-member of an aromatic heterocycle, (iii) unsaturated 5- to 7-member ring of a non-heterocycle, (iv) 6-member ring of an aromatic non-heterocycle;

wherein

the substituents of $Ar^1$, $Ar^2$, $Ar^3$, $Ar^4$ and $Ar^5$ are selected the same or different from the group comprising H, D, F, C(-O)$R^2$, CN, $Si(R^2)_3$, P(-O)$(R^2)_2$, $OR^2$, S(-O)$R^2$, S(-O)$_2R^2$, substituted or unsubstituted straight-chain alkyl having 1 to 20 carbon atoms, substituted or unsubstituted branched alkyl having 1 to 20 carbon atoms, substituted or unsubstituted cyclic alkyl having 3 to 20 carbon atoms, substituted or unsubstituted alkenyl or alkynyl groups having 2 to 20 carbon atoms, substituted or unsubstituted substituted or unsubstituted aromatic ring systems having 6 to 40 aromatic ring atoms, and substituted or unsubstituted heteroaromatic ring systems having 5 to 40 aromatic ring atoms, unsubstituted $C_6$ to $C_{18}$ aryl, unsubstituted $C_3$ to $C_{18}$ heteroaryl, a fused ring system comprising 2 to 6 unsubstituted 5- to 7-member rings and the rings are selected from the group comprising unsaturated 5- to 7-member ring of a heterocycle, 5- to 6-member of an aromatic heterocycle, unsaturated 5- to 7-member ring of a non-heterocycle, and 6-member ring of an aromatic non-heterocycle,

wherein $R^2$ may be selected from H, D, straight-chain alkyl having 1 to 6 carbon atoms, branched alkyl having 1 to 6 carbon atoms, cyclic alkyl having 3 to 6 carbon atoms, alkenyl or alkynyl groups having 2 to 6 carbon atoms, $C_6$ to $C_{18}$ aryl or $C_3$ to $C_{18}$ heteroaryl.

Preferably, the substituents of $Ar^1$, $Ar^2$, $Ar^3$, $Ar^4$ and $Ar^5$ are selected the same or different from the group comprising H, straight-chain alkyl having 1 to 6 carbon atoms, branched alkyl having 1 to 6 carbon atoms, cyclic alkyl having 3 to 6 carbon atoms, alkenyl or alkynyl groups having 2 to 6 carbon atoms, $C_6$ to $C_{18}$ aryl, $C_3$ to $C_{18}$ heteroaryl, a fused ring system comprising 2 to 4 unsubstituted 5- to 7-member rings and the rings are selected from the group comprising unsaturated 5- to 7-member ring of a heterocycle, 5- to 6-member of an aromatic heterocycle, unsaturated 5- to 7-member ring of a non-heterocycle, and 6-member ring of an aromatic non-heterocycle; more preferred the substituents are selected the same or different from the group consisting of H, straight-chain alkyl having 1 to 4 carbon atoms, branched alkyl having 1 to 4 carbon atoms, cyclic alkyl having 3 to 4 carbon atoms and/or phenyl.

Thereby, the compound of formula (IIIa) or (IIIb) may have a rate onset temperature suitable for mass production.

According to an embodiment of the semiconductor material and/or organic semiconductor layer, wherein the substantially covalent matrix compound comprises a compound of formula (IIIa) or formula (IIIb):

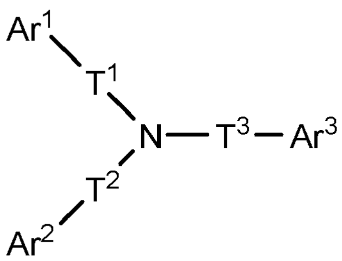
(IIIa),
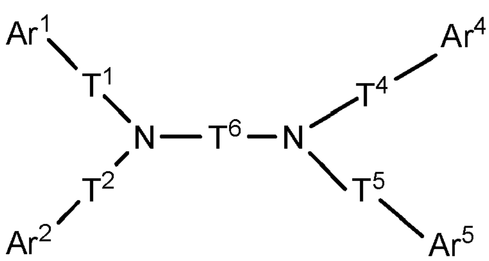
(IIIb),

wherein

$T^1$, $T^2$, $T^3$, $T^4$ and $T^5$ may be independently selected from a single bond, phenylene, biphenylene, terphenylene or naphthenylene, preferably a single bond or phenylene;

$T^6$ is phenylene, biphenylene, terphenylene or naphthenylene;

$Ar^1$, $Ar^2$, $Ar^3$, $Ar^4$ and $Ar^5$ may be independently selected from unsubstituted $C_6$ to $C_{20}$ aryl, or unsubstituted $C_3$ to $C_{20}$ heteroarylene, unsubstituted biphenylene, unsubstituted fluorene, substituted 9-fluorene, substituted 9,9-fluorene, unsubstituted naphthalene, unsubstituted anthracene, unsubstituted phenanthrene, unsubstituted pyrene, unsubstituted perylene, unsubstituted triphenylene, unsubstituted tetracene, unsubstituted tetraphene, unsubstituted dibenzofurane, unsubstituted dibenzothiophene, unsubstituted xanthene, unsubstituted carbazole, substituted 9-phenylcarbazole, unsubstituted azepine, unsubstituted dibenzo[b,f]azepine, unsubstituted 9,9'-spirobi[fluorene], unsubstituted spiro[fluorene-9,9'-xanthene], or a unsubstituted aromatic fused ring system comprising at least three unsubstituted aromatic rings selected from the group comprising unsubstituted non-hetero, unsubstituted hetero 5-member rings, unsubstituted 6-member rings and/or unsubstituted 7-member rings, unsubstituted fluorene, or a fused ring system comprising 2 to 6 unsubstituted 5- to 7-member rings and the rings are selected from the group comprising (i) unsaturated 5- to 7-member ring of a heterocycle, (ii) 5- to 6-member of an aromatic heterocycle, (iii) unsaturated 5- to 7-member ring of a non-heterocycle, (iv) 6-member ring of an aromatic non-heterocycle.

According to an embodiment of the semiconductor material and/or organic semiconductor layer, wherein the

substantially covalent matrix compound comprises a compound of formula (IIIa) or formula (IIIb):

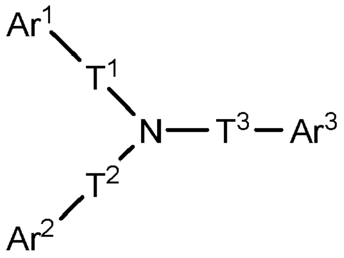 (IIIa), 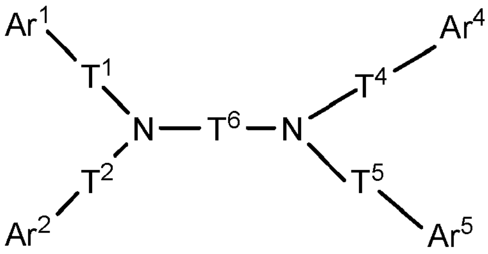 (IIIb),

wherein

- $T^1$, $T^2$, $T^3$, $T^4$ and $T^5$ may be independently selected from a single bond, phenylene, biphenylene, terphenylene or naphthenylene, preferably a single bond or phenylene;
- $T^6$ is phenylene, biphenylene, terphenylene or naphthenylene;
- $Ar^1$, $Ar^2$, $Ar^3$, $Ar^4$ and $Ar^5$ may be independently selected from unsubstituted $C_6$ to $C_{20}$ aryl, or unsubstituted $C_3$ to $C_{20}$ heteroarylene, unsubstituted biphenylene, unsubstituted fluorene, substituted 9-fluorene, substituted 9,9-fluorene, unsubstituted naphthalene, unsubstituted anthracene, unsubstituted phenanthrene, unsubstituted pyrene, unsubstituted perylene, unsubstituted triphenylene, unsubstituted tetracene, unsubstituted tetraphene, unsubstituted dibenzofurane, unsubstituted dibenzothiophene, unsubstituted xanthene, unsubstituted carbazole, substituted 9-phenylcarbazole, unsubstituted azepine, unsubstituted dibenzo[b,f]azepine, unsubstituted 9,9'-spirobi[fluorene], unsubstituted spiro[fluorene-9,9'-xanthene].

Thereby, the compound of formula (IIIa) or (IIIb) may have a rate onset temperature suitable for mass production.

According to an embodiment wherein $T^1$, $T^2$, $T^3$, $T^4$ and $T^5$ may be independently selected from a single bond, phenylene, biphenylene or terphenylene.

According to an embodiment wherein $T^1$, $T^2$, $T^3$, $T^4$ and $T^5$ may be independently selected from phenylene, biphenylene or terphenylene and one of $T^1$, $T^2$, $T^3$, $T^4$ and $T^5$ are a single bond. According to an embodiment wherein $T^1$, $T^2$, $T^3$, $T^4$ and $T^5$ may be independently selected from phenylene or biphenylene and one of $T^1$, $T^2$, $T^3$, $T^4$ and $T^5$ are a single bond.

According to an embodiment wherein $T^1$, $T^2$, $T^3$, $T^4$ and $T^5$ may be independently selected from phenylene or biphenylene and two of $T^1$, $T^2$, $T^3$, $T^4$ and $T^5$ are a single bond.

According to an embodiment wherein $T^1$, $T^2$ and $T^3$ may be independently selected from phenylene and one of $T^1$, $T^2$ and $T^3$ are a single bond.

According to an embodiment wherein $T^1$, $T^2$ and $T^3$ may be independently selected from phenylene and two of $T^1$, $T^2$ and $T^3$ are a single bond.

According to an embodiment wherein $T^6$ may be phenylene, biphenylene, terphenylene. According to an embodiment wherein $T^6$ may be phenylene.

According to an embodiment wherein $T^6$ may be biphenylene. According to an embodiment wherein $T^6$ may be terphenylene.

According to an embodiment wherein $Ar^1$, $Ar^2$, $Ar^3$, $Ar^4$ and $Ar^5$ may be independently selected from formulae (E1) to (E16):

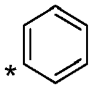 (E1), 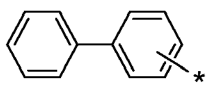 (E2), 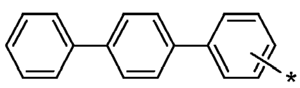 (E3), 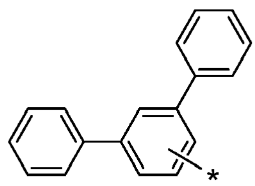 (E4),

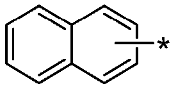 (E5), 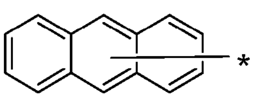 (E6), 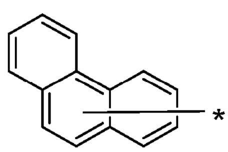 (E7), 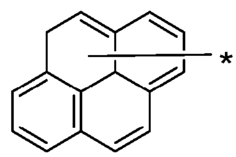 (E8),

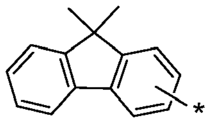 (E9), 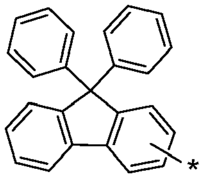 (E10), 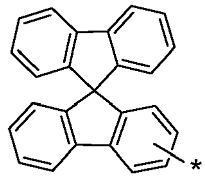 (E11),

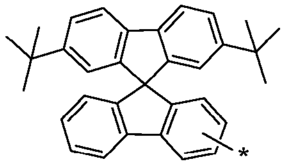 (E12), 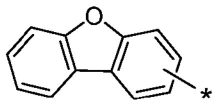 (E13), 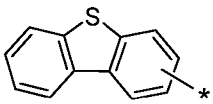 (E14),

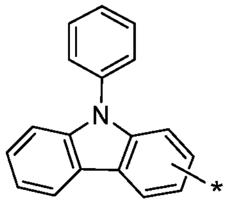 (E15), 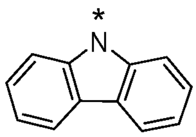 (E16),

wherein the asterix "*" denotes the binding position.

According to an embodiment, wherein $Ar^1$, $Ar^2$, $Ar^3$, $Ar^4$ and $Ar^5$ may be independently selected from (E1) to (E15); alternatively selected from (E1) to (E10) and (E13) to (E15).

According to an embodiment, wherein $Ar^1$, $Ar^2$, $Ar^3$, $Ar^4$ and $Ar^5$ may be independently selected from the group consisting of (E1), (E2), (E5), (E7), (E9), (E10), (E13) to (E16).

The rate onset temperature may be in a range particularly suited to mass production, when $Ar^1$, $Ar^2$, $Ar^3$, $Ar^4$ and $Ar^5$ are selected in this range.

The "matrix compound of formula (IIIa) or formula (IIIb) " may be also referred to as "hole transport compound".

According to one embodiment the compound of formula (IIIa) or formula (IIIb) may comprises at least ≥ 1 to ≤ 6 substituted or unsubstituted aromatic fused ring systems comprising heteroaromatic rings.

According to one embodiment the compound of formula (IIIa) or formula (IIIb) may comprises at least ≥ 1 to ≤ 6 substituted or unsubstituted aromatic fused ring systems comprising heteroaromatic rings and at least ≥ 1 to ≤ 3 substituted or unsubstituted unsaturated 5- to 7-member ring of a heterocycle, preferably ≥ 2 to ≤ 5 substituted or unsubstituted aromatic fused ring systems comprising heteroaromatic rings.

According to one embodiment the compound of formula (IIIa) or formula (IIIb) may comprises at least ≥ 1 to ≤ 6 substituted or unsubstituted aromatic fused ring systems comprising heteroaromatic rings and at least ≥ 1 to ≤ 3 substituted or unsubstituted unsaturated 5- to 7-member ring of a heterocycle, preferably ≥ 2 to ≤ 5 substituted or unsubstituted aromatic fused ring systems comprising heteroaromatic rings, and at least ≥ 1 to ≤ 3 substituted or unsubstituted unsaturated 5- to 7-member ring of a heterocycle, further preferred 3 or 4 substituted or unsubstituted aromatic fused ring systems comprising heteroaromatic rings and optional at least ≥ 1 to ≤ 3 substituted or unsubstituted unsaturated 5- to 7-member ring of a heterocycle, and additional preferred wherein the aromatic fused ring systems comprising heteroaromatic rings are unsubstituted and optional at least ≥ 1 to ≤ 3 unsubstituted unsaturated 5- to 7-member ring of a heterocycle.

According to one embodiment the compound of formula (IIIa) or formula (IIIb) may comprises:

- a substituted or unsubstituted aromatic fused ring systems with at least ≥ 2 to ≤ 6, preferably ≥ 3 to ≤ 5, or 4 fused aromatic rings selected from the group comprising substituted or unsubstituted non-hetero aromatic rings, substituted or unsubstituted hetero 5-member rings, substituted or unsubstituted 6-member rings and/or substituted or unsubstituted unsaturated 5-to 7- member ring of a heterocycle; or
- an unsubstituted aromatic fused ring systems with at least ≥ 2 to ≤ 6, preferably ≥ 3 to ≤ 5, or 4 fused aromatic rings selected from the group comprising unsubstituted non-hetero aromatic rings, unsubstituted hetero 5-member rings, unsubstituted 6-member rings and/or unsubstituted unsaturated 5- to 7-member ring of a heterocycle.

It should be noted here that the wording "aromatic fused ring system" may include at least one aromatic ring and

at least one substituted or unsubstituted unsaturated 5- to 7- member ring. It should be noted here that the substituted or unsubstituted unsaturated 5- to 7- member ring may not be an aromatic ring.

According to one embodiment, the substantially covalent matrix compound comprises at least one naphthyl group, carbazole group, dibenzofurane group, dibenzothiophene group and/or substituted fluorenyl group, wherein the substituents are independently selected from methyl, phenyl or fluorenyl.

According to an embodiment of the present invention, wherein the compound of formula (IIIa) or formula (IIIb) are selected from formulae (F1) to (F23):

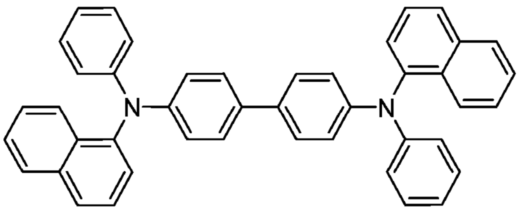
(F1),

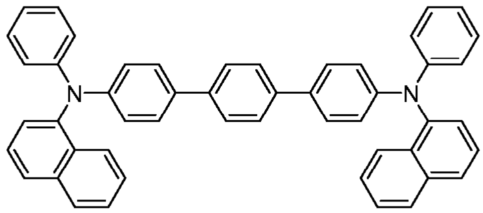
(F2),

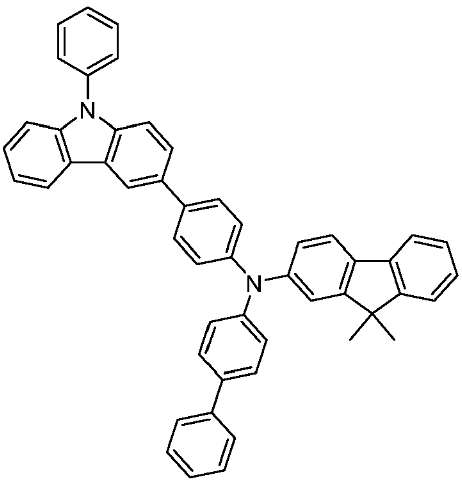
(F3),

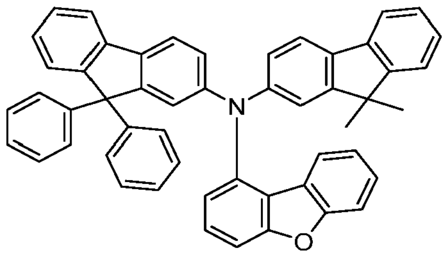
(F4),

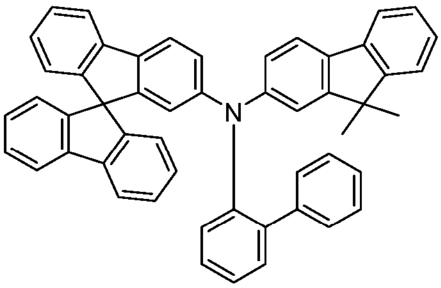
(F5),

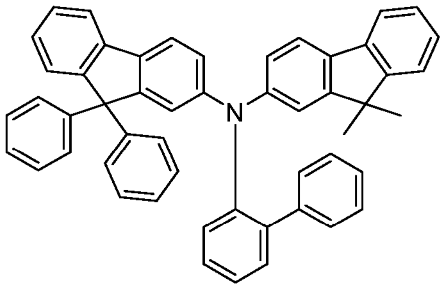
(F6),

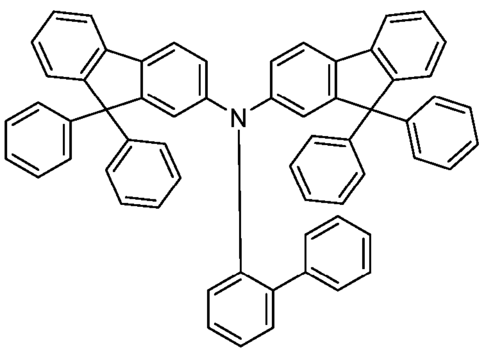
(F7),

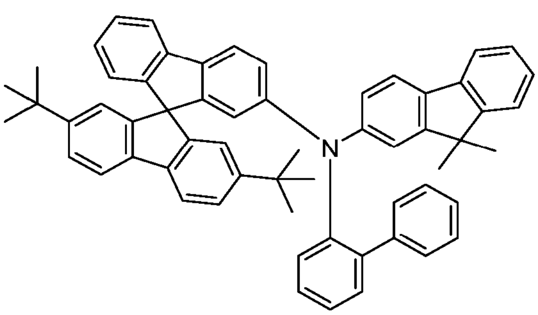
(F8),

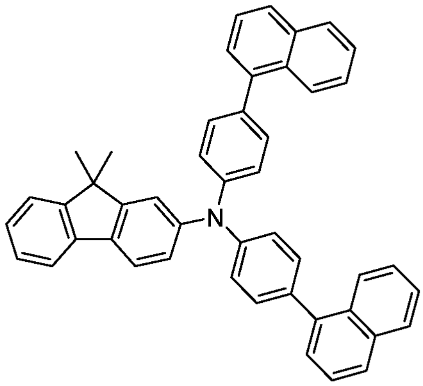
(F9),

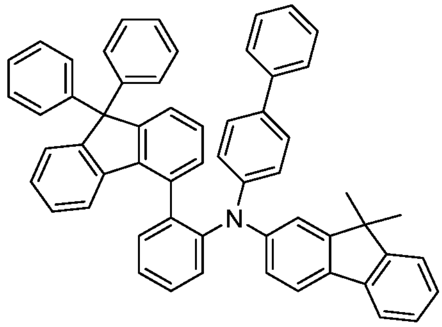
(F10),

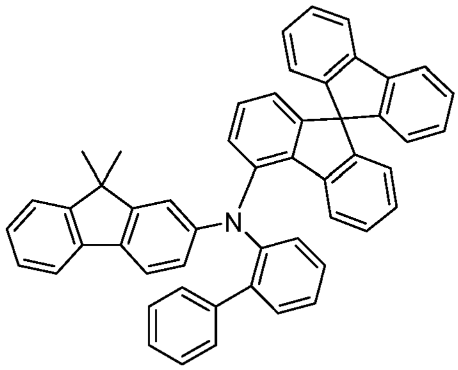
(F11),

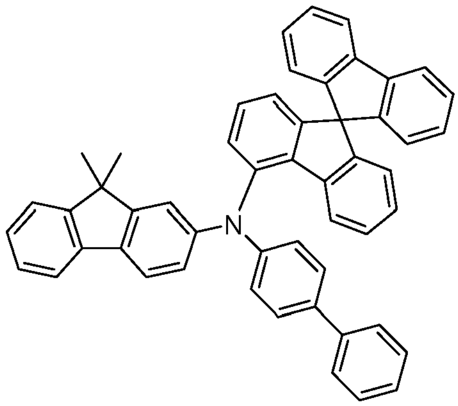
(F12),

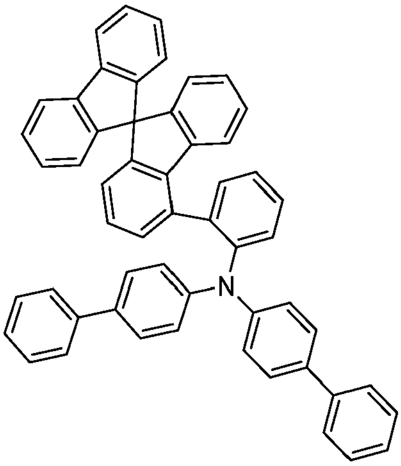
(F13),

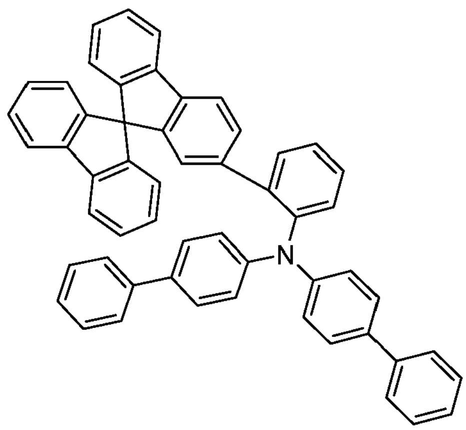
(F14),

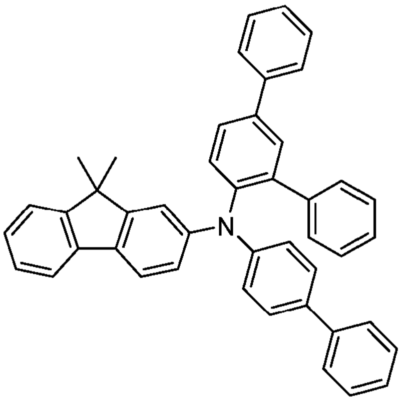 (F15),

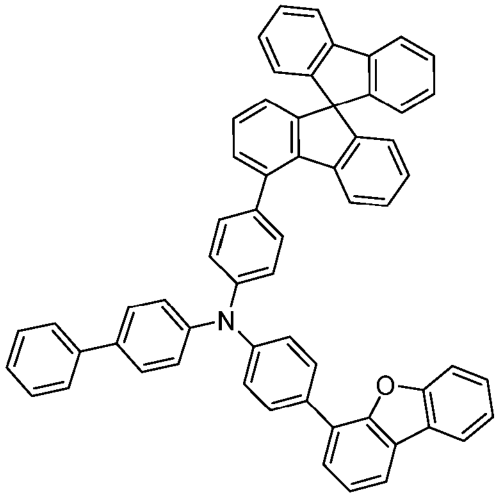 (F16),

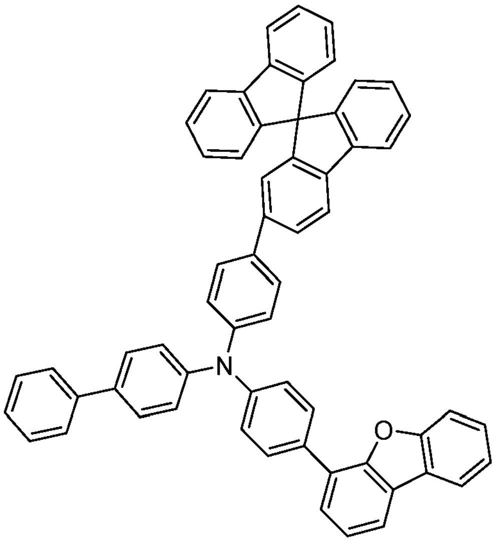 (F17)

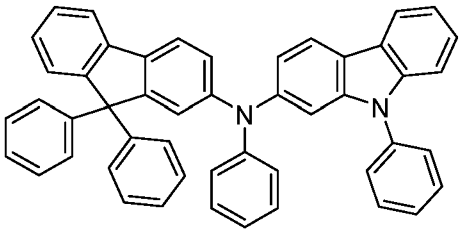 (F18)

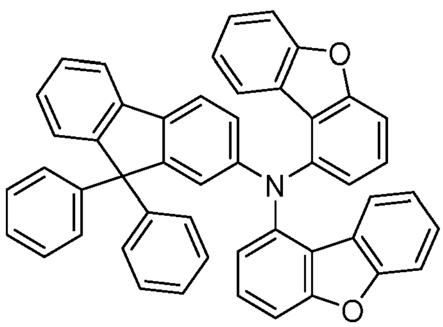 (F19),

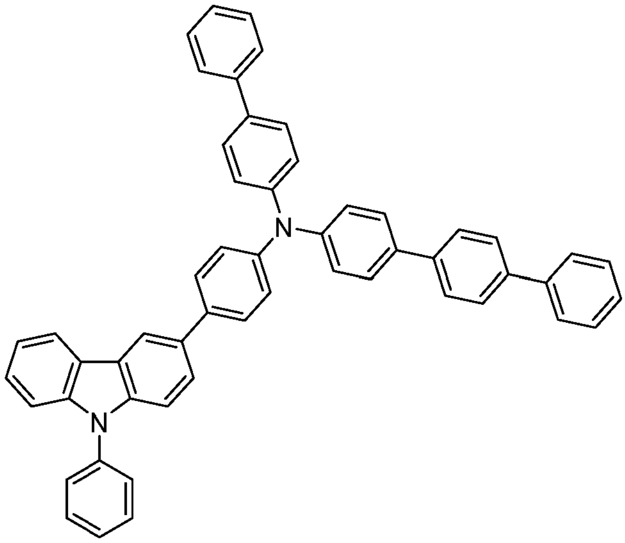 (F20),

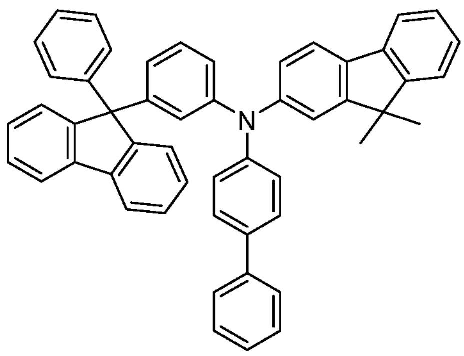

(F21),

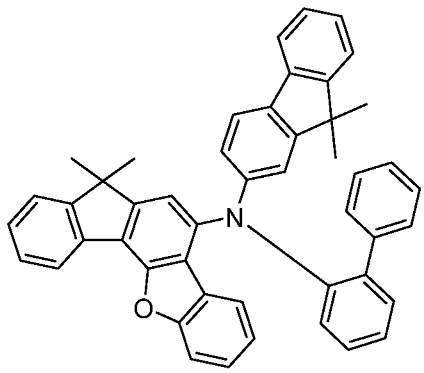

(F22),

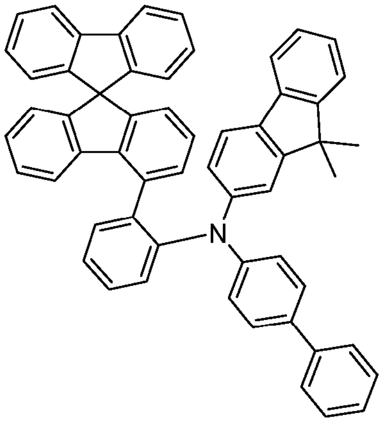

(F23);

preferably the compound of formula (IIIa) or formula (IIIb) is selected from formulae (F3) to (F23), more preferred from (F4) to (F23), most preferred from (F18).

*Organic electronic device*

According to another aspect of the present invention, an organic electronic device is provided, wherein the organic electronic device comprises a semiconductor material, wherein at least one semiconductor material comprises a compound of Formula (I).

According to another aspect of the present invention, an organic electronic device is provided, wherein the organic electronic device comprises an organic semiconductor layer, wherein the organic semiconductor layer comprises a compound of Formula (I).

According to one embodiment of the present invention, the organic electronic device comprising an anode layer, a cathode layer, and an organic semiconductor layer according to claim 9, preferably the organic semiconductor layer is arranged between the anode layer and the cathode layer.

Preferably, the organic semiconductor layer is arranged adjacent to the anode layer, preferably the organic semiconductor layer is arranged in direct contact with the anode layer.

Preferably such organic semiconductor layer is a hole injection layer.

Surprisingly it has been shown that for many applications within the present invention such an organic electronic device has improved properties, especially in view of the improved operating voltage, improved cd/A efficiency, improved external quantum efficiency and/or improved stability in operating voltage over time.

According to one embodiment of the present invention, the organic electronic device is selected from the group comprising a light emitting device, thin film transistor, a battery, a display device, a photovoltaic cell (OPV), or an organic photodetector, and preferably a light emitting device, preferably the electronic device is part of a display device or lighting device.

According to one embodiment the organic electronic device comprises a compound according to Formula (I) of the present invention is a light emitting device, a thin film transistor, a battery, a display device or a photovoltaic device, and preferably a light emitting device, preferably the electronic device is part of a display device or lighting device.

According to one embodiment of the invention, the organic electronic devices further comprises at least one photoactive layer, wherein the at least one photoactive layer is arranged between the anode layer and the cathode layer.

According to one embodiment of the invention, the organic electronic devices comprises at least one photoactive layer and the at least one organic semiconductor layers is arranged between the anode and the at least one photoactive layer.

According to one embodiment the organic electronic device comprises an anode layer, a cathode layer, at least one photoactive layer and at least one semiconductor layer, wherein the at least one semiconductor layer is arranged between the anode layer and the at least one photoactive layer; and wherein the at least one organic semiconductor layer comprises a compound of Formula (I).

According to one embodiment, the organic electronic device comprises an anode layer, a cathode layer, and at least one organic semiconductor layer, wherein the at least one organic semiconductor layer is arranged between the anode layer and the cathode layer, and wherein the at least one organic semiconductor layer is the organic semiconductor layer according to the present invention.

According to one embodiment of the invention, the organic semiconductor layer is arranged and/or provided adjacent to the anode layer.

According to one embodiment of the invention, the organic semiconductor layer of the present invention is a hole-injection layer.

In case the semiconductor layer of the present invention is a hole-injection layer and/ or is arranged and/or provided adjacent to the anode layer then it is especially preferred that this layer consists essentially of the compound of Formula (I).

In the context of the present specification the term "consisting essentially of " especially means and/or includes a concentration of ≥ 90% (vol/vol) more preferred ≥ 95% (vol/vol) and most preferred ≥ 99% (vol/vol).

According to another aspect, the semiconductor layer may have a layer thickness of at least about ≥ 0.5 nm to about ≤ 10 nm, preferably of about ≥ 2 nm to about ≤ 8 nm, also preferred of about ≥ 3 nm to about ≤ 5 nm.

According to one embodiment of the invention, the semiconductor layer of the present invention may further comprise a substantially covalent matrix compound. Preferably at least one semiconductor layer further comprising a substantially covalent matrix compound is arranged and/or provided adjacent to the anode layer.

According to one embodiment of the invention, the electronic organic device is an electroluminescent device, preferably an organic light emitting diode.

According to one embodiment of the invention, the electronic organic device is an electroluminescent device, preferably an organic light emitting diode and the light is emitted through the cathode layer.

The present invention furthermore relates to a display device comprising an organic electronic device according to the present invention.

According to one embodiment of the invention the electronic organic device is an electroluminescent device, preferably an organic light emitting diode. The present invention furthermore relates to a display device comprising an organic electronic device according to the present invention.

## Further layers

In accordance with the invention, the organic electronic device may comprise, besides the layers already mentioned above, further layers. Exemplary embodiments of respective layers are described in the following:

### *Substrate*

The substrate may be any substrate that is commonly used in manufacturing of, electronic devices, such as organic light-emitting diodes. If light is to be emitted through the substrate, the substrate shall be a transparent or semitransparent material, for example a glass substrate or a transparent plastic substrate. If light is to be emitted through the top surface, the substrate may be both a transparent as well as a non-transparent material, for example a glass substrate, a plastic substrate, a metal substrate or a silicon substrate.

### *Anode layer*

The anode layer, also named anode electrode, may be formed by depositing or sputtering a material that is used to form the anode layer. The material used to form the anode layer may be a high work-function material, so as to facilitate hole injection. The anode layer may be a transparent or reflective electrode. Transparent conductive oxides, such as indium tin oxide (ITO), indium zinc oxide (IZO), tin-dioxide ($SnO2$), aluminum zinc oxide (AlZO) and zinc oxide (ZnO), may be used to form the anode layer. The anode layer may also be formed using metals, typically silver (Ag), gold (Au), or metal alloys.

The anode layer may comprise two or more anode sub-layers.

According to one embodiment, the anode layer comprises a first anode sub-layer and a second anode sub-layer,

wherein the first anode sub-layer is arranged closer to the substrate and the second anode sub-layer is arranged closer to the cathode layer.

According to one embodiment, the anode layer may comprise a first anode sub-layer comprising or consisting of Ag or Au and a second anode-sub-layer comprising or consisting of transparent conductive oxide.

According to one embodiment, the anode layer comprises a first anode sub-layer, a second anode sub-layer and a third anode sub-layer, wherein the first anode sub-layer is arranged closer to the substrate and the second anode sub-layer is arranged closer to the cathode layer, and the third anode sub-layer is arranged between the substrate and the first anode sub-layer.

According to one embodiment, the anode layer may comprise a first anode sub-layer comprising or consisting of Ag or Au, a second anode-sub-layer comprising or consisting of transparent conductive oxide and optionally a third anode sub-layer comprising or consisting of transparent conductive oxide. Preferably the first anode sub-layer may comprise or consists of Ag, the second anode-sublayer may comprise or consists of ITO or IZO and the third anode sub-layer may comprises or consists of ITO or IZO.

Preferably the first anode sub-layer may comprise or consists of Ag, the second anode-sublayer may comprise or consists of ITO and the third anode sub-layer may comprise or consist of ITO.

Preferably, the transparent conductive oxide in the second and third anode sub-layer may be selected the same.

According to one embodiment, the anode layer may comprise a first anode sub-layer comprising Ag or Au having a thickness of 100 to 150 nm, a second anode sub-layer comprising or consisting of a transparent conductive oxide having a thickness of 3 to 20 nm and a third anode sub-layer comprising or consisting of a transparent conductive oxide having a thickness of 3 to 20 nm.

It is to be understood that the third anode layer is not part of the substrate.

*Hole injection layer*

A hole injection layer (HIL) may be formed on the anode electrode by vacuum deposition, spin coating, printing, casting, slot-die coating, Langmuir-Blodgett (LB) deposition, or the like. When the HIL is formed using vacuum deposition, the deposition conditions may vary according to the compound that is used to form the HIL, and the desired structure and thermal properties of the HIL. In general, however, conditions for vacuum deposition may include a deposition temperature of 100° C to 500° C, a pressure of $10^{-8}$ to $10^{-3}$ Torr (1 Torr equals 133.322 Pa), and a deposition rate of 0.1 to 10 nm/sec.

When the HIL is formed using spin coating or printing, coating conditions may vary according to the compound that is used to form the HIL, and the desired structure and thermal properties of the HIL. For example, the coating conditions may include a coating speed of about 2000 rpm to about 5000 rpm, and a thermal treatment temperature of about 80° C to about 200° C. Thermal treatment removes a solvent after the coating is performed.

The HIL may be formed of any compound that is commonly used to form a HIL. Examples of compounds that may be used to form the HIL include a phthalocyanine compound, such as copper phthalocyanine (CuPc), 4,4',4"-tris (3-methylphenylphenylamino) triphenylamine (m-MTDATA), TDATA, 2T-NATA, polyaniline/dodecylbenzenesulfonic acid (Pani/DBSA), poly(3,4-ethylenedioxythiophene)/poly(4-styrenesulfonate) (PEDOT/PSS), polyaniline/camphor sulfonic acid (Pani/CSA), and polyaniline)/poly(4-styrenesulfonate (PANI/PSS).

The HIL may comprise or consist of p-type dopant and the p-type dopant may be selected from tetrafluoro-tetracyanoquinonedimethane (F4TCNQ), 2,2'-(perfluoronaphthalen-2,6-diylidene) dimalononitrile or 2,2',2"-(cyclopropane-1,2,3-triylidene)tris(2-(p-cyanotetrafluorophenyl)acetonitrile) but not limited hereto. The HIL may be selected from a hole-transporting matrix compound doped with a p-type dopant. Typical examples of known doped hole transport materials are: copper phthalocyanine (CuPc), which HOMO level is approximately - 5.2 eV, doped with tetrafluoro-tetracyanoquinonedimethane (F4TCNQ), which LUMO level is about -5.2 eV; zinc phthalocyanine (ZnPc) (HOMO = -5.2 eV) doped with F4TCNQ; $\alpha$-NPD (N,N'-Bis(naphthalen-1-yl)-N,N'-bis(phenyl)-benzidine) doped with F4TCNQ. $\alpha$-NPD doped with 2,2'-(perfluoronaphthalen-2,6-diylidene) dimalononitrile. The p-type dopant concentrations can be selected from 1 to 20 wt.-%, more preferably from 3 wt.-% to 10 wt.-%.

The thickness of the HIL may be in the range from about 1 nm to about 100 nm, and for example, from about 1 nm to about 25 nm. When the thickness of the HIL is within this range, the HIL may have excellent hole injecting characteristics, without a substantial penalty in driving voltage.

*Hole transport layer*

According to one embodiment of the present invention, the organic electronic device may further comprise a hole transport layer, wherein the hole transport layer is arranged between the anode layer and the cathode layer, preferably between the organic semiconductor layer of the present invention and the cathode layer.

The hole transport layer (HTL) may be formed on the HIL by vacuum deposition, spin coating, slot-die coating, printing, casting, Langmuir-Blodgett (LB) deposition, or the like. When the HTL is formed by vacuum deposition or spin

coating, the conditions for deposition and coating may be similar to those for the formation of the HIL. However, the conditions for the vacuum or solution deposition may vary, according to the compound that is used to form the HTL.

The HTL may be formed of any compound that is commonly used to form a HTL. Compounds that can be suitably used are disclosed for example in Yasuhiko Shirota and Hiroshi Kageyama, Chem. Rev. 2007, 107, 953-1010 and incorporated by reference. Examples of the compound that may be used to form the HTL are: carbazole derivatives, such as N-phenylcarbazole or polyvinylcarbazole; benzidine derivatives, such as N,N'-bis(3-methylphenyl)-N,N'-diphenyl-[1,1-biphenyl]-4,4'-diamine (TPD), or N,N'-di(naphthalen-1-yl)-N,N'-diphenyl benzidine (alpha-NPD); and triphenylamine-based compound, such as 4,4',4"-tris(N-carbazolyl)triphenylamine (TCTA). Among these compounds, TCTA can transport holes and inhibit excitons from being diffused into the EML.

According to a preferred embodiment of the present invention, the hole transport layer may comprise a substantially covalent matrix compound.

According to one embodiment of the present invention, the hole transport layer may comprise the same substantially covalent matrix compound as the organic semiconductor layer of the present invention, preferably, the hole transport layer may comprise the same compound of formula (IIIa) or (IIIb) as the organic semiconductor layer of the present invention.

The thickness of the HTL may be in the range of about 5 nm to about 250 nm, preferably, about 10 nm to about 200 nm, further about 20 nm to about 190 nm, further about 40 nm to about 180 nm, further about 60 nm to about 170 nm, further about 80 nm to about 160 nm, further about 100 nm to about 160 nm, further about 120 nm to about 140 nm. A preferred thickness of the HTL may be 170 nm to 200 nm.

When the thickness of the HTL is within this range, the HTL may have excellent hole transporting characteristics, without a substantial penalty in driving voltage.

*Electron blocking layer*

The function of an electron blocking layer (EBL) is to prevent electrons from being transferred from an emission layer to the hole transport layer and thereby confine electrons to the emission layer. Thereby, efficiency, operating voltage and/or lifetime may be improved. Typically, the electron blocking layer comprises a triarylamine compound. The triarylamine compound may have a LUMO level closer to vacuum level than the LUMO level of the hole transport layer. The electron blocking layer may have a HOMO level that is further away from vacuum level compared to the HOMO level of the hole transport layer. The thickness of the electron blocking layer may be selected between 2 and 20 nm.

If the electron blocking layer has a high triplet level, it may also be described as triplet control layer.

The function of the triplet control layer is to reduce quenching of triplets if a phosphorescent green or blue emission layer is used. Thereby, higher efficiency of light emission from a phosphorescent emission layer can be achieved. The triplet control layer is selected from triarylamine compounds with a triplet level above the triplet level of the phosphorescent emitter in the adjacent emission layer. Suitable compounds for the triplet control layer, in particular the triarylamine compounds, are described in EP 2 722 908 A1.

*Photoactive layer (PAL)*

The photoactive layer converts an electrical current into photons or photons into an electrical current.

The PAL may be formed on the HTL by vacuum deposition, spin coating, slot-die coating, printing, casting, LB deposition, or the like. When the PAL is formed using vacuum deposition or spin coating, the conditions for deposition and coating may be similar to those for the formation of the HIL. However, the conditions for deposition and coating may vary, according to the compound that is used to form the PAL.

It may be provided that the photoactive layer does not comprise the compound of Formula (I).

The photoactive layer may be an emission layer (EML), also named light-emitting layer, or a light-absorbing layer.

*Emission layer (EML)*

According to an embodiment, the organic electronic device of the present invention may further comprise an emission layer (EML), wherein the emission layer is arranged between the anode layer and the cathode layer, preferably the emission layer is arranged betweent the organic semiconductor layer and the cathode layer.

The EML may be formed on the HTL by vacuum deposition, spin coating, slot-die coating, printing, casting, LB deposition, or the like. When the EML is formed using vacuum deposition or spin coating, the conditions for deposition and coating may be similar to those for the formation of the HIL. However, the conditions for deposition and coating may vary, according to the compound that is used to form the EML.

The emission layer (EML) may comprise an organic emitter host and a light-emitting compound dopant. Examples of the organic emitter host are Alq3, 4,4'-N,N'-dicarbazole-biphenyl (CBP), poly(n-vinylcarbazole) (PVK),

9,10-di(naphthalene-2-yl)anthracene (ADN), 4,4',4"-tris(carbazol-9-yl)-triphenylamine(TCTA), 1,3,5-tris(N-phenylbenzimidazole-2-yl)benzene (TPBI), 3-tert-butyl-9,10-di-2-naphthylanthracenee (TBADN), distyrylarylene (DSA) and bis(2-(2-hydroxyphenyl)benzo-thiazolate)zinc (Zn(BTZ)2).

The emitter dopant may be a phosphorescent or fluorescent emitter. Phosphorescent emitters and emitters which emit light via a thermally activated delayed fluorescence (TADF) mechanism may be preferred due to their higher efficiency. The emitter may be a small molecule or a polymer.

Examples of red emitter dopants are PtOEP, Ir(piq)3, and Btp2lr(acac), but are not limited thereto. These compounds are phosphorescent emitters, however, fluorescent red emitter dopants could also be used.

Examples of phosphorescent green emitter dopants are Ir(ppy)3 (ppy = phenylpyridine), Ir(ppy)2(acac), Ir(mpyp)3.

Examples of phosphorescent blue emitter dopants are F2Irpic, (F2ppy)2Ir(tmd) and Ir(dfppz)3 and ter-fluorene. 4.4'-bis(4-diphenyl amiostyryl)biphenyl (DPAVBi), 2,5,8,11-tetra-tert-butyl perylene (TBPe) are examples of fluorescent blue emitter dopants.

It may be provided that the emission layer does not comprise the compound of Formula (I).

The amount of the emitter dopant may be in the range from about 0.01 to about 50 parts by weight, based on 100 parts by weight of the host. Alternatively, the emission layer may consist of a light-emitting polymer. The EML may have a thickness of about 10 nm to about 100 nm, for example, from about 20 nm to about 60 nm. When the thickness of the EML is within this range, the EML may have excellent light emission, without a substantial penalty in driving voltage.

According to a preferred embodiment of the present invention, the emission layer comprises a light-emitting compound of formula (IV):

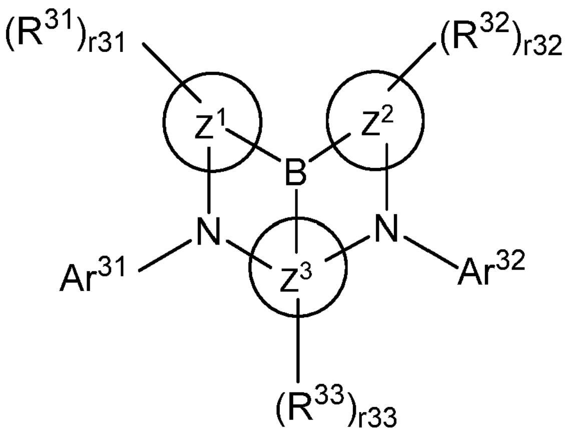

(IV),

wherein

$Z^1$, $Z^2$ and $Z^3$ are the same as or different from each other, and are each independently selected from the group comprising a monocyclic to polycyclic aromatic hydrocarbon ring or monocyclic to polycyclic aromatic hetero ring;
$Ar^{31}$ and $Ar^{32}$ are the same as or selected different from each other, and are each independently a substituted or unsubstituted alkyl group, a substituted or unsubstituted aryl group, or a substituted or unsubstituted heteroaryl group, or are bonded to an adjacent substituent to form a substituted or unsubstituted aromatic ring or a substituted or unsubstituted aliphatic ring;
$R^{31}$, $R^{32}$ and $R^{33}$ are the same as or different from each other, and are each independently selected from the group comprising hydrogen, deuterium, a substituted or unsubstituted alkyl group, a substituted or unsubstituted silyl group, a substituted or unsubstituted amine group, a substituted or unsubstituted aryl group, or a substituted or unsubstituted heteroaryl group, or adjacent substituents are bonded to each other to form a substituted or unsubstituted aromatic ring or a substituted or unsubstituted aliphatic ring,
wherein one or more substituents selected from the group consisting of deuterium, an alkyl group having 1 to 6 carbon atoms, an alkyl silyl group having 1 to 30 carbon atoms, an aryl silyl group having 6 to 50 carbon atoms, an alkylamine group having 1 to 30 carbon atoms, an alkylarylamine group having 1 to 50 carbon atoms, an arylamine group having 6 to 50 carbon atoms, an aryl group having 6 to 30 carbon atoms, and a heteroaryl group having 2 to 30 carbon atoms, or a substituent to which two or more substituents selected from the group are linked, or adjacent substituents are bonded to each other to form an aliphatic hydrocarbon ring having 3 to 60 carbon atoms, which is unsubstituted or substituted with the substituent;
$r^{31}$, $r^{32}$ and $r^{33}$ are each an integer from 0, 1, 2, 3 or 4, and when $r^{31}$ to $r^{33}$ are 2 or higher, substituents in the parenthesis are the same as or different from each other.

According to one embodiment, for formula (III):

$Z^1$, $Z^2$ and $Z^3$ are the same as or selected different from each other, and are each independently selected from the group comprising a monocyclic to bicyclic aromatic hydrocarbon ring, or a monocyclic to bicyclic aromatic hetero ring containing O, N or S;

$Ar^{31}$ and $Ar^{32}$ are the same as or selected different from, and are each independently selected from the group comprising an alkyl group having 1 to 10 carbon atoms, which is unsubstituted or substituted with an aryl group, an aryl group having 6 to 30 carbon atoms, which is unsubstituted or substituted with an aryl group, or a heteroaryl group having 2 to 30 carbon atoms;

$R^{31}$, $R^{32}$ and $R^{33}$ are the same as or selected different from each other, and are each independently selected from the group comprising hydrogen, deuterium, a substituted or unsubstituted alkyl group, a substituted or unsubstituted cycloalkyl group, a substituted or unsubstituted silyl group, a substituted or unsubstituted aryl group, or a substituted or unsubstituted heteroaryl group.

According to one embodiment, wherein for formula (III):

$Z^1$, $Z^2$ and $Z^3$ are the same as or selected different from each other, and are each independently selected from the group comprising a benzene ring or a thiophene ring;

$Ar^{31}$ and $Ar^{32}$ are the same as or selected different from each other, and are each independently selected from the group comprising phenyl group, a biphenyl group, a naphthyl group, a dimethyl fluorenyl group, a diphenyl fluorenyl group, a dibenzofuran group, or a dibenzothiophene group;

$R^{31}$, $R^{32}$ and $R^{33}$ are the same as or selected different from each other, and are each independently selected from the group comprising hydrogen, deuterium, a substituted or unsubstituted alkyl group having 1 to 10 carbon atoms, a substituted or unsubstituted cycloalkyl group having 5 to 30 carbon atoms, a substituted or unsubstituted silyl group having 1 to 10 carbon atoms, a substituted or unsubstituted aryl group having 6 to 30 carbon atoms, or a substituted or unsubstituted heteroaryl group having 2 to 30 carbon atoms.

According to a preferred embodiment of the present invention, the emission layer comprises a light-emitting compound of formula (IV) is selected from formulae (BD1) to (BD9):

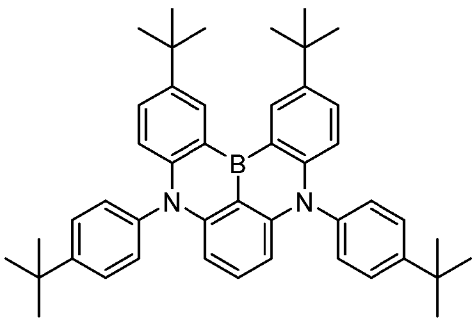
(BD1),

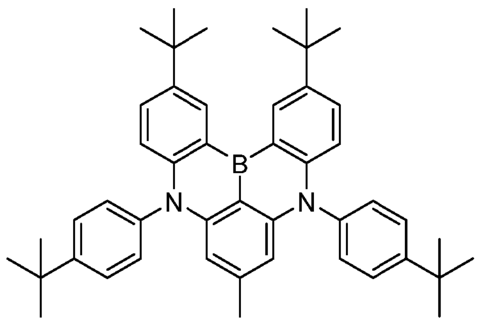
(BD2),

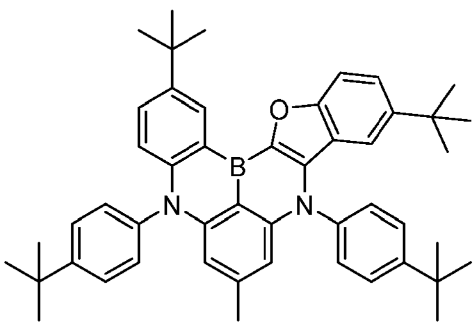
(BD3),

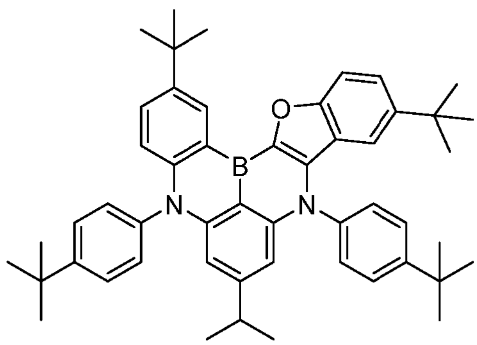
(BD4),

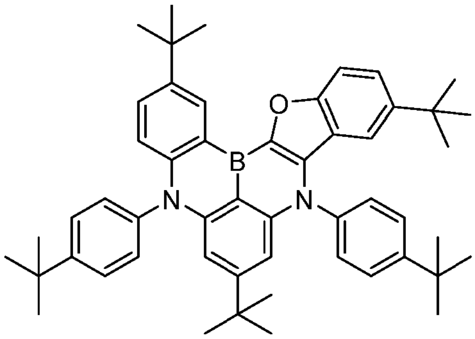
(BD5),
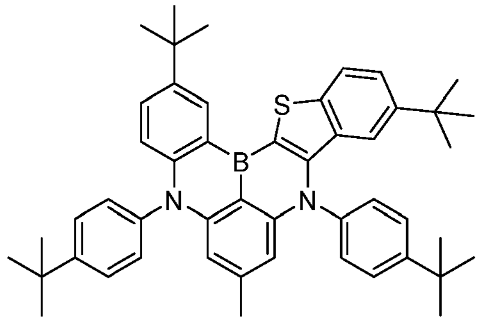
(BD6),
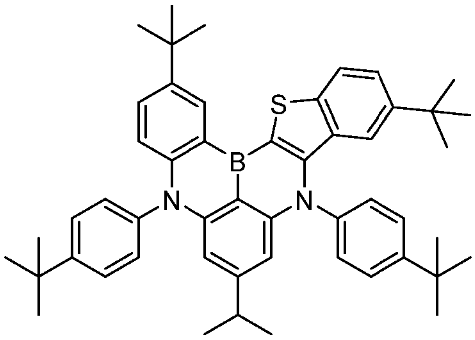
(BD7),
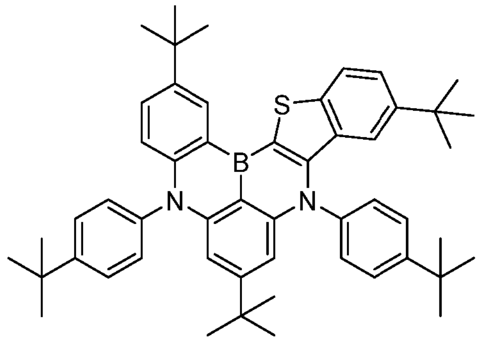
(BD8),
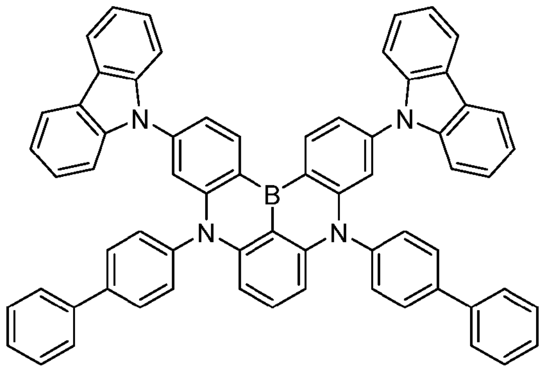
(BD9).

According to a preferred embodiment of the present invention, the emission layer comprises an organic emitter host compound, wherein the organic emitter host compound comprises

- at least one condensed aromatic ring system consisting of 3 to 5 rings, and
- 3 to 7 aromatic or heteroaromatic rings, wherein one or more sub-groups of the aromatic and/or heteroaromatic rings may be condensed to form fused aromatic or heteroaromatic ring systems;

wherein the molecular weight Mw of the organic emitter host compound is in the range of ≥ 400 and ≤ 2000 g/mol.

According to a preferred embodiment of the present invention, the organic emitter host compound has the formula (V)

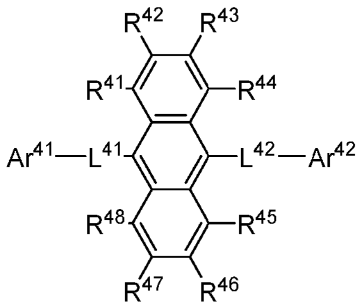 (V),

wherein

$Ar^{41}$ and $Ar^{42}$ are independently selected from substituted or unsubstituted $C_6$ to $C_{24}$ aryl, substituted or unsubstituted $C_3$ to $C_{24}$ heteroaryl;
$L^{41}$ and $L^{42}$ are independently selected from a direct bond or substituted or unsubstituted $C_6$ to $C_{24}$ arylene, substituted or unsubstituted $C_3$ to $C_{24}$ heteroarylene;
$R^{41}$ to $R^{48}$ are independently selected from H, D, substituted or unsubstituted $C_1$ to $C_{12}$ alkyl, substituted or unsubstituted $C_6$ to $C_{19}$ aryl, substituted or unsubstituted $C_3$ to $C_{12}$ heteroaryl;
wherein
the substituents on $Ar^{41}$, $Ar^{42}$, $L^{41}$, $L^{42}$, $R^{41}$ to $R^{48}$ are independently selected from D, $C_6$ to $C_{10}$ aryl, $C_3$ to $C_9$ heteroaryl, $C_1$ to $C_6$ alkyl, $C_1$ to $C_6$ alkoxy, $C_3$ to $C_6$ branched alkyl, $C_3$ to $C_6$ cyclic alkyl, $C_3$ to $C_6$ branched alkoxy, $C_3$ to $C_6$ cyclic alkoxy, partially or perfluorinated $C_1$ to $C_{16}$ alkyl, partially or perfluorinated $C_1$ to $C_{16}$ alkoxy, partially or perdeuterated $C_1$ to $C_6$ alkyl, partially or perdeuterated $C_1$ to $C_6$ alkoxy, halogen, F or CN.

According to a preferred embodiment of the present invention, the organic emitter host and/or compound of formula (V) is selected from formulae (BH1) to (BH13):

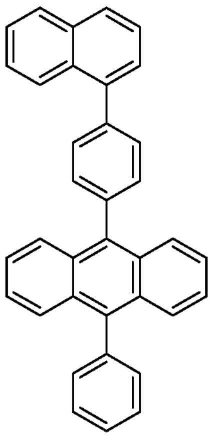 (BH1), 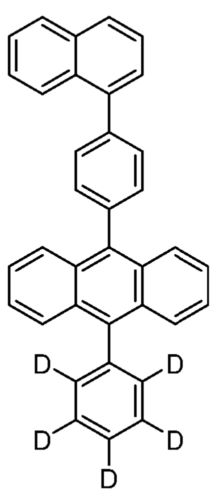 (BH2), 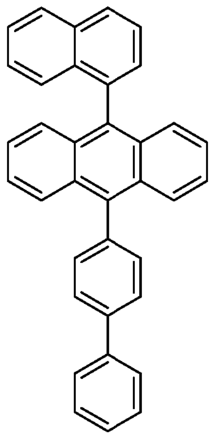 (BH3), 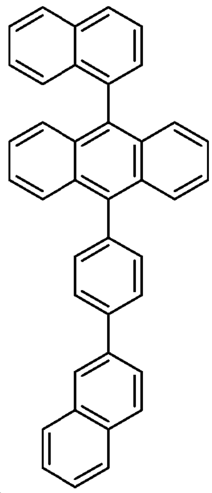 (BH4),

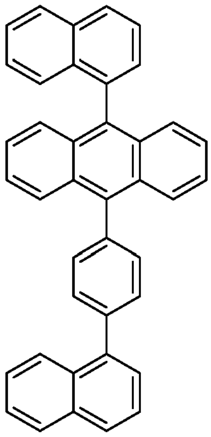 (BH5), 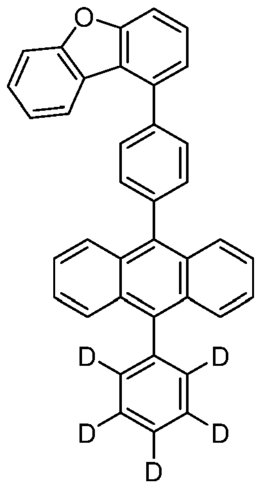 (BH6), 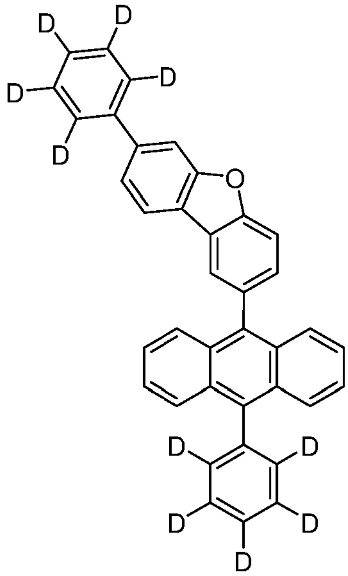 (BH7),

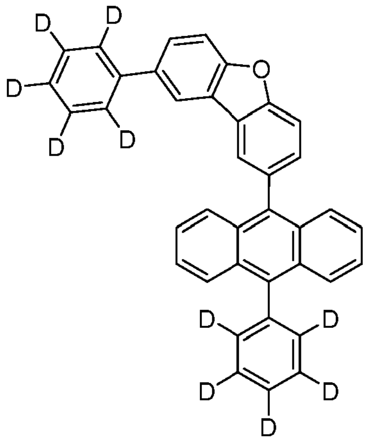 (BH8), 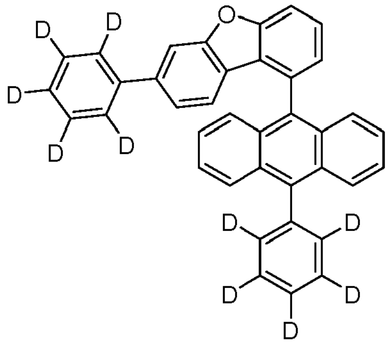 (BH9),

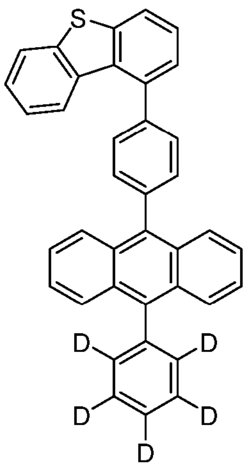 (BH10), 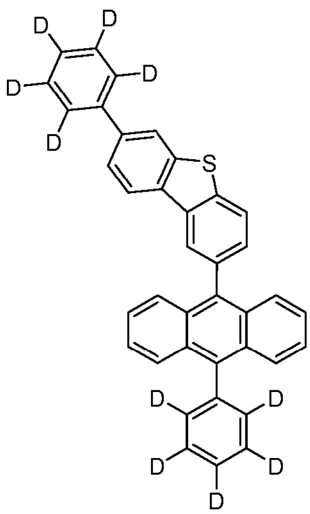 (BH11),

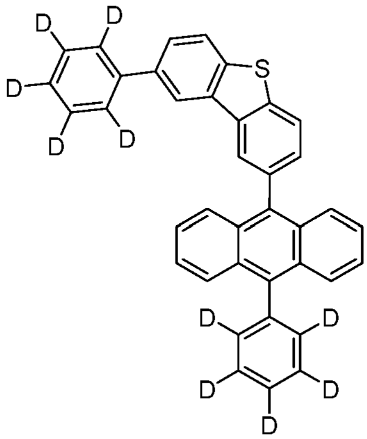 (BH12), 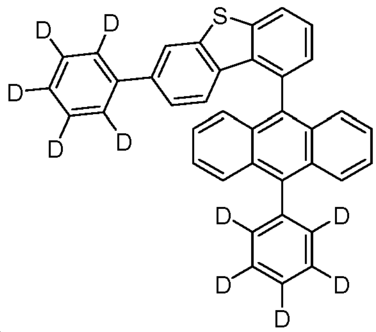 (BH13).

According to a preferred embodiment of the present invention, the emission layer comprises a light-emitting dopant of formula (IV) and an organic emitter host of formula (V).

According to a preferred embodiment of the present invention, the organic semiconductor layer comprises a compound of Formula (I) and a compound of formula (IIIa) or formula (IIIb), the hole transport layer comprises a compound of formula (IIIa) or formula (IIIb), preferably the organic semiconductor layer and the hole transport layer comprise the same compound of formula (IIIa) or formula (IIIb) and the emission layer comprises a light-emitting dopant of formula (IV) and an organic emitter host of formula (V);
wherein the organic semiconductor layer is arranged between the anode layer and the hole transport layer, the hole transport layer is arranged between the organic semiconductor layer and the emission layer, and the emission layer is arranged between the hole transport layer and the cathode layer.

According to a preferred embodiment of the present invention, the organic semiconductor layer comprises a compound of Formula (I) and a compound of formula (IIIa) or formula (IIIb), the hole transport layer comprises a compound of formula (IIIa) or formula (IIIb), preferably the organic semiconductor layer and the hole transport layer comprise the same compound of formula (IIIa) or formula (IIIb) and the emission layer comprises a light-emitting dopant of formula (IV) and an organic emitter host of formula (V);

wherein the organic semiconductor layer is arranged between the anode layer and the hole transport layer, the hole transport layer is arranged between the organic semiconductor layer and the emission layer, and the emission layer is arranged between the hole transport layer and the cathode layer;
wherein the anode layer may comprise a first anode sub-layer comprising Ag or Au having a thickness of 100 to 150 nm, a second anode sub-layer comprising or consisting of a transparent conductive oxide having a thickness of 3 to 20 nm and a third anode sub-layer comprising or consisting of a transparent conductive oxide having a thickness of 3 to 20 nm, preferably the transparent conductive oxide is selected from ITO or IZO.

*Hole blocking layer (HBL)*

A hole blocking layer (HBL) may be formed on the EML, by using vacuum deposition, spin coating, slot-die coating, printing, casting, LB deposition, or the like, in order to prevent the diffusion of holes into the ETL. When the EML comprises a phosphorescent dopant, the HBL may have also a triplet exciton blocking function.

The HBL may also be named auxiliary ETL or a-ETL.

When the HBL is formed using vacuum deposition or spin coating, the conditions for deposition and coating may be similar to those for the formation of the HIL. However, the conditions for deposition and coating may vary, according to the compound that is used to form the HBL. Any compound that is commonly used to form a HBL may be used. Examples of compounds for forming the HBL include oxadiazole derivatives, triazole derivatives, phenanthroline derivatives and triazine derivatives.

The HBL may have a thickness in the range from about 5 nm to about 100 nm, for example, from about 10 nm to about 30 nm. When the thickness of the HBL is within this range, the HBL may have excellent hole-blocking properties, without a substantial penalty in driving voltage.

*Electron transport layer (ETL)*

The organic electronic device according to the present invention may further comprise an electron transport layer (ETL), wherein the electron transport layer is arranged between the anode layer and the cathode layer, preferably between

the organic semiconductor layer and the cathode layer.

According to another embodiment of the present invention, the electron transport layer may further comprise an azine compound, preferably a triazine compound.

In one embodiment, the electron transport layer may further comprise a dopant selected from an alkali organic complex, preferably LiQ.

The thickness of the ETL may be in the range from about 15 nm to about 50 nm, for example, in the range from about 20 nm to about 40 nm. When the thickness of the EII, is within this range, the ETL may have satisfactory electron-injecting properties, without a substantial penalty in driving voltage.

According to another embodiment of the present invention, the organic electronic device may further comprise a hole blocking layer and an electron transport layer, wherein the hole blocking layer and the electron transport layer comprise an azine compound. Preferably, the azine compound is a triazine compound.

*Electron injection layer (EIL)*

An optional EIL, which may facilitates injection of electrons from the cathode, may be formed on the ETL, preferably directly on the electron transport layer. Examples of materials for forming the EII, include lithium 8-hydroxyquinolinolate (LiQ), LiF, NaCl, CsF, Li2O, BaO, Ca, Ba, Yb, Mg which are known in the art. Deposition and coating conditions for forming the EII, are similar to those for formation of the HIL, although the deposition and coating conditions may vary, according to the material that is used to form the EIL.

The thickness of the EII, may be in the range from about 0.1 nm to about 10 nm, for example, in the range from about 0.5 nm to about 9 nm. When the thickness of the EII, is within this range, the EII, may have satisfactory electron-injecting properties, without a substantial penalty in driving voltage.

*Cathode layer*

The cathode layer is formed on the ETL or optional EIL. The cathode layer may be formed of a metal, an alloy, an electrically conductive compound, or a mixture thereof. The cathode layer may have a low work function. For example, the cathode layer may be formed of lithium (Li), magnesium (Mg), aluminum (Al), aluminum (Al)-lithium (Li), calcium (Ca), barium (Ba), ytterbium (Yb), magnesium (Mg)-indium (In), magnesium (Mg)-silver (Ag), or the like. Alternatively, the cathode layer may be formed of a transparent conductive oxide, such as ITO or IZO.

The thickness of the cathode layer may be in the range from about 5 nm to about 1000 nm, for example, in the range from about 10 nm to about 100 nm. When the thickness of the cathode layer is in the range from about 5 nm to about 50 nm, the cathode layer may be transparent or semitransparent even if formed from a metal or metal alloy.

It is to be understood that the cathode layer is not part of an electron injection layer or the electron transport layer.

*Organic light-emitting diode (OLED)*

The organic electronic device according to the invention may be an organic light-emitting device.

According to one aspect of the present invention, there is provided an organic light-emitting diode (OLED) comprising: a substrate; an anode layer formed on the substrate; an organic semiconductor layer comprising compound of Formula (I), a hole transport layer, an emission layer, an electron transport layer and a cathode layer.

According to another aspect of the present invention, there is provided an OLED comprising: a substrate; an anode layer formed on the substrate; an organic semiconductor layer comprising a compound of Formula (I), a hole transport layer, an electron blocking layer, an emission layer, a hole blocking layer, an electron transport layer and a cathode layer.

According to another aspect of the present invention, there is provided an OLED comprising: a substrate; an anode layer formed on the substrate; an organic semiconductor layer comprising a compound of Formula (I), a hole transport layer, an electron blocking layer, an emission layer, a hole blocking layer, an electron transport layer, an electron injection layer, and a cathode layer.

According to various embodiments of the present invention, there may be provided OLEDs layers arranged between the above mentioned layers, on the substrate or on the top layer.

*Organic electronic device*

The organic electronic device according to the invention may be a light emitting device, or a photovoltaic cell, and preferably a light emitting device.

According to another aspect of the present invention, there is provided a method of manufacturing an organic electronic device, the method using:

- at least one deposition source, preferably two deposition sources and more preferred at least three deposition sources.

The methods for deposition that can be suitable comprise:

- deposition via vacuum thermal evaporation;
- deposition via solution processing, preferably the processing is selected from spin-coating, printing, casting; and/or
- slot-die coating.

According to various embodiments of the present invention, there is provided a method using:

- a first deposition source to release the compound of Formula (I) according to the invention, and
- a second deposition source to release the substantially covalent matrix compound;

the method comprising the steps of forming the organic semiconductor layer; whereby for an organic light-emitting diode (OLED):

- the organic semiconductor layer is formed by releasing the compound of Formula (I) according to the invention from the first deposition source and the substantially covalent matrix compound from the second deposition source.

According to various embodiments of the present invention, the method may further include forming on the anode layer, at least one layer selected from the group consisting of forming a hole transport layer or forming a hole blocking layer, and an emission layer between the anode layer and the first electron transport layer.

According to various embodiments of the present invention, the method may further include the steps for forming an organic light-emitting diode (OLED), wherein

- on a substrate an anode layer is formed,
- on the anode layer an organic semiconductor layer comprising a compound of Formula (I) is formed,
- on the organic semiconductor layer comprising a compound of Formula (I) a hole transport layer is formed,
- on the hole transport layer an emission layer is formed,
- on the emission layer an electron transport layer is formed, optionally a hole blocking layer is formed on the emission layer,
- and finally a cathode layer is formed,
- optional a hole blocking layer is formed in that order between the first anode layer and the emission layer,
- optional an electron injection layer is formed between the electron transport layer and the cathode layer.

According to various embodiments, the OLED may have the following layer structure, wherein the layers having the following order:
anode layer, organic semiconductor layer comprising a compound of Formula (I) according to the invention, first hole transport layer, second hole transport layer, emission layer, optional hole blocking layer, electron transport layer, optional electron injection layer, and cathode layer.

According to another aspect of the invention, it is provided an electronic device comprising at least one organic light emitting device according to any embodiment described throughout this application, preferably, the electronic device comprises the organic light emitting diode in one of embodiments described throughout this application. More preferably, the electronic device is a display device.

Hereinafter, the embodiments are illustrated in more detail with reference to examples. However, the present disclosure is not limited to the following examples. Reference will now be made in detail to the exemplary aspects.

## Description of the Drawings

The aforementioned components, as well as the claimed components and the components to be used in accordance with the invention in the described embodiments, are not subject to any special exceptions with respect to their size, shape, material selection and technical concept such that the selection criteria known in the pertinent field can be applied without limitations.

Additional details, characteristics and advantages of the object of the invention are disclosed in the dependent claims and the following description of the respective figures which in an exemplary fashion show preferred embodiments according to the invention. Any embodiment does not necessarily represent the full scope of the invention, however, and reference is made therefore to the claims and herein for interpreting the scope of the invention. It is to be understood that

both the foregoing general description and the following detailed description are exemplary and explanatory only and are intended to provide further explanation of the present invention as claimed.

Figures 1 to 6

FIG. 1 is a schematic sectional view of an organic electronic device, according to an exemplary embodiment of the present invention;
FIG. 2 is a schematic sectional view of an organic light-emitting diode (OLED), according to an exemplary embodiment of the present invention;
FIG. 3 is a schematic sectional view of an organic light-emitting diode (OLED), according to an exemplary embodiment of the present invention.
FIG. 4 is a schematic sectional view of an organic light-emitting diode (OLED), according to an exemplary embodiment of the present invention;
FIG. 5 is a schematic sectional view of an organic light-emitting diode (OLED), according to an exemplary embodiment of the present invention.
FIG. 6 is a schematic sectional view of an organic light-emitting diode (OLED), according to an exemplary embodiment of the present invention.

Hereinafter, the figures 1 to 6 are illustrated in more detail with reference to examples. However, the present disclosure is not limited to the following figures.

Herein, when a first element is referred to as being formed or disposed "on" or "onto" a second element, the first element can be disposed directly on the second element, or one or more other elements may be disposed there between. When a first element is referred to as being formed or disposed "directly on" or "directly onto" a second element, no other elements are disposed there between.

FIG. 1 is a schematic sectional view of an organic electronic device 101, according to an exemplary embodiment of the present invention. The organic electronic device 101 includes a substrate (110), an anode layer (120), an organic semiconductor layer comprising a compound of Formula (I) (130), a photoactive layer (PAL) (151) and a cathode layer (190).

FIG. 2 is a schematic sectional view of an organic light-emitting diode (OLED) 100, according to an exemplary embodiment of the present invention. The OLED 100 includes a substrate (110), an anode layer (120), an organic semiconductor layer comprising a compound of Formula (I) (130), an emission layer (EML) (150) and a cathode layer (190).

FIG. 3 is a schematic sectional view of an organic light-emitting diode (OLED) 100, according to an exemplary embodiment of the present invention. The OLED 100 includes a substrate (110), an anode layer (120), an organic semiconductor layer comprising a compound of Formula (I) (130), a hole transport layer (HTL) (140), an emission layer (EML) (150), an electron transport layer (ETL) (160) and a cathode layer (190).

FIG. 4 is a schematic sectional view of an organic light-emitting diode (OLED) 100, according to an exemplary embodiment of the present invention. The OLED 100 includes a substrate (110), an anode layer (120), an organic semiconductor layer comprising a compound of Formula (I) (130), a hole transport layer (HTL) (140), an electron blocking layer (EBL) (145), an emission layer (EML) (150), a hole blocking layer (HBL) (155), an electron transport layer (ETL) (160), an optional electron injection layer (EIL) (180), and a cathode layer (190).

FIG. 5 is a schematic sectional view of an organic light-emitting diode (OLED) 100, according to an exemplary embodiment of the present invention. The OLED 100 includes a substrate (110), an anode layer (120) that comprises a first anode sub-layer (121) and a second anode sub-layer (122), an organic semiconductor layer comprising compound of Formula (I) (130), a hole transport layer (HTL) (140), an electron blocking layer (EBL) (145), an emission layer (EML) (150), a hole blocking layer (EBL) (155), an electron transport layer (ETL) (160) and a cathode layer (190).

FIG. 6 is a schematic sectional view of an organic light-emitting diode (OLED) 100, according to an exemplary embodiment of the present invention. The OLED 100 includes a substrate (110), an anode layer (120) that comprises a first anode sub-layer (121), a second anode sub-layer (122) and a third anode sub-layer (123), an organic semiconductor layer comprising compound of Formula (I) (130), a hole transport layer (HTL) (140), an electron blocking layer (EBL) (145), an emission layer (EML) (150), a hole blocking layer (EBL) (155), an electron transport layer (ETL) (160) and a cathode layer (190). The layers are disposed exactly in the order as mentioned before.

In the description above the method of manufacture an organic electronic device 101 of the present invention is for example started with a substrate (110) onto which an anode layer (120) is formed, on the anode layer (120), an organic semiconductor layer comprising compound of Formula (I) (130), a photoactive layer (151) and a cathode layer 190 are formed, exactly in that order or exactly the other way around.

In the description above the method of manufacture an OLED 100 of the present invention is started with a substrate (110) onto which an anode layer (120) is formed, on the anode layer (120), an organic semiconductor layer comprising compound of Formula (I) (130), optional a hole transport layer (140), optional an electron blocking layer (145), an emission layer (150), optional a hole blocking layer (155), optional an electron transport layer (160), optional an electron injection layer (180), and a cathode layer (190) are formed, exactly in that order or exactly the other way around.
The organic semiconductor layer comprising a compound of Formula (I) (130) can be a hole injection layer.
While not shown in Fig. 1 to Fig. 6, a capping layer and/or a sealing layer may further be formed on the cathode layer (190), in order to seal the OLEDs 100. In addition, various other modifications may be applied thereto.
Hereinafter, one or more exemplary embodiments of the present invention will be described in detail with, reference to the following examples. However, these examples are not intended to limit the purpose and scope of the one or more exemplary embodiments of the present invention.

## Detailed discussion

The invention is furthermore illustrated by the following examples which are illustrative only and non-binding.
Compounds of Formula (I) may be prepared by methods known in the art and as described below.

Synthesis of 5,6-difluoro-3-hydroxy-2-(pyridin-2-yl)-1H-inden-1-one

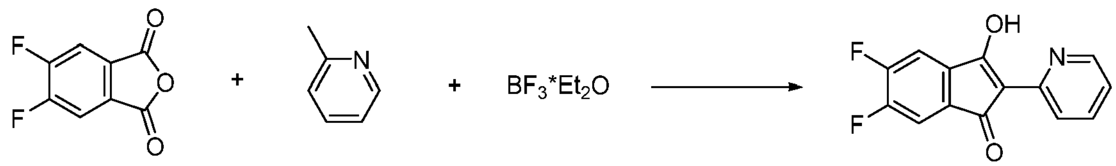


5.22g (27,41mmol) of bortrifluoride etherate was added to a dried pressure flask under argon atmosphere and 17.22g (93.54mmol) of 5,6-difluoroisobenzofuran-1,3-dione and 40ml (42.37g, 0.45mol) of 2-picoline were added. The pressure flask was closed and the reaction mixture was heated to 180°C overnight. After cooling to room temperature 100ml saturated aqueous sodium bicarbonate solution were added and the resulting suspension was filtered and washed with water and dried in vacuum. 25ml ethanol was added to the crude product and heated to reflux. The hot suspension was filtered and the solid washed with hot ethanol. After drying in vacuum 10.1g (42%) product was obtained as a yellow ochre solid.

Synthesis of bis((5,6-difluoro-1-oxo-2-(pyridin-2-yl)-1H-inden-3-yl)oxy)copper (M28)

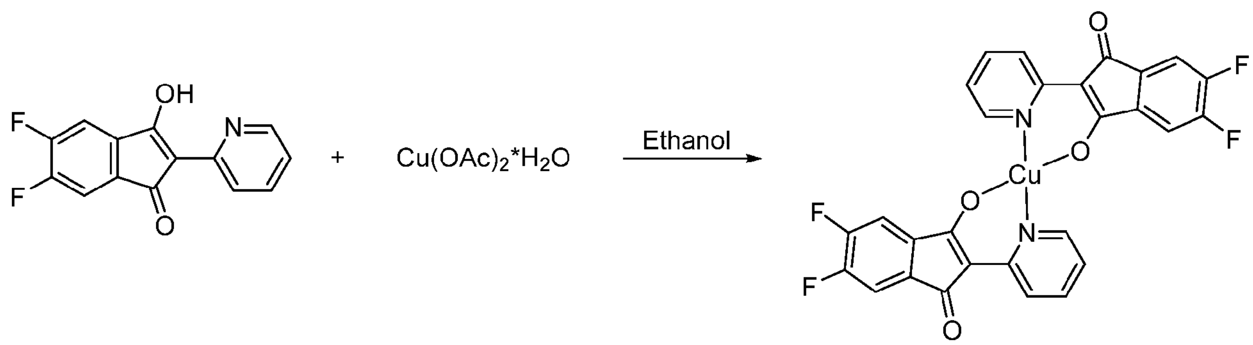


4.0g (15.43mmol) of 5,6-difluoro-3-hydroxy-2-(pyridin-2-yl)-1H-inden-1-one was suspended 40mL ethanol. 1.54g (7.72mmol) Cu(OAc)2*H2O was added and the mixture was heated to reflux overnight. The hot suspension was filtered and washed with 20ml warm ethanol and then hexane. The product was dried in vacuum to obtain 4.37g (98%) dark brown solid.

Synthesis of bis((5,6-difluoro-1-oxo-2-(pyridin-2-yl)-1H-inden-3-yl)oxy)zinc (M7)

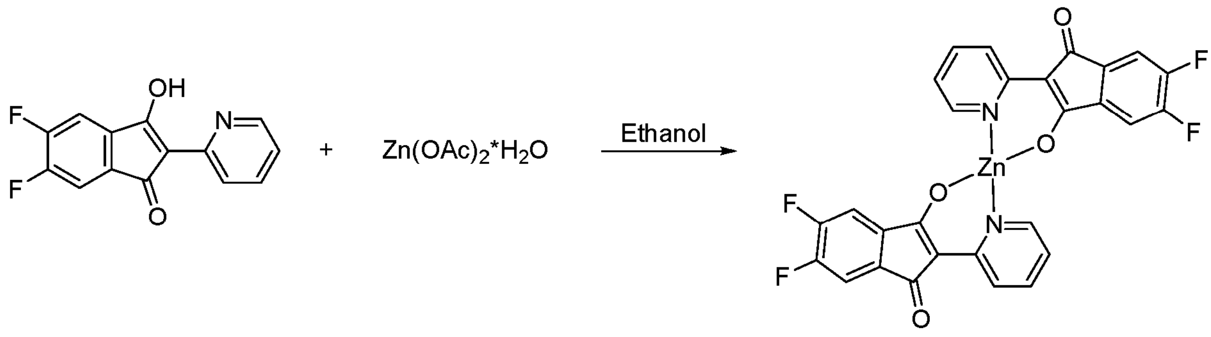


4.39g (16.94mmol) of 5,6-difluoro-3-hydroxy-2-(pyridin-2-yl)-1H-inden-1-one was suspended 45mL ethanol. 1.86g (8.47mmol) Zn(OAc)2*2H2O was added and the mixture was heated to reflux overnight. The hot suspension was filtered and washed with 20ml warm ethanol. The product was dried in vacuum to obtain 4.77g (97%) orange solid.

Synthesis of 5,6-difluoro-2-(5-fluoropyridin-2-yl)-3-hydroxy-1H-inden-1-one

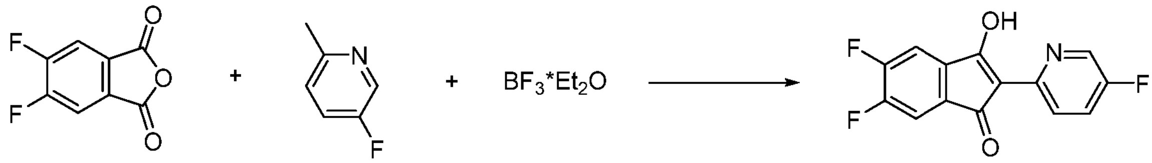

1.57g (10.86mmol) of bortrifluoride etherate was added to a dried pressure flask under argon atmosphere and 5.0g (27.16mmol) of 5,6-difluoroisobenzofuran-1,3-dione and 8ml (8.76g, 78.83mmol) of 5-fluoro-2methylpyridin were added. The pressure flask was closed and the reaction mixture was heated to 190°C overnight. After cooling to room temperature saturated aqueous sodium bicarbonate solution were added. The resulting suspension was filtered and washed with water and dried in vacuum. The crude product was dissolved in dichloromethane, filtered over silica gel and eluted with a mixture of dichloromethane and methanol. The solvent was removed and the residue was heated in ethanol under reflux. The solid was filtered off, washed with ethanol and dried in vacuum to obtain 2.17g (29%) product as yellow brownish solid.

Synthesis of bis((5,6-difluoro-2-(5 -fluoropyridin-2-yl)-1-oxo-1H-inden-3-yl)oxy)zinc (M8)

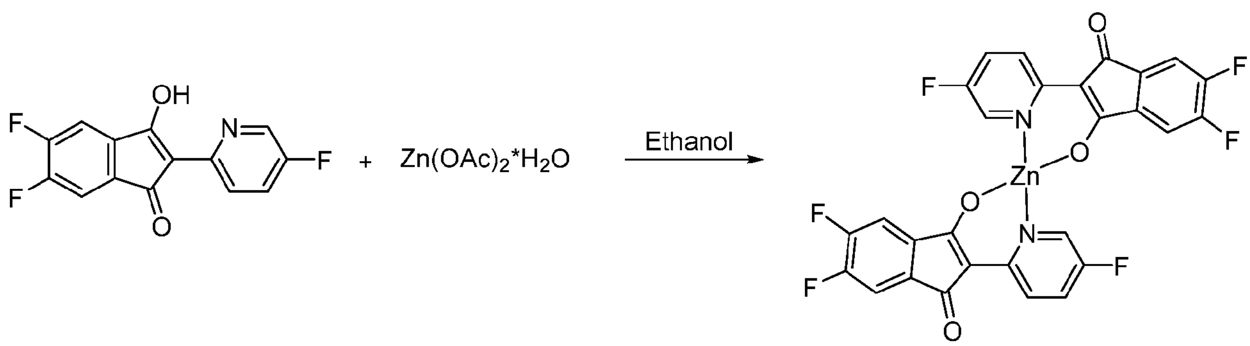


1.89g (6.82mmol) of 5,6-difluoro-2-(5-fluoropyridin-2-yl)-3-hydroxy-1H-inden-1-one was suspended 20mL ethanol. 0.75g (3.41mmol) Zn(OAc)2*2H2O was added and the mixture was heated to reflux overnight. The hot suspension was filtered and washed with warm ethanol. The product was dried in vacuum to obtain 1.98g (94%) dark

red solid.

Calculated LUMP of compounds of formula (I) and comparative compounds

The energy of the lowest unoccupied molecular orbital (LUMO) of compounds of formula (I) and comparative compounds was calculated with the program package ORCA V5.0.3 (Max Planck Institute für Kohlenforschung, Kaiser Wilhelm Platz 1, 45470, Muelheim/Ruhr, Germany) and WEASEL 1.9.2 (FAccTs GmbH, Rolandstrasse 67, 50677 Köln, Germany). The LUMO energy levels of the molecular structures are determined by applying the hybrid functional B3LYP with a Def2-TZVP basis set and the Stuttgart/Dresden (SDD) effective core potential (ECP) for the metals from the optimized geometries obtained by applying the functional BP86 with a Def2-SVP basis set the Stuttgart/Dresden (SDD) effective core potential (ECP) for the metals. For materials containing a Ce(IV) cation, the LUMO energy levels of the molecular structures are determined by applying the hybrid functional B3LYP with a SARC-ZORA-TZVP basis set for the metals and a ZORA-Def2-TZVP basis set for all other atoms from the optimized geometries obtained by applying the functional BP86 with a SARC-ZORA-TZVP basis set for the metals and a ZORA-Def2-SVP basis set for all other atoms. All the calculations were performed in the gas phase. All relativistic calculations were performed by applying the zero order regular approximation (ZORA). If more than one conformation is viable, the conformation with the lowest total energy is selected. Depending on the metal cation different multiplicities may be applied. For the following metal cations the multiplicity is shown in brackets: $Zn2+$ (singlet), $Cu2+$ (dublet), $Mn2+$ (sextet), $Ce4+$ (singlet). The LUMO values in Table 1 was calculated by this method unless noted otherwise.

According to an embodiment of compound of formula (I), the LUMO of compound of formula (I) is selected in the range of ≤ -0.5 eV and ≥ -6.5 eV, preferably ≤ -1.0 eV and ≥ -6 eV, more preferred ≤ -1.5 eV and ≥ -5.8 eV; and even more preferred ≤ -2.0 eV and ≥ -5.8 eV; wherein the LUMO is calculated by the method described above.

Melting point

The melting point (mp) is determined as peak temperatures from the DSC curves of the above TGA-DSC measurement or from separate DSC measurements (Mettler Toledo DSC822e, heating of samples from room temperature to completeness of melting with heating rate 10 K/min under a stream of pure nitrogen. Sample amounts of 4 to 6 mg are placed in a 40 μL Mettler Toledo aluminum pan with lid, a <1 mm hole is pierced into the lid).

Glass transition temperature

The glass transition temperature (Tg) is measured under nitrogen and using a heating rate of 10 K per min in a Mettler Toledo DSC 822e differential scanning calorimeter as described in DIN EN ISO 11357, published in March 2010.

Decomposition temperature Tdec

The decomposition temperature Tdec is measured by loading a sample of 9 to 11 mg into a Mettler Toledo 100 μL aluminum pan without lid under nitrogen in a Mettler Toledo TGA-DSC 1 machine. The following heating program was used: 25°C isothermal for 3 min; 25°C to 600°C with 10 K/min.

The decomposition temperature was determined based on the onset of the decomposition in TGA.

The decomposition temperature indicates the temperature at which the compound decomposes. The higher the decomposition temperature the higher the thermal stability of a compound.

Rate onset temperature

The rate onset temperature (TRO) is determined by loading 100 mg compound into a VTE source. As VTE source a point source for organic materials may be used as supplied by Kurt J. Lesker Com-pany (www.lesker.com) or CreaPhys GmbH (http://www.creaphys.com). The VTE source is heated at a constant rate of 15 K/min at a pressure of less than 10-5 mbar and the temperature inside the source measured with a thermocouple. Evaporation of the compound is detected with a QCM detector which detects deposition of the compound on the quartz crystal of the detector. The deposition rate on the quartz crystal is measured in Ǻngstrom per second. To determine the rate onset temperature, the deposition rate is plotted against the VTE source temperature. The rate onset is the temperature at which noticeable deposition on the QCM detector occurs. For accurate results, the VTE source is heated and cooled three time and only results from the second and third run are used to determine the rate onset temperature.

To achieve good control over the evaporation rate of a compound, the rate onset temperature may be in the range of 200 to 350°C. If the rate onset temperature is substantially below 200° C the evaporation may be too rapid and therefore difficult to control. If the rate onset temperature is above 300° C the evaporation rate may be too low which may result in low

tact time and decomposition of the compound of formula (I) in VTE source may occur due to prolonged exposure to elevated temperatures.

The rate onset temperature is an indirect measure of the volatility of a compound. The higher the rate onset temperature the lower is the volatility of a compound.

In Table 2 are shown rate onset temperatures TRO for compounds of formula (I) and comparative compounds.

General procedure for fabrication of OLEDs

A glass substrate with an anode layer comprising a first anode sub-layer of 120 nm Ag, a second anode sub-layer of 8 nm ITO and a third anode sub-layer of 10 nm ITO was cut to a size of 50 mm x 50 mm x 0.7 mm, ultrasonically washed with water for 60 minutes and then with isopropanol for 20 minutes. The first anode sub-layer is arranged between the second anode sub-layer, and the third anode sub-layer. The liquid film was removed in a nitrogen stream, followed by plasma treatment. The plasma treatment was performed in an atmosphere comprising 97.6 vol.-% nitrogen and 2.4 vol.-% oxygen.

Then N-([1,1'-biphenyl]-4-yl)-9,9-dimethyl-N-(4-(9-phenyl-9H-carbazol-3-yl)phenyl)-9H-fluoren-2-amine ([1242056-42-3]) was vacuum deposited with a compound according to Table 4 (either an inventive compound or a comparative compound) to form a hole injection layer (p-HIL) having a thickness 10 nm.

Then -([1,1'-biphenyl]-4-yl)-9,9-dimethyl-N-(4-(9-phenyl-9H-carbazol-3-yl)phenyl)-9H-fluoren-2-amine ([1242056-42-3]) was vacuum deposited, to form a hole transport layer having a thickness of 128 nm
Then N-(4-(dibenzo[b,d]furan-4-yl)phenyl)-N-(4-(9-phenyl-9H-fluoren-9-yl)phenyl)-[1,1'-biphenyl]-4-amine ([1824678-59-2]) was vacuum deposited on the HTL, to form an electron blocking layer (EBL) having a thickness of 5 nm.

Then 97 vol.-% H09 (Sun Fine Chemicals, Korea) as EML host and 3 vol.-% BD200 (Sun Fine Chemicals, Korea) as fluorescent blue dopant were deposited on the EBL, to form a blue-emitting emission layer (EML) with a thickness of 20 nm.

Then, a hole blocking layer (HBL) having a thickness of 5 nm is formed on the emission layer by depositing 2-(3'-(9,9-dimethyl-9H-fluoren-2-yl)-[1,1'-biphenyl]-3-yl)-4,6-diphenyl-1,3,5-triazine ([1955543-57-3]).

Then, 50 vol.-% 4'-(4-(4-(4,6-diphenyl-1,3,5-triazin-2-yl)phenyl)naphthalen-1-yl)-[1,1'-biphenyl]-4-carbonitrile ([2032421-37-5]) and 50 vol.-% LiQ ([25387-93-3]or [850918-68-2]; 8-Hydroxyquinolinolato-lithium) were vacuum deposited on the HBL to form an electron transport layer (ETL) having a thickness of 31 nm.

Then Yb was evaporated at a rate of 0.01 to 1 Å/s at 10-7 mbar to form an electron injection layer (EIL) with a thickness of 2nm on the ETL.

Ag/Mg (90:10 vol%) is evaporated at a rate of 0.01 to 1 Å/s at 10-7 mbar to form a cathode with a thickness of 13 nm on the EIL.

Then, N-([1,1'-biphenyl]-4-yl)-9,9-dimethyl-N-(4-(9-phenyl-9H-carbazol-3-yl)phenyl)-9H-fluoren-2-amine ([1242056-42-3]) was vacuum deposited on the cathode layer to form a capping layer with a thickness of 75 nm.

The OLED stack is protected from ambient conditions by encapsulation of the device with a glass slide. Thereby, a cavity is formed, which includes a getter material for further protection.

To assess the performance of the inventive examples compared to the prior art, the current efficiency is measured at 20°C. The current-voltage characteristic is determined using a Keithley 2635 source measure unit, by sourcing a voltage in V and measuring the current in mA flowing through the device under test. The voltage applied to the device is varied in steps of 0.1V in the range between 0V and 10V. Likewise, the luminance-voltage characteristics and CIE coordinates are determined by measuring the luminance in $cd/m^2$ using an Instrument Systems CAS-140CT array spectrometer (calibrated by Deutsche Akkreditierungsstelle (DAkkS)) for each of the voltage values. The cd/A efficiency at 10 mA/cm2 is determined by interpolating the luminance-voltage and current-voltage characteristics, respectively.

In bottom emission devices, the emission is predominately Lambertian and quantified in percent external quantum efficiency (EQE). To determine the efficiency EQE in % the light output of the device is measured using a calibrated photodiode at 10 mA/cm2.

In top emission devices, the emission is forward directed, non-Lambertian and also highly dependent on the mirco-cavity. Therefore, the efficiency EQE will be higher compared to bottom emission devices. To determine the efficiency EQE in % the light output of the device is measured using a calibrated photodiode at 10 mA/cm2.

Lifetime LT of the device is measured at room temperature (20°C) or 60°C and 30 $mA/cm^2$, using a Keithley 2400 sourcemeter, and recorded in hours.

The brightness of the device is measured using a calibrated photo diode. The lifetime LT is defined as the time till the brightness of the device is reduced to 97 % of its initial value.

The increase in operating voltage $\Delta V$ is used as a measure of the operational voltage stability of the device. This increase is determined during the LT measurement and by subtracting the operating voltage after 1 hour after the start of operation of the device from the operating voltage after 100 hours.

$$\Delta V=[V(100\ h)-V(1h)].$$

The smaller the value of $\Delta V$ the better is the operating voltage stability.

Technical Effect of the invention

In **Table 1,** the difference between the LUMO energy level and HOMO energy of some compounds of formula (I) are shown.

Table 1: $E_{gap}$ values of compounds of formula (I)

| Referred to as: | Structure | $E_{gap}$ [eV] |
|---|---|---|
| M7 | 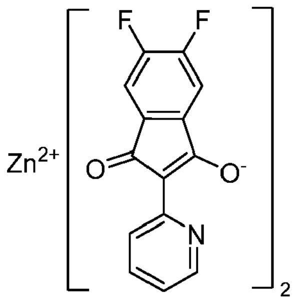 | 3.47 |
| M8 | 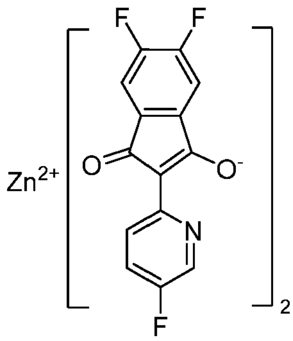 | 3.44 |

In Table 2 (cf below) are shown the calculated LUMO in electron volt of compounds of formula (I) and comparative compounds. The following multiplicities were applied dependent on metal cation: Zn2+ (singlet), Cu2+ (doublet), Mn2+ (sextet), Ce4+ (singlet).

As can be seen in **Table 2,** the LUMO of compounds of formula (I) is in the range suitable for organic electronic devices.

In **Table 3 (cf. below)** some properties of compounds of Formula (I) and comparative examples are shown.

The comparative compound CC-1 exhibits a LUMO of -3.98 eV, a melting point of 282°C, a glass transition temperature 71°C, a decomposition temperature of >282°C, and a rate onset temperature of 146°C. This rather low glass transition temperature leads to difficulties in processing in some applications.

The compound of Formula (I) M28 exhibit a LUMO of -4.06 eV, a glass transition temperature of 320°C, the decomposition temperature is improved to >470°C, and the rate onset temperature is improved to 289°C compared to comparative compound CC-1.

The compound of Formula (I) M7 exhibit a LUMO of -2.58 eV eV, the decomposition temperature is improved to >430°C, and the rate onset temperature is improved to 238°C compared to comparative compound CC-1.

The compound of Formula (I) M8 exhibit a LUMO of -2.70 eV, the decomposition temperature is improved to >430°C °C, and the rate onset temperature is improved to 238 °C compared to comparative compound CC-1.

In summary, in compounds of formula (I), the LUMO and/or thermal properties are improved compared to comparative compounds. In particular, the decomposition temperature and/or rate onset temperature are improved.

A high decomposition temperature and/or rate onset temperature may be beneficial for the preparation of organic electronic devices in vacuum via a thermal evaporation process.

In **Table 4** are shown characteristics of organic electronic devices comprising a semiconductor layer comprising compounds of formula (I) and comparative compound CC-1.

In comparative example 1 of Table 4, a comparative metal complex CC-1 was used.

The device according to the comparative example exhibits an operational voltage of 3.86 V, current efficiency (Ceff) of 133 cd/A, an external quantum efficiency (EQE) of 13.0%, a lifetime (LT97) of 75 h, and voltage rise over time (ΔU) of 0.964V.

In inventive example 1 of Table 4 an inventive metal complex M28 was used.

The device according to inventive example 1 exhibits a reduced operational voltage of 3.71 V, a higher current efficiency of 138 cd/A, a higher external quantum efficiency of 13.4%, a high lifetime of 76 h, and a reduced voltage rise over time of 0.152 V compared to comparative example 1.

In inventive example 2 of Table 4 an inventive metal complex M7 was used.

The device according to inventive example 1 exhibits a reduced operational voltage of 3.69 V, a higher current efficiency of 137 cd/A, a higher external quantum efficiency of 13.2%, a high lifetime of 75 h, and a reduced voltage rise over time of 0.066 V compared to comparative example 1.

In inventive example 3 of Table 4 an inventive metal complex M8 was used.

The device according to inventive example 1 exhibits a reduced operational voltage of 3.65 V, a higher current efficiency of 135 cd/A, a high external quantum efficiency of 13.1 %, a high lifetime of 71 h, and a reduced voltage rise over time of 0.085 V compared to comparative example 1.

The inventive example 1 to 3 of Table 4 using a metal complex according to the present invention all exhibit a lower operational voltage than the comparative example.

A lower operating voltage may be important for the battery life of organic electronic devices, in particular mobile devices.

The inventive examples 1 to 3 of Table 4 using a metal complex according to the present invention all higher external quantum efficiency and/or current efficiency than the comparative example.

A high efficiency may be beneficial for reduced power consumption and improved battery life, in particular in mobile devices.

The inventive examples 1 to 3 of Table 4 using a metal complex according to the present invention all exhibit a good lifetime.

A long lifetime may result in improved long-term stability of electronic devices.

The inventive examples 1 to 3 of Table 4 using a metal complex according to the present invention all exhibit a lower voltage rise over time than the comparative example.

A low voltage rise over time may result in improved long-term stability of electronic devices.

Table 2: Structure and LUMO values of some inventive compounds

| Referred to as: | Structure | LUMO [eV] | Referred to as: | Structure | LUMO [eV] |
|---|---|---|---|---|---|
| M1 | 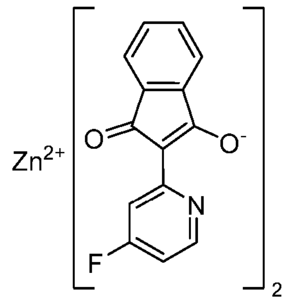 | -2.45 | M96 | 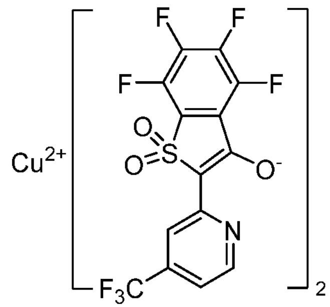 | -4.66 |
| M2 | 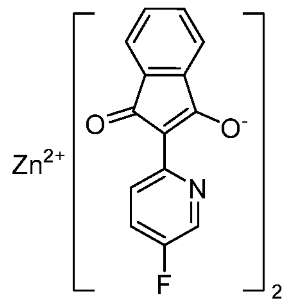 | -2.42 | M97 | 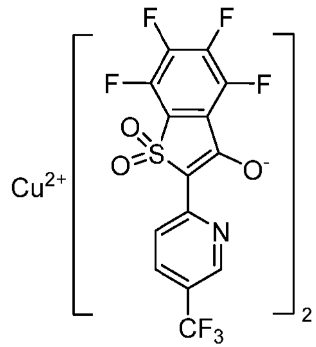 | -4.67 |
| M3 | 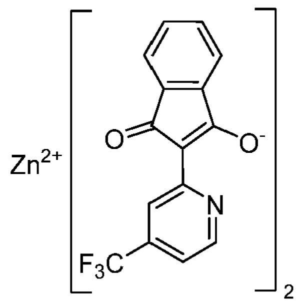 | -2.67 | M98 | 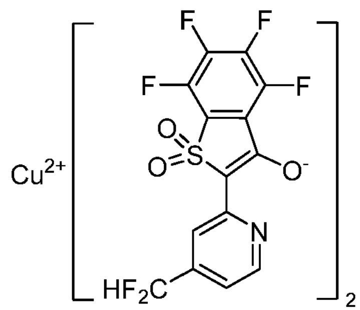 | -4.55 |

(continued)

| Referred to as: | Structure | LUMO [eV] | Referred to as: | Structure | LUMO [eV] |
|---|---|---|---|---|---|
| M4 | 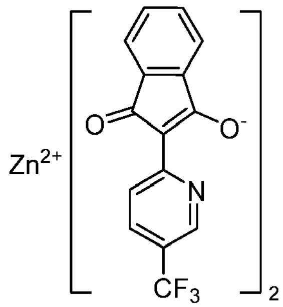 | -2.66 | M99 | 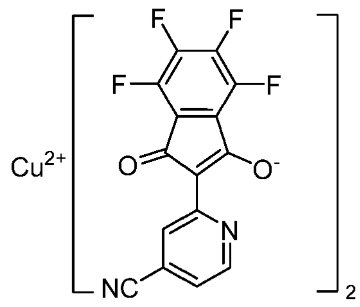 | -4.71 |
| M5 | 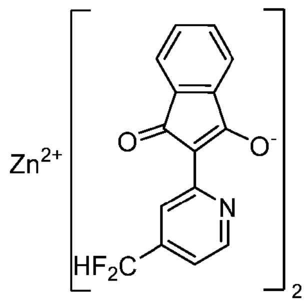 | -2.51 | M100 | 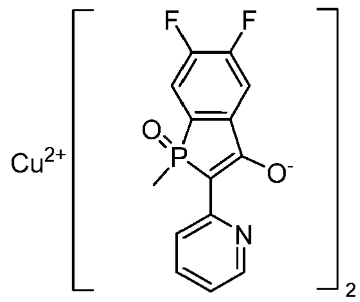 | -3.82 |
| M6 | 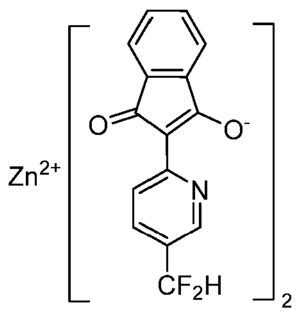 | -2.53 | M101 | 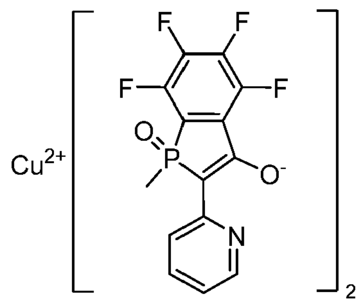 | -3.87 |

(continued)

| Referred to as: | Structure | LUMO [eV] | Referred to as: | Structure | LUMO [eV] |
|---|---|---|---|---|---|
| M7 | 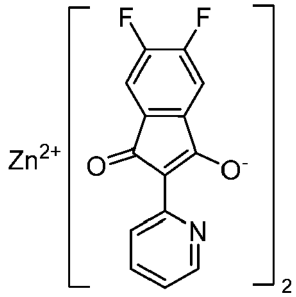 | -2.58 | M102 | 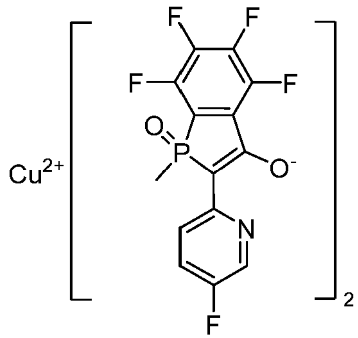 | -4.06 |
| M8 | 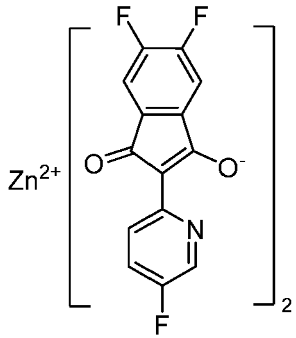 | -2.70 | M103 | 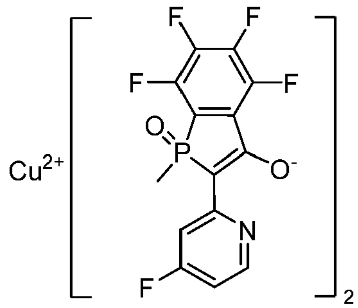 | -4.05 |
| M9 | 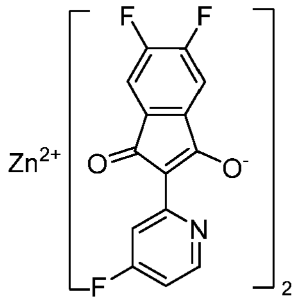 | -2.73 | M104 | 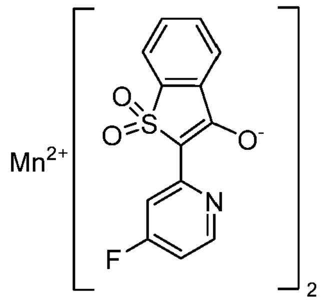 | -2.85 |

(continued)

| Referred to as: | Structure | LUMO [eV] | Referred to as: | Structure | LUMO [eV] |
|---|---|---|---|---|---|
| M10 | 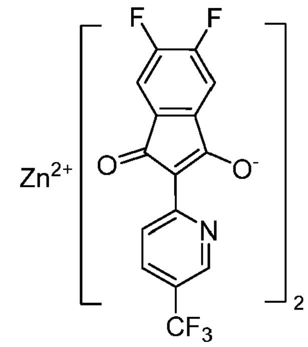 | -2.93 | M105 | 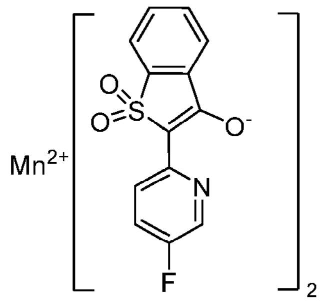 | -2.89 |
| M11 | 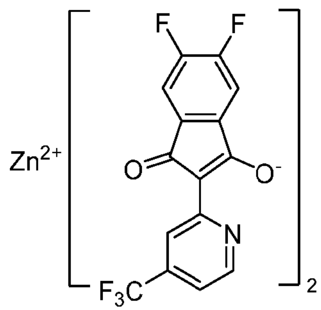 | -2.94 | M106 | 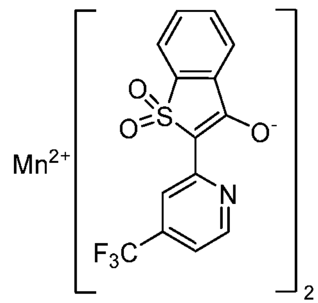 | -3.19 |
| M12 | 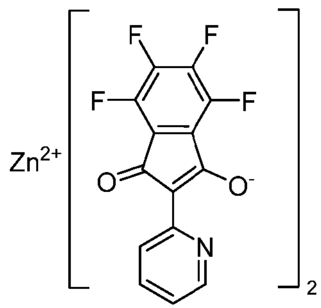 | -2.83 | M107 | 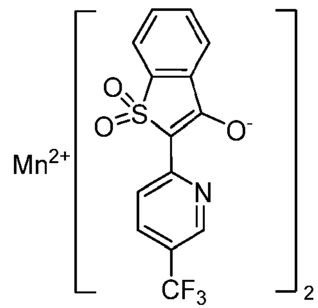 | -3.15 |

(continued)

| Referred to as: | Structure | LUMO [eV] | Referred to as: | Structure | LUMO [eV] |
|---|---|---|---|---|---|
| M13 | 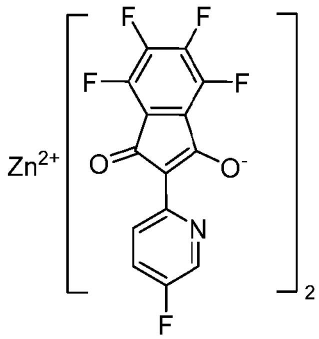 | -2.94 | M108 | 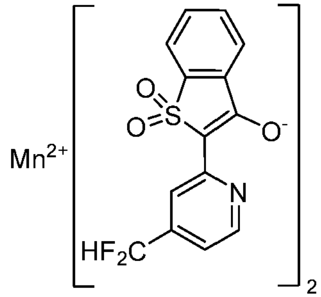 | -3.07 |
| M14 | 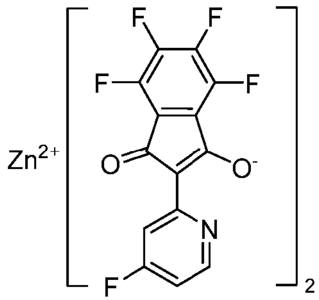 | -2.98 | M109 | 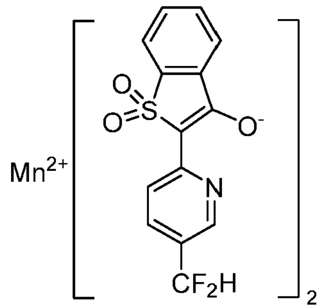 | -3.03 |
| M15 | 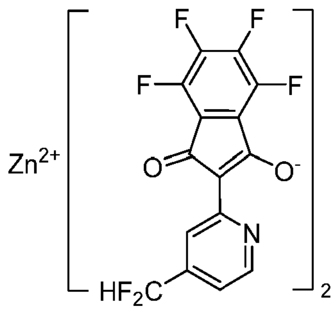 | -3.06 | M110 | 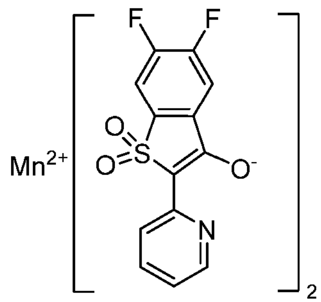 | -2.98 |

(continued)

| Referred to as: | Structure | LUMO [eV] | Referred to as: | Structure | LUMO [eV] |
|---|---|---|---|---|---|
| M16 | 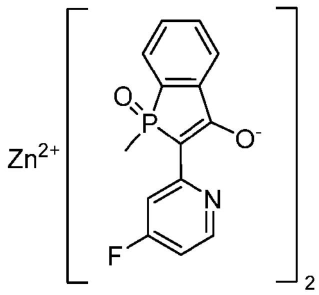 C5ON_LD-5_Zn | -2.25 | M111 | 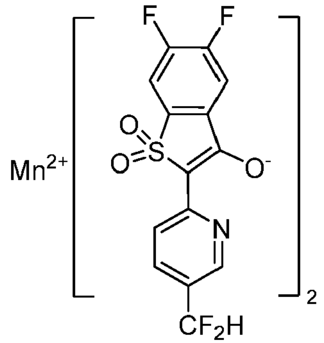 | -3.21 |
| M17 | 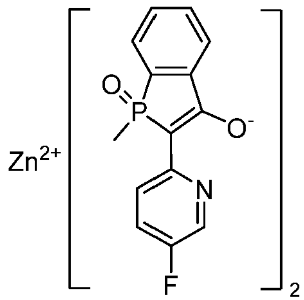 C5ON_LD-6_Zn | -2.26 | M112 | 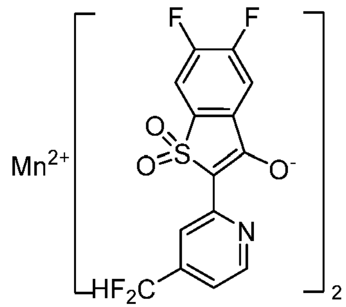 | -3.24 |
| M18 | 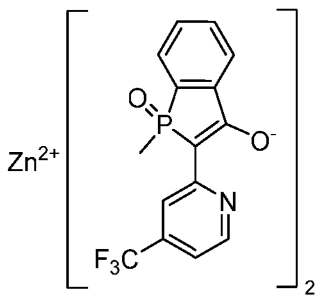 C5ON_LD-7_Zn | -2.60 | M113 | 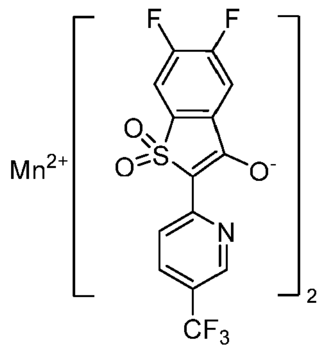 | -3.39 |

(continued)

| Referred to as: | Structure | LUMO [eV] | Referred to as: | Structure | LUMO [eV] |
|---|---|---|---|---|---|
| M19 | 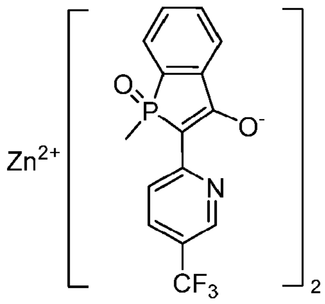 C5ON_LD-8_Zn | -2.56 | M114 | 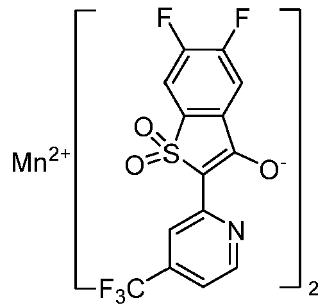 | -3.10 |
| M20 | 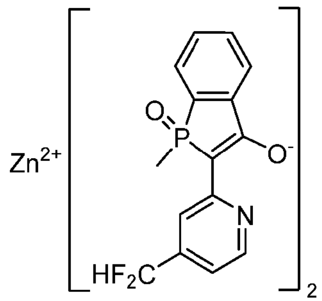 | -2.47 | M115 | 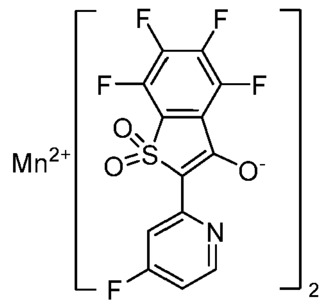 | -3.27 |
| M21 | 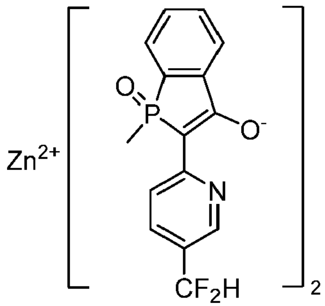 | -2.45 | M116 | 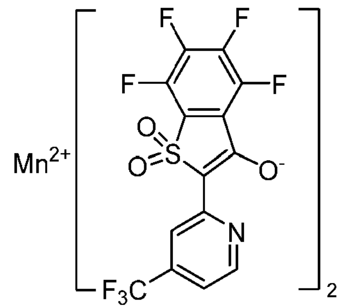 | -3.57 |

(continued)

| Referred to as: | Structure | LUMO [eV] | Referred to as: | Structure | LUMO [eV] |
|---|---|---|---|---|---|
| M22 | 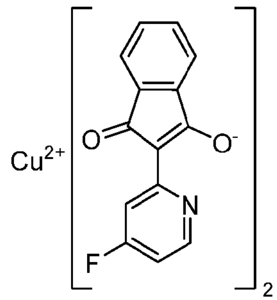 | -3.99 | M117 | 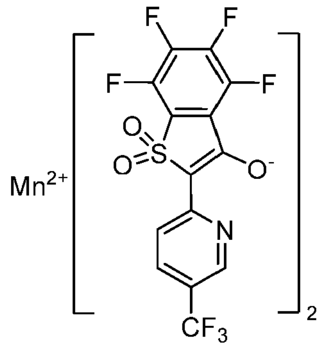 | -3.54 |
| M23 | 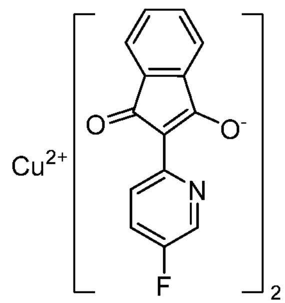 | -4.00 | M118 | 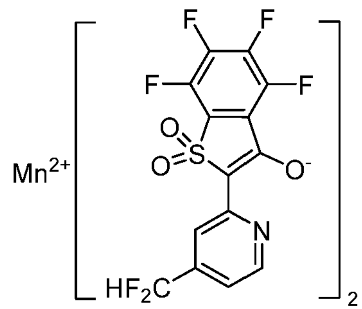 | -3.45 |
| M24 | 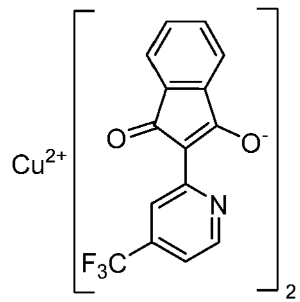 | -4.18 | M119 | 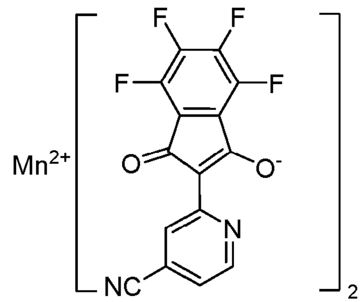 | -3.63 |

(continued)

| Referred to as: | Structure | LUMO [eV] | Referred to as: | Structure | LUMO [eV] |
|---|---|---|---|---|---|
| M25 | 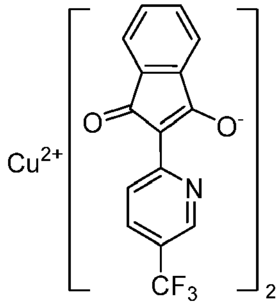 | -4.17 | M120 | 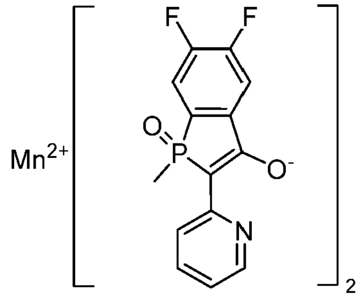 | -2.66 |
| M26 | 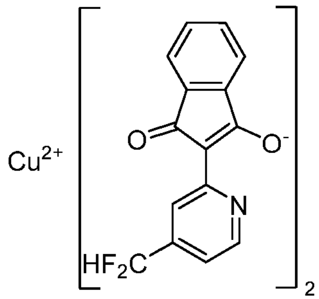 | -4.03 | M121 | 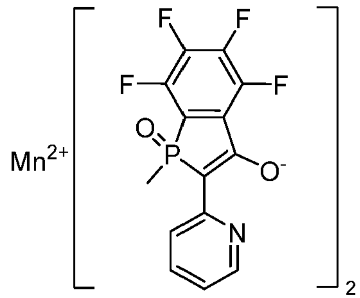 | -2.80 |
| M27 | 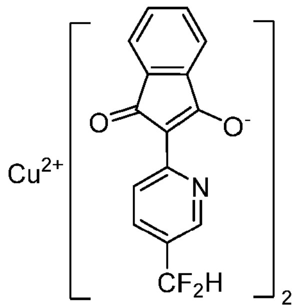 | -4.08 | M122 | 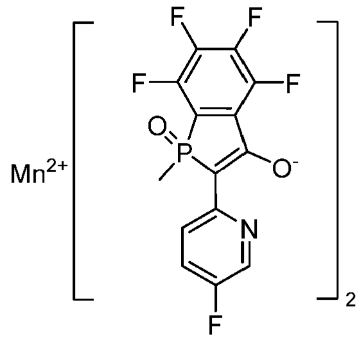 | -2.94 |

(continued)

| Referred to as: | Structure | LUMO [eV] | Referred to as: | Structure | LUMO [eV] |
|---|---|---|---|---|---|
| M28 | 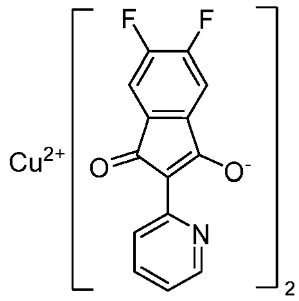 | -4.07 | M123 | 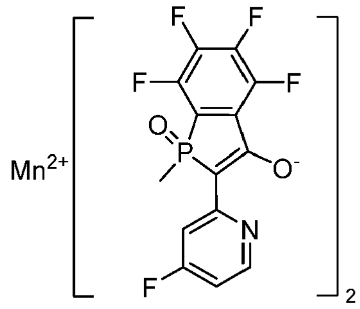 | -2.92 |
| M29 | 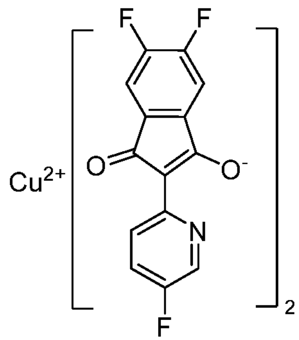 | -4.27 | M124 | 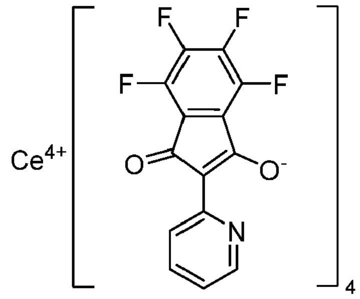 | -4.38 |
| M30 | 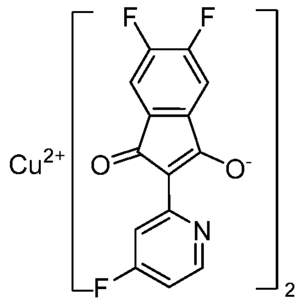 | -4.26 | M125 | 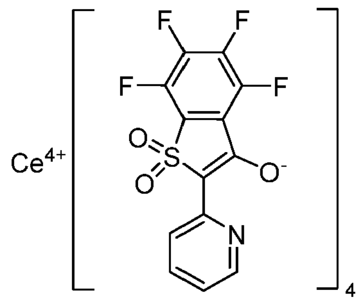 | -4.52 |

(continued)

| Referred to as: | Structure | LUMO [eV] | Referred to as: | Structure | LUMO [eV] |
|---|---|---|---|---|---|
| M31 | 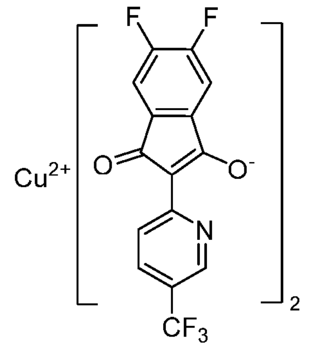 | -4.43 | **M126** | 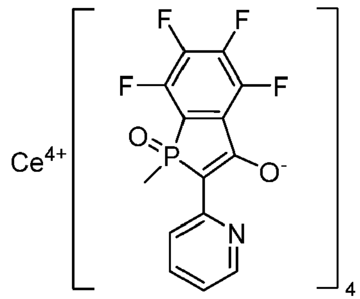 | -4.03 |
| M32 | 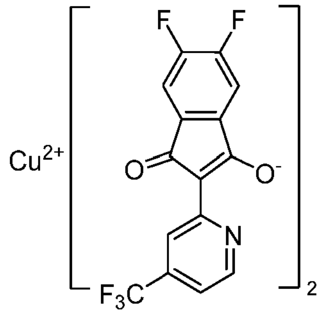 | -4.46 | **M189** | 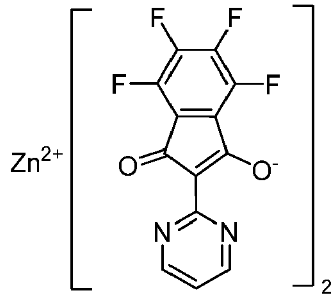 | -2.90 |
| M33 | 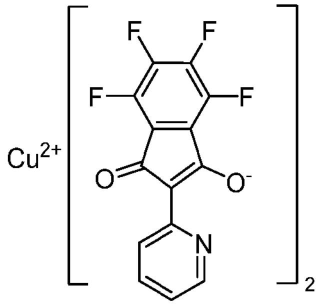 | -4.17 | M190 | 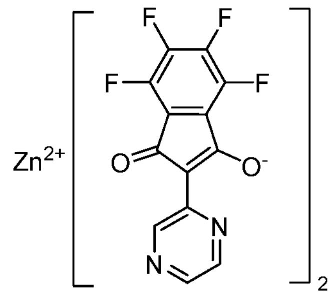 | -3.14 |

(continued)

| Referred to as: | Structure | LUMO [eV] | Referred to as: | Structure | LUMO [eV] |
|---|---|---|---|---|---|
| M34 | 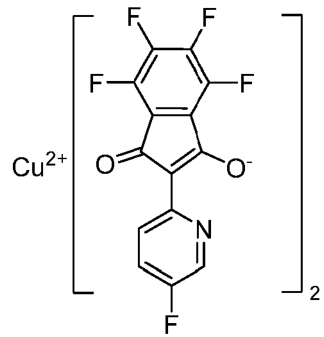 | -4.37 | M191 | 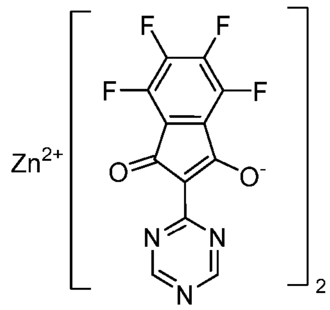 | -3.26 |
| M35 | 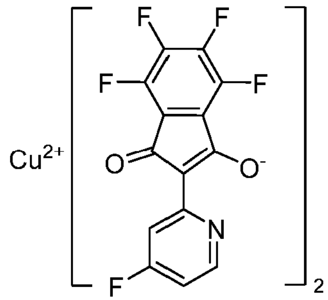 | -4.36 | M192 | 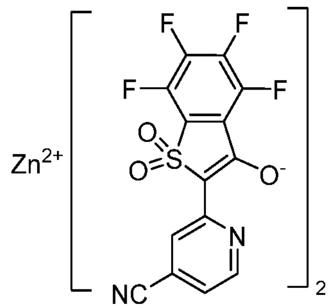 | -3.59 |
| M36 | 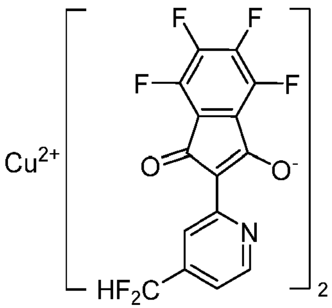 | -4.42 | M193 | 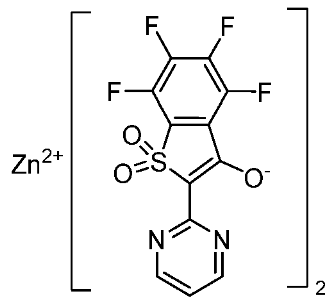 | -3.08 |

(continued)

| Referred to as: | Structure | LUMO [eV] | Referred to as: | Structure | LUMO [eV] |
|---|---|---|---|---|---|
| M37 | 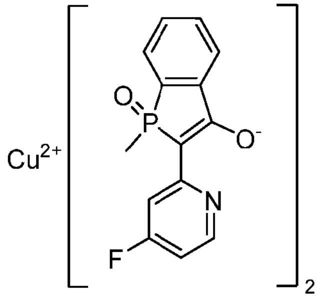 | -3.75 | M194 | 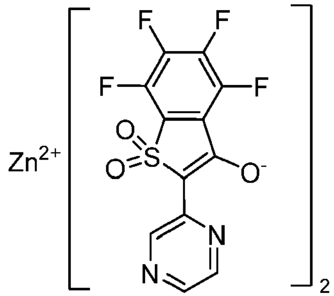 | -3.32 |
| M38 | 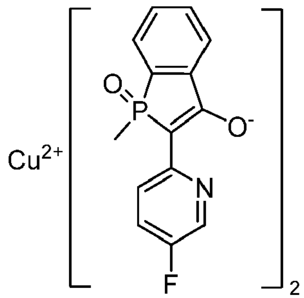 | -3.75 | M195 | 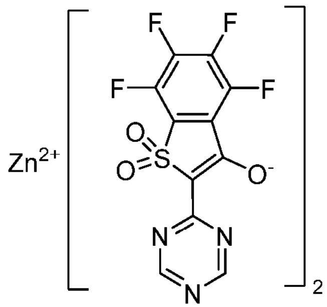 | -3.48 |
| M39 | 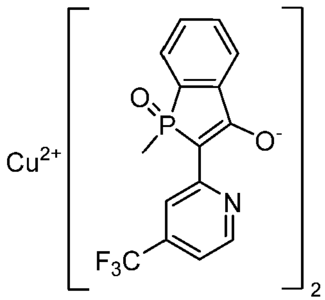 | -3.94 | M196 | 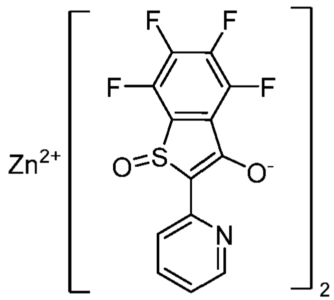 | -2.67 |

(continued)

| Referred to as: | Structure | LUMO [eV] | Referred to as: | Structure | LUMO [eV] |
|---|---|---|---|---|---|
| M40 | 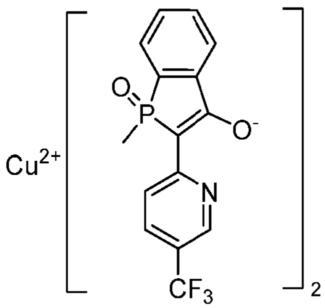 | -3.91 | M197 | 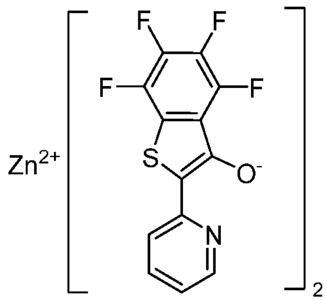 | -2.31 |
| M41 | 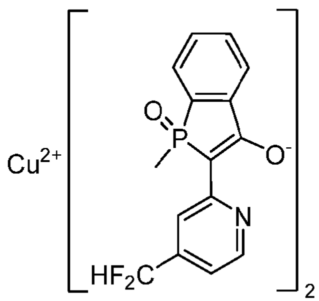 | -3.82 | M198 | 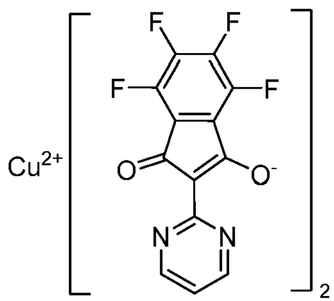 | -4.38 |
| M42 | 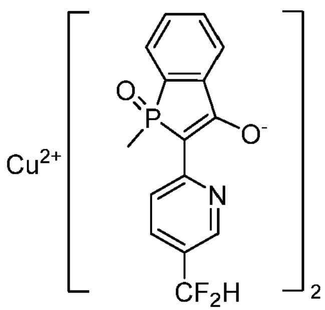 | -3.77 | M199 | 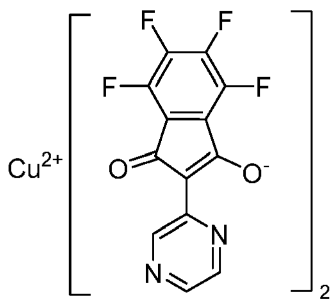 | -4.54 |

(continued)

| Referred to as: | Structure | LUMO [eV] | Referred to as: | Structure | LUMO [eV] |
|---|---|---|---|---|---|
| M43 | 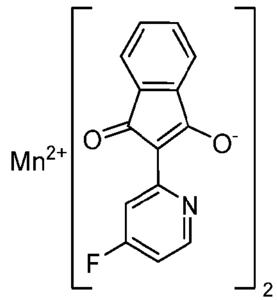 | -2.69 | M200 | 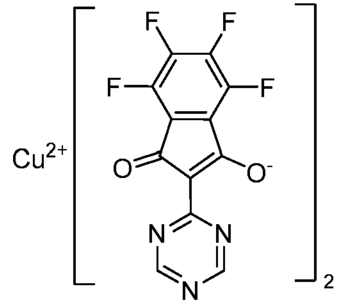 | -4.75 |
| M44 | 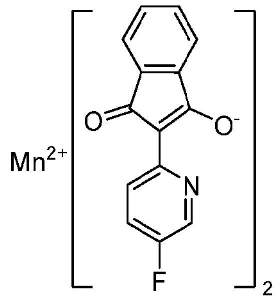 | -2.70 | M201 | 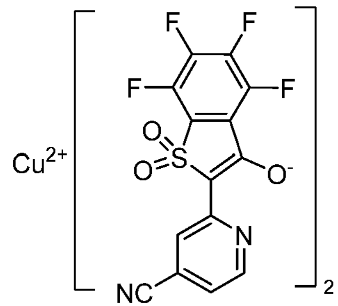 | -4.81 |
| M45 | 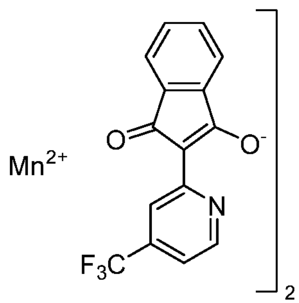 | -2.97 | M202 | 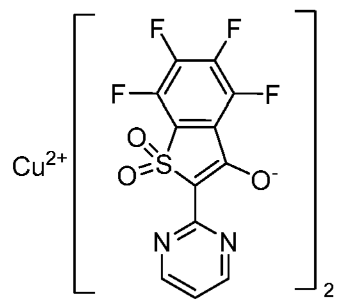 | -4.59 |

(continued)

| Referred to as: | Structure | LUMO [eV] | Referred to as: | Structure | LUMO [eV] |
|---|---|---|---|---|---|
| M46 | 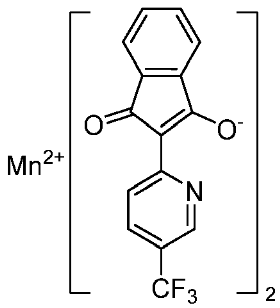 | -2.93 | M203 | 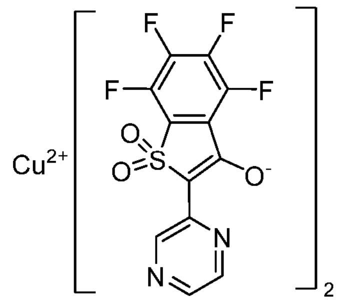 | -4.69 |
| M47 | 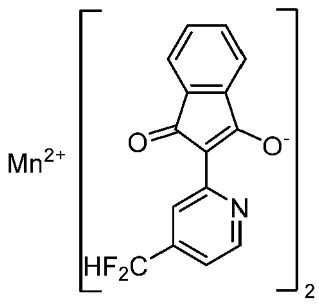 | -2.79 | M204 | 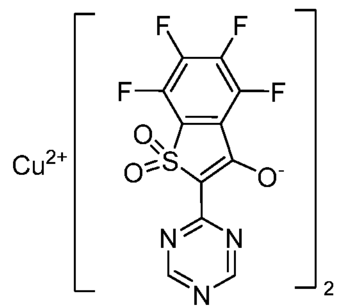 | -4.95 |
| M48 | 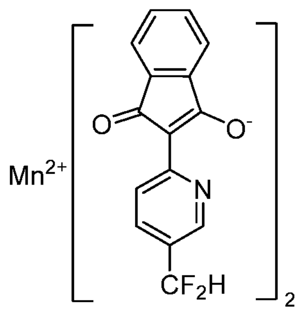 | -2.82 | M205 | 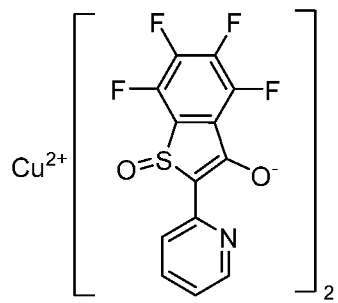 | -4.09 |

(continued)

| Referred to as: | Structure | LUMO [eV] | Referred to as: | Structure | LUMO [eV] |
|---|---|---|---|---|---|
| M49 | 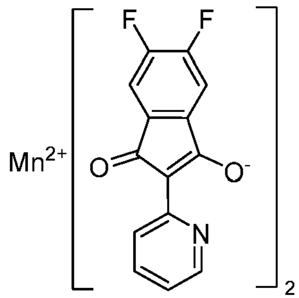 | -2.82 | M206 | 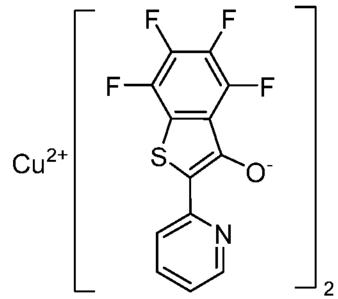 | -3.67 |
| M50 | 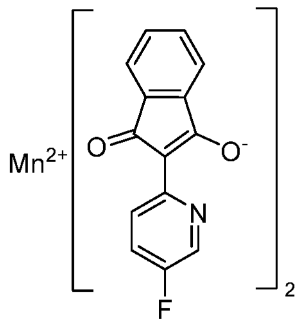 | -2.97 | M207 | 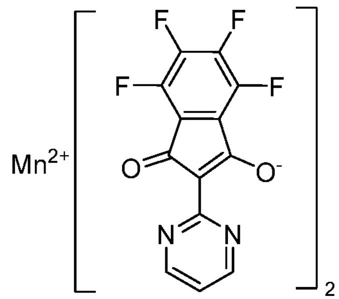 | -3.17 |
| M51 | 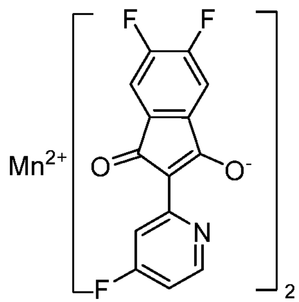 | -2.97 | M208 | 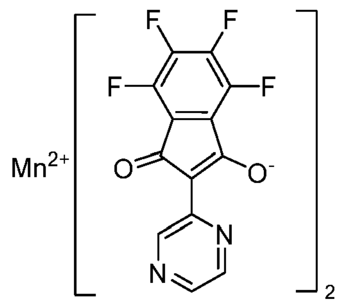 | -3.41 |

(continued)

| Referred to as: | Structure | LUMO [eV] | Referred to as: | Structure | LUMO [eV] |
|---|---|---|---|---|---|
| M52 | 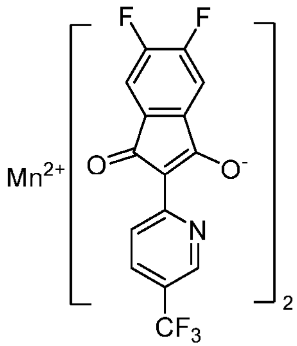 | -3.20 | M209 | 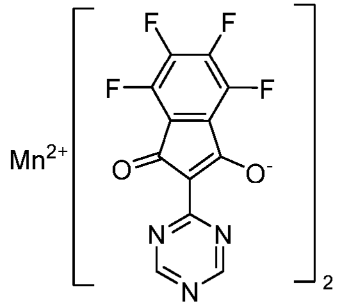 | -3.52 |
| M53 | 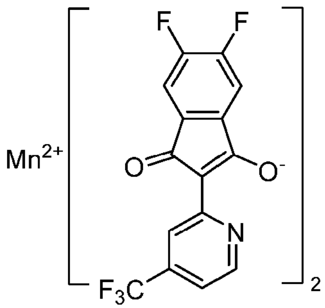 | -3.23 | M210 | 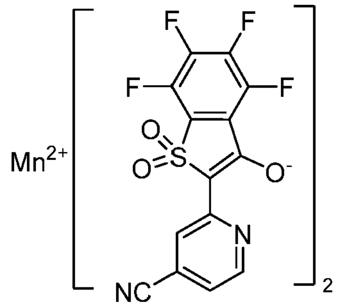 | -3.83 |
| M54 | 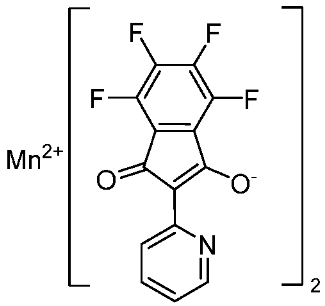 | -3.02 | M211 | 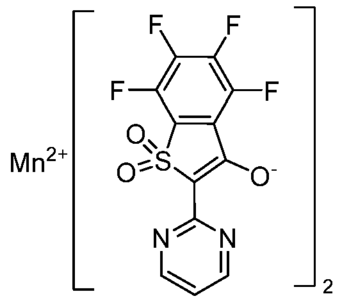 | -3.38 |

(continued)

| Referred to as: | Structure | LUMO [eV] | Referred to as: | Structure | LUMO [eV] |
|---|---|---|---|---|---|
| M55 | 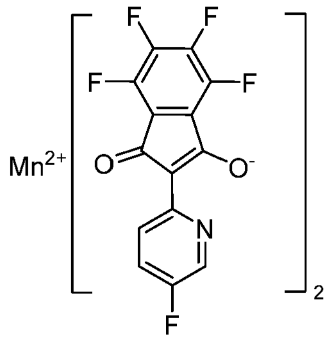 | -3.16 | M212 | 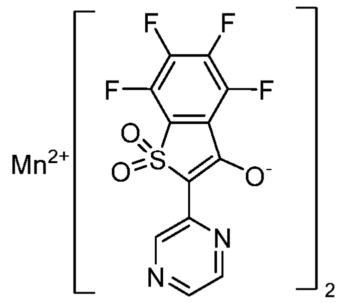 | -3.62 |
| M56 | 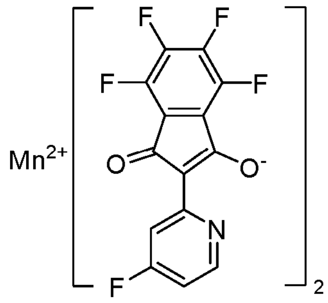 | -3.17 | M213 | 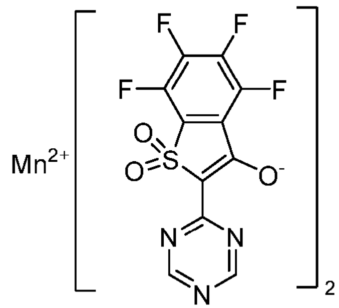 | -3.76 |
| M57 | 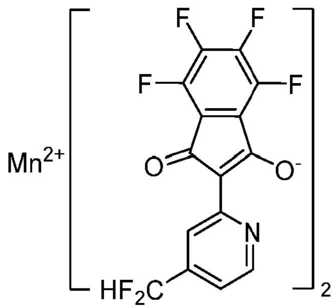 | -3.27 | M214 | 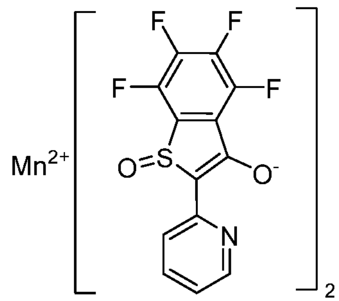 | -2.94 |

(continued)

| Referred to as: | Structure | LUMO [eV] | Referred to as: | Structure | LUMO [eV] |
|---|---|---|---|---|---|
| M58 | 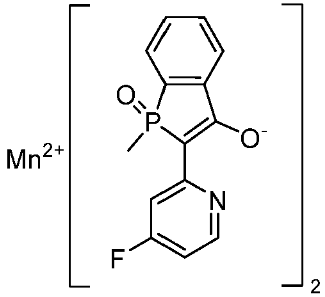 | -2.53 | M215 | 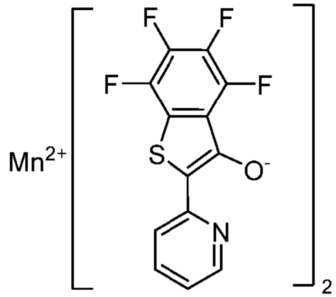 | -2.58 |
| M59 | 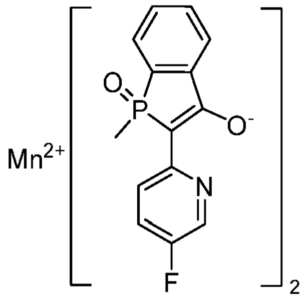 | -2.57 | M216 | 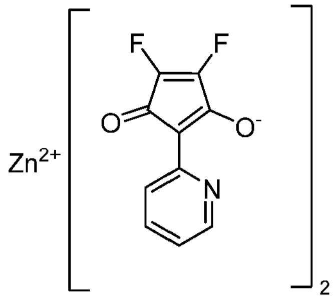 | -2.86 |
| M60 | 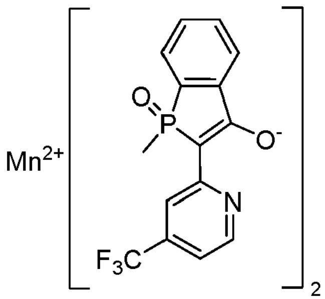 | -2.88 | M217 | 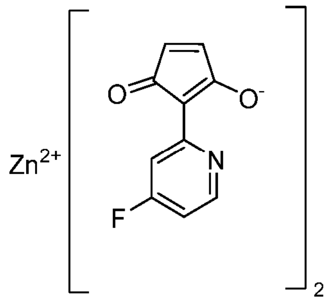 | -2.77 |

(continued)

| Referred to as: | Structure | LUMO [eV] | Referred to as: | Structure | LUMO [eV] |
|---|---|---|---|---|---|
| M61 | 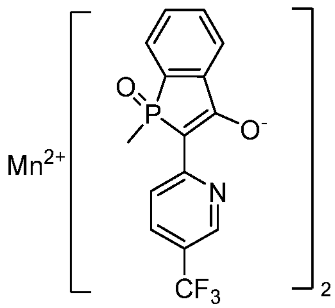 | -2.84 | M218 | 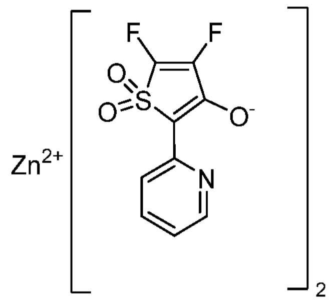 | -2.82 |
| M62 | 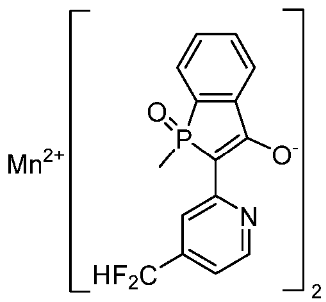 | -2.75 | M219 | 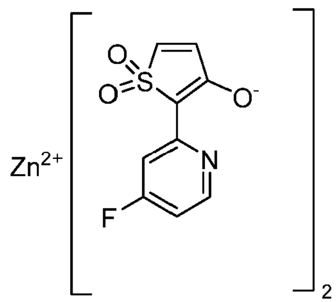 | -2.81 |
| M63 | 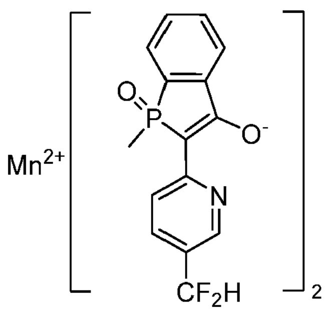 | -2.72 | M220 | 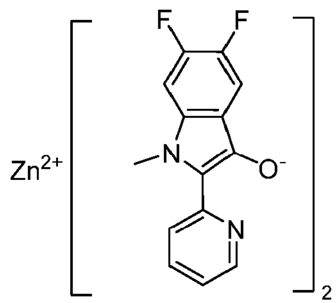 | -1.94 |

(continued)

| Referred to as: | Structure | LUMO [eV] | Referred to as: | Structure | LUMO [eV] |
|---|---|---|---|---|---|
| M64 | 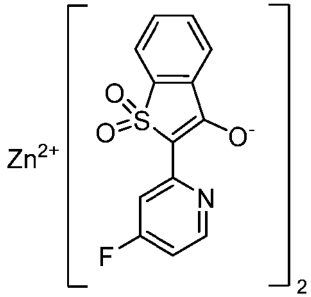 | -2.58 | M221 | 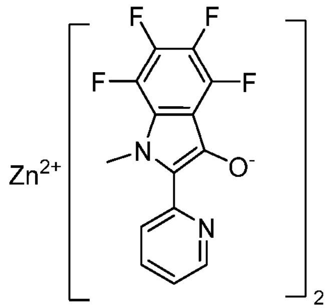 | -2.11 |
| M65 | 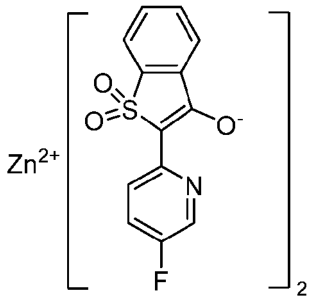 | -2.58 | M222 | 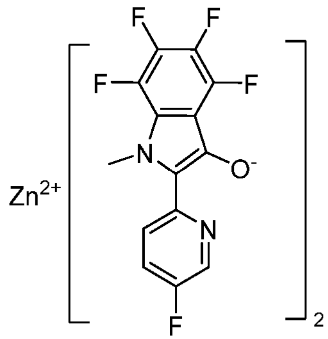 | -2.29 |
| M66 | 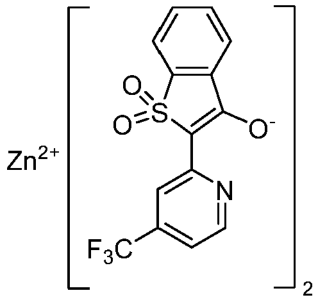 | -2.90 | M223 | 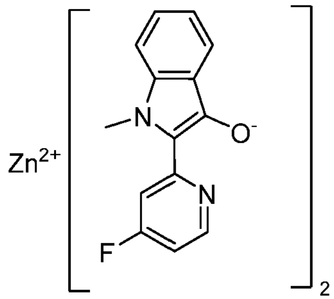 | -1.78 |

(continued)

| Referred to as: | Structure | LUMO [eV] | Referred to as: | Structure | LUMO [eV] |
|---|---|---|---|---|---|
| M67 | 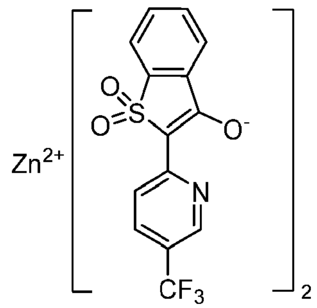 | -2.88 | M224 | 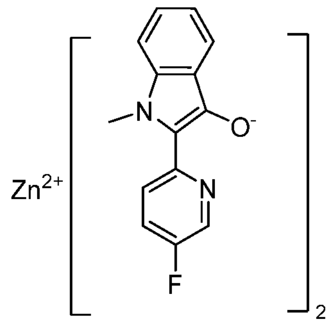 | -1.86 |
| M58 | 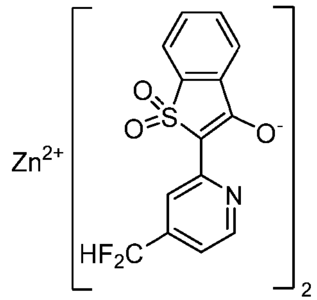 | -2.78 | M225 | 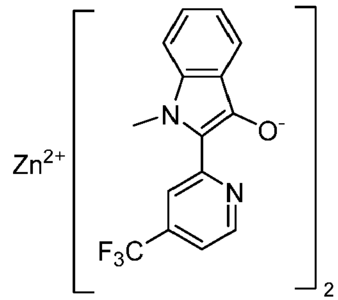 | -2.25 |
| M69 | 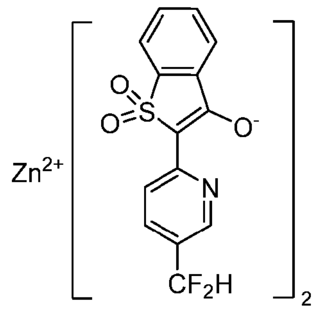 | -2.75 | M226 | 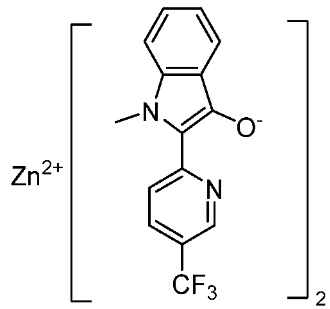 | -2.17 |

(continued)

| Referred to as: | Structure | LUMO [eV] | Referred to as: | Structure | LUMO [eV] |
|---|---|---|---|---|---|
| M70 | 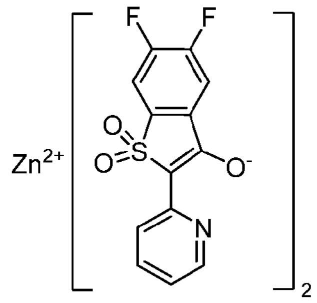 | -2.70 | M227 | 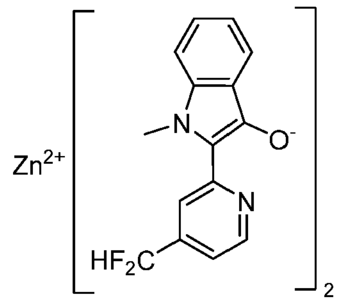 | -2.08 |
| M71 | 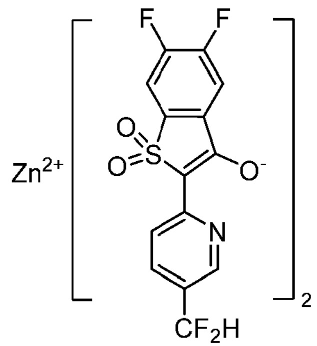 | -2.94 | M228 | 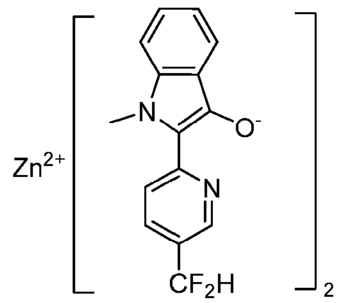 | -2.1 |
| M72 | 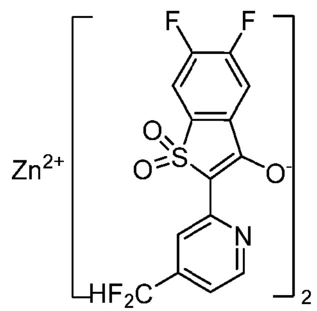 | -2.95 | M229 | 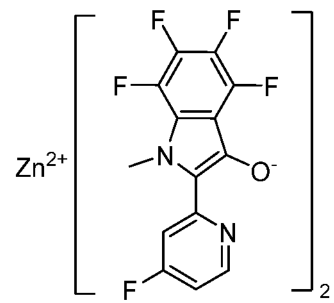 | -2.21 |

(continued)

| Referred to as: | Structure | LUMO [eV] | Referred to as: | Structure | LUMO [eV] |
|---|---|---|---|---|---|
| M73 | 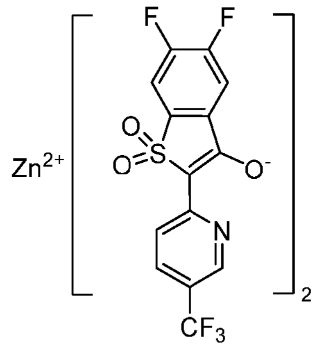 | -3.12 | M230 | 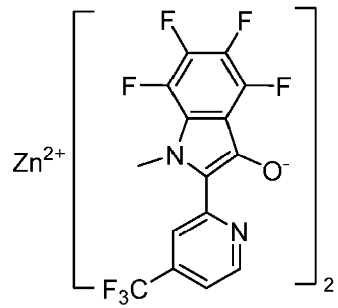 | -2.66 |
| M74 | 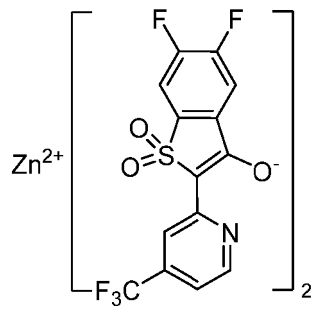 | -3.14 | M231 | 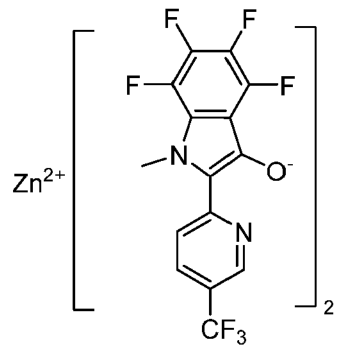 | -2.59 |
| M75 | 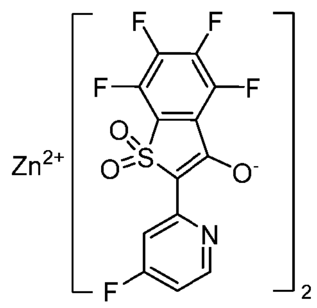 | -3.02 | M232 | 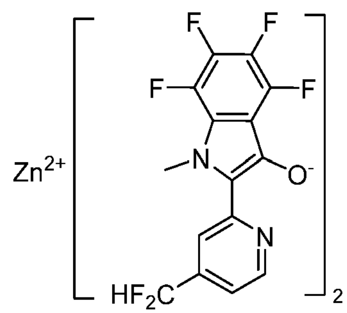 | -2.47 |

(continued)

| Referred to as: | Structure | LUMO [eV] | Referred to as: | Structure | LUMO [eV] |
|---|---|---|---|---|---|
| M76 | 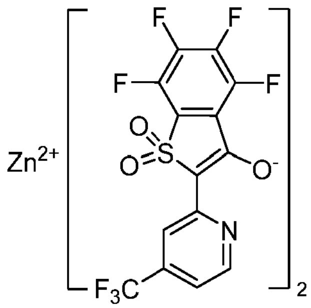 | -3.30 | M233 | 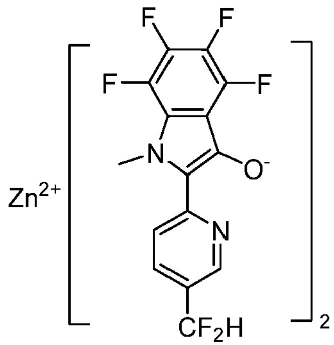 | -2.46 |
| M77 | 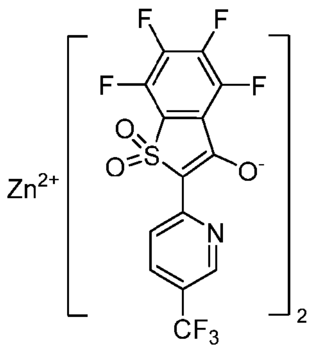 | -3.28 | M234 | 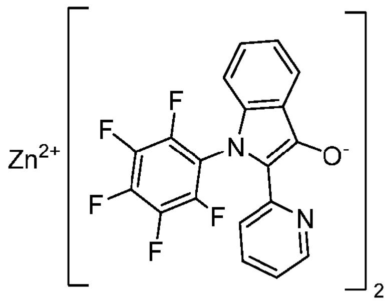 | -1.98 |
| M78 | 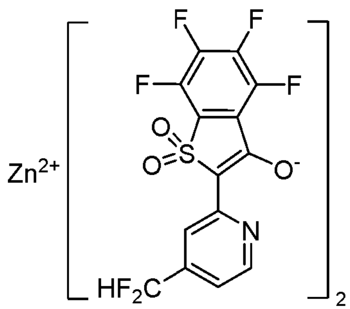 | -3.18 | M235 | 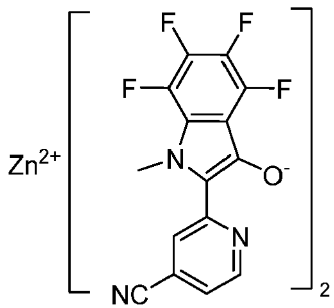 | -3.10 |

(continued)

| Referred to as: | Structure | LUMO [eV] | Referred to as: | Structure | LUMO [eV] |
|---|---|---|---|---|---|
| M79 | 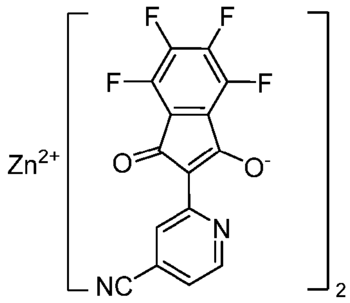 | -3.36 | M236 | 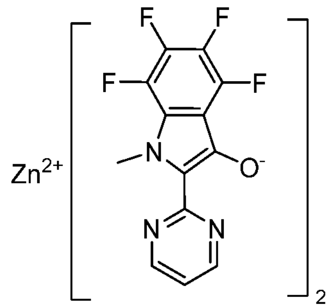 | -2.31 |
| M80 | 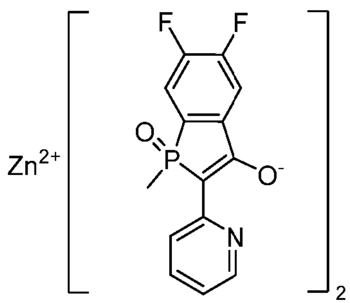 | -2.37 | M237 | 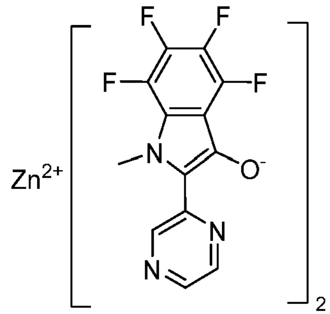 | -2.65 |
| M81 | 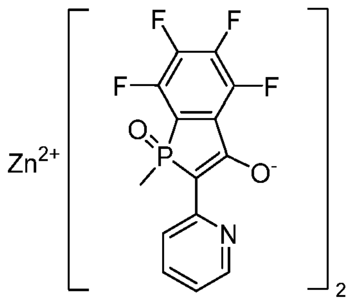 | -2.54 | M238 | 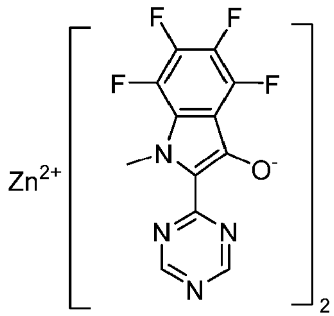 | -2.71 |

(continued)

| Referred to as: | Structure | LUMO [eV] | Referred to as: | Structure | LUMO [eV] |
|---|---|---|---|---|---|
| M82 | 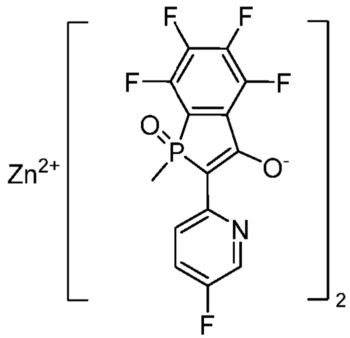 | -2.66 | M239 | 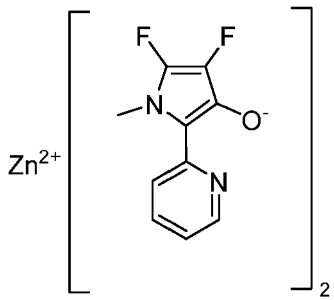 | -1.62 |
| M83 | 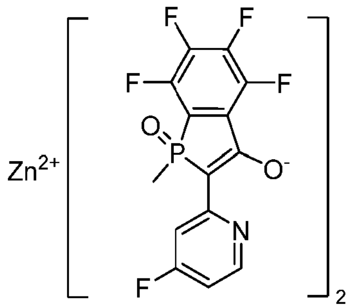 | -2.68 | M240 | 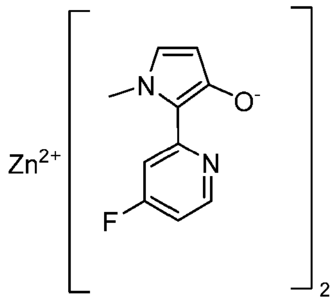 | -1.39 |
| M84 | 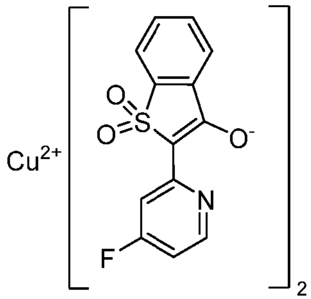 | -4.15 | M241 | 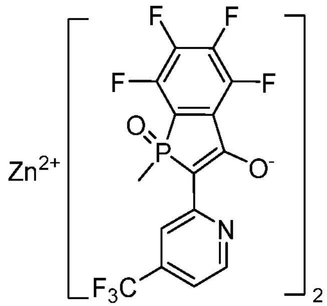 | -2.97 |

(continued)

| Referred to as: | Structure | LUMO [eV] | Referred to as: | Structure | LUMO [eV] |
|---|---|---|---|---|---|
| M85 | 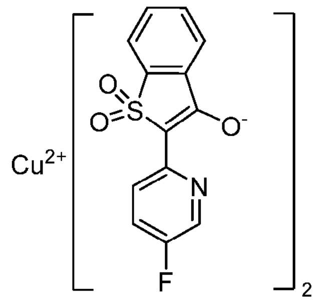 | -4.16 | M242 | 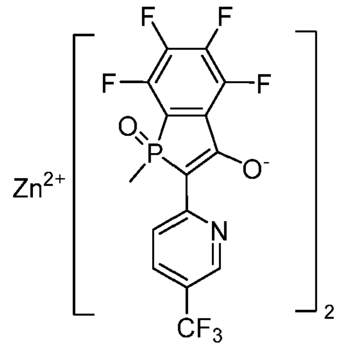 | -2.95 |
| M86 | 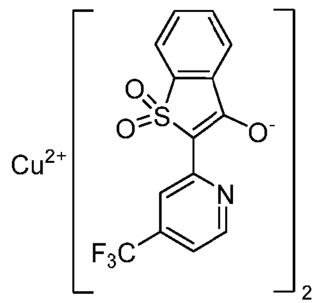 | -4.34 | M243 | 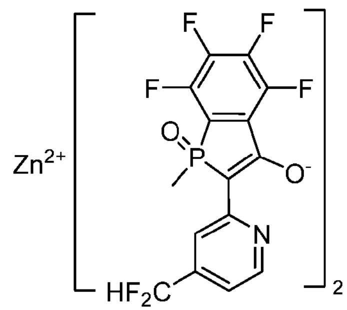 | -2.85 |
| M87 | 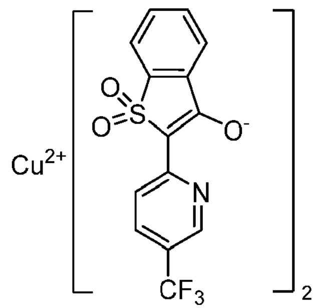 | -4.32 | M244 | 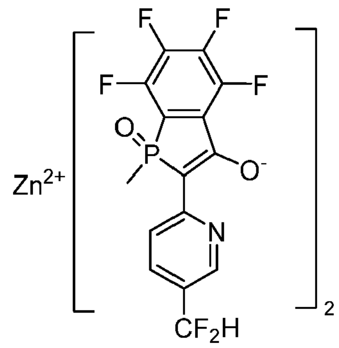 | -2.78 |

(continued)

| Referred to as: | Structure | LUMO [eV] | Referred to as: | Structure | LUMO [eV] |
|---|---|---|---|---|---|
| M88 | 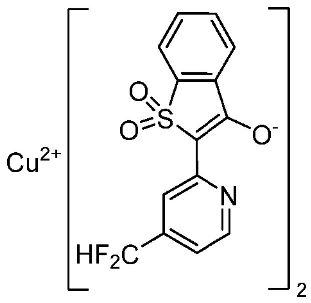 | -4.24 | M245 | 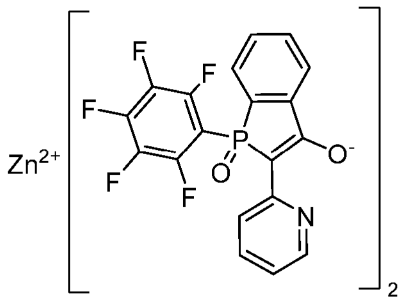 | -2.33 |
| M89 | 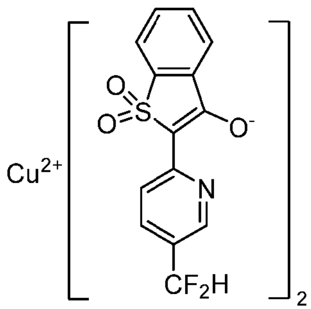 | -4.14 | M246 | 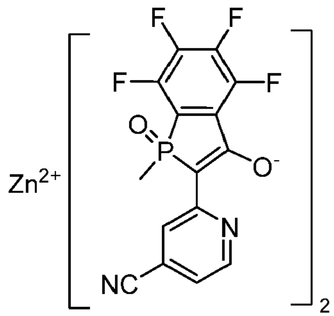 | -3.29 |
| M90 | 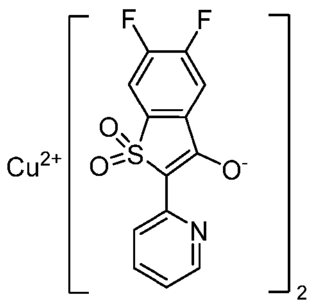 | -4.23 | M247 | 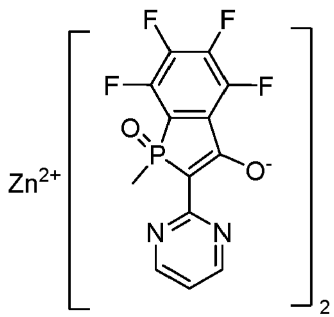 | -2.75 |

(continued)

| Referred to as: | Structure | LUMO [eV] | Referred to as: | Structure | LUMO [eV] |
|---|---|---|---|---|---|
| M91 | 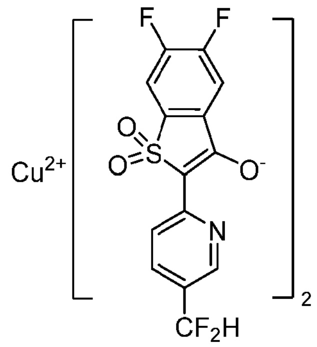 | -4.40 | M248 | 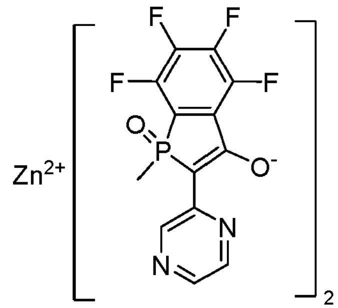 | -2.98 |
| M92 | 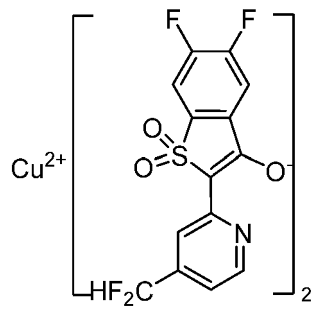 | -4.45 | M249 | 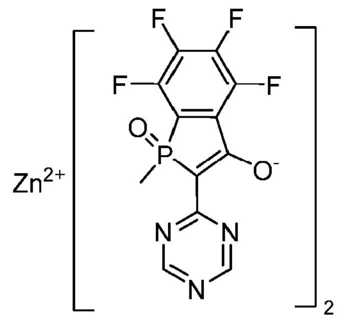 | -3.14 |
| M93 | 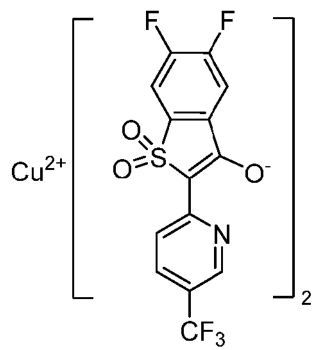 | -4.58 | M250 | 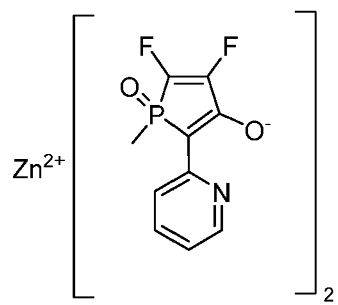 | -2.41 |

(continued)

| Referred to as: | Structure | LUMO [eV] | Referred to as: | Structure | LUMO [eV] |
|---|---|---|---|---|---|
| M94 | 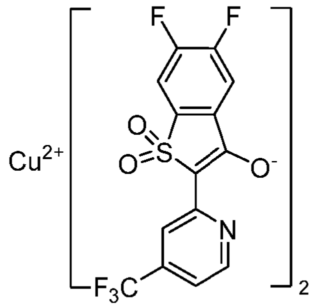 | -4.41 | M251 | 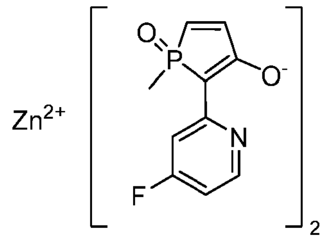 | -2.39 |
| M95 | 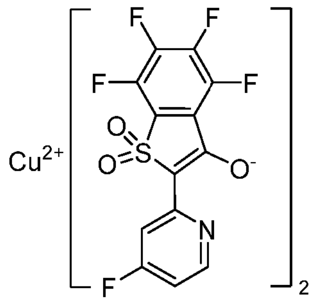 | -4.47 | M252 | 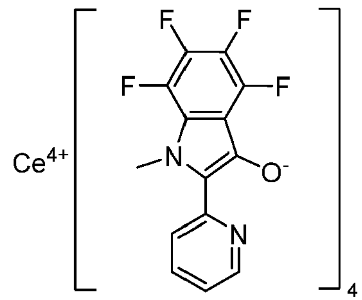 | -3.11 |

Table 3: Properties of compounds of Formula (I) and comparative compounds

| Referred to as: | Structure | LUMO [eV] | mp (°C) | Tg (°C) | $T_{dec}$ (°C) | $T_{RO}$ (°C) |
|---|---|---|---|---|---|---|
| Comparative Compound | 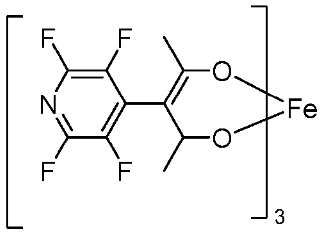 CC-1 | -3.98 | 282 | 71 | >282 | 146 |
| M28 | 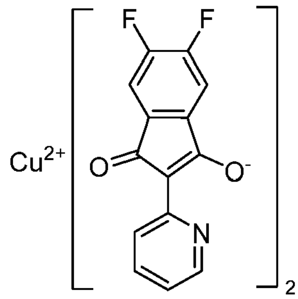 | -4.06 | Not observed | 320 | >470 | 289 |
| M7 | 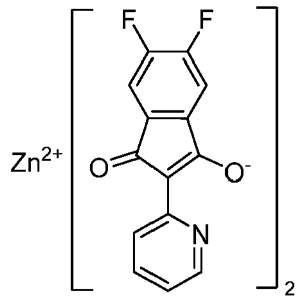 | -2.58 | Not observed | Not observed | >430 | 238 |
| M8 | 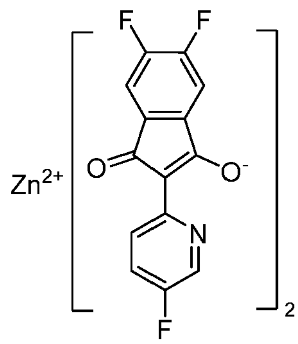 | -2.70 | Not observed | not observed | >430 | 246 |

Table 4: Performance and stability of organic electroluminescent devices comprising a semiconductor layer comprising a compound of formula (I)

| **Device Example** | **Metal complex in p-HIL** | **Concentration of metal complex in p-HIL [wt%]** | **CIEy** | **Voltage at 10mA/cm² [V]** | **Ceff at 10mA/cm² [cd/A]** | **Ceff/CIEy at 10mA/cm² [cd/A]** | **EQE at 10mA/cm² [%]** | **LT97 at 30mA/cm² [h]** | **ΔU at 30mA/cm² (1-100h) [V]** |
|---|---|---|---|---|---|---|---|---|---|
| Comparative Example | CC-1 | 5 | 0.049 | 3.86 | 6.5 | 133 | 13.0 | 75 | 0.964 |
| Inventive Example 1 | M28 | 5 | 0.047 | 3.71 | 6.6 | 138 | 13.4 | 76 | 0.152 |
| Inventive Example 2 | M7 | 5 | 0.048 | 3.69 | 6.5 | 137 | 13.2 | 75 | 0.066 |
| Inventive Example 3 | M8 | 6 | 0.048 | 3.65 | 6.4 | 135 | 13.1 | 71 | 0.085 |

The particular combinations of elements and features in the above detailed embodiments are exemplary only; the interchanging and substitution of these teachings with other teachings in this and the patents/applications incorporated by reference are also expressly contemplated. As those skilled in the art will recognize, variations, modifications, and other implementations of what is described herein can occur to those of ordinary skill in the art without departing from the spirit and the scope of the invention as claimed. Accordingly, the foregoing description is by way of example only and is not intended as limiting. In the claims, the word "comprising" does not exclude other elements or steps, and the indefinite article "a" or "an" does not exclude a plurality. The mere fact that certain measures are recited in mutually different dependent claims does not indicate that a combination of these measured cannot be used to advantage. The invention's scope is defined in the following claims and the equivalents thereto. Furthermore, reference signs used in the description and claims do not limit the scope of the invention as claimed.

## Claims

**1.** A compound of Formula (I):

$$M^{n\oplus}(L^{\ominus})_n\ (AL)_m \quad (I),$$

wherein:

M is a metal ion,
n is the valency of M and selected from 1 to 4;
AL is an ancillary ligand which coordinates to the metal ion M;
m is an integer selected from 0 to 2
L is a ligand of formula (II)

E-U (II)

wherein E is selected from formula (III)

$$^{\ominus}G{-}A \quad (III),$$

G is selected from O and S;
A is bound to U via a single or double bond and selected from formula (IV)

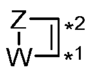
(IV)

wherein the asterisks "*1" denotes the binding position to U; and the asterisks "*2" denotes the binding position to G; whereby
W is selected from S, NR', C=S, and Q=O;
wherein Q is selected from C; S, SO, PR',
wherein R' is selected from substituted or unsubstituted $C_1$ to $C_{16}$ alkyl, substituted or unsubstituted $C_6$ to $C_{40}$ aryl, substituted or unsubstituted $C_3$ to $C_{40}$ heteroaryl;
wherein the one or more substituents on R' is independently selected from D, electron-withdrawing group, F, CN, $NO_2$, substituted or unsubstituted $C_1$ to $C_6$ alkyl, partially fluorinated or perfluorinated $C_1$ to $C_6$ alkyl, $CH_2F$, $CHF_2$, CHDF, $CD_2F$, $CDF_2$, $CF_3$, substituted or unsubstituted $C_1$ to $C_6$ alkoxy, partially fluorinated or perfluorinated $C_1$ to $C_6$ alkoxy, $OCH_2F$, $OCHF_2$, OCHDF, $OCD_2F$, $OCDF_2$, $OCF_3$;
Z is selected from formula (V);

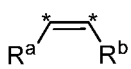
(V),

wherein the asterisks "*" denotes the binding positions to the other moieties of formula (IV);

$R^a$, and $R^b$ are independently selected from H, D, substituted or unsubstituted $C_1$ to $C_{12}$ alkyl, substituted or unsubstituted $C_6$ to $C_{19}$ aryl, substituted or unsubstituted $C_2$ to $C_{20}$ heteroaryl, substituted or unsubstituted 6-membered heteroaryl, electron-withdrawing group, $NO_2$, partially fluorinated or perfluorinated $C_1$ to $C_6$ alkyl, $CH_2F$, $CHF_2$, CHDF, $CD_2F$, $CDF_2$, $CF_3$, substituted or unsubstituted $C_1$ to $C_6$ alkoxy, partially fluorinated or perfluorinated $C_1$ to $C_6$ alkoxy, $OCH_2F$, $OCHF_2$, OCHDF, $OCD_2F$, $OCDF_2$, $OCF_3$, For CN, wherein the one or more substituents on $R^a$, and $R^b$ is independently selected from D, electron-withdrawing group, F, CN, $NO_2$, substituted or unsubstituted $C_1$ to $C_6$ alkyl, partially fluorinated or perfluorinated $C_1$ to $C_6$ alkyl, $CH_2F$, $CHF_2$, CHDF, $CD_2F$, $CDF_2$, $CF_3$, substituted or unsubstituted $C_1$ to $C_6$ alkoxy, partially fluorinated or perfluorinated $C_1$ to $C_6$ alkoxy, $OCH_2F$, $OCHF_2$, OCHDF, $OCD_2F$, $OCDF_2$, $OCF_3$;

or

$R^a$, and $R^b$ form together, irrespective of the previous definition, a substituted or unsubstituted aryl ring, substituted or unsubstituted aryl ring system, substituted or unsubstituted heteroaryl ring or substituted or unsubstituted heteroaryl ring system;

wherein the one or more substituents on the aryl ring, the aryl ring system, the heteroaryl ring and heteroaryl ring system is independently selected from D, electron-withdrawing group, F, CN, $NO_2$, substituted or unsubstituted $C_1$ to $C_6$ alkyl, partially fluorinated or perfluorinated $C_1$ to $C_6$ alkyl, $CH_2F$, $CHF_2$, CHDF, $CD_2F$, $CDF_2$, $CF_3$, substituted or unsubstituted $C_1$ to $C_6$ alkoxy, partially fluorinated or perfluorinated $C_1$ to $C_6$ alkoxy, $OCH_2F$, $OCHF_2$, OCHDF, $OCD_2F$, $OCDF_2$, $OCF_3$;

wherein U is selected from formula (VI)

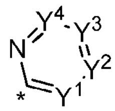

(VI)

wherein the asterisks "*" denotes the binding position;

$Y^1$ is selected from $CR^1$ or N;

$Y^2$ is selected from $CR^2$ or N;

$Y^3$ is selected from $CR^3$ or N;

$Y^4$ is selected from $CR^4$ or N;

wherein 0, 1, or 2 of the group consisting of $Y^1$ , $Y^2$,$Y^3$, $Y^4$ are selected from N;

$R^1$, $R^2$, $R^3$, and $R^4$ are independently selected from H, D, substituted or unsubstituted $C_1$ to $C_{12}$ alkyl, substituted or unsubstituted $C_6$ to $C_{19}$ aryl, substituted or unsubstituted $C_2$ to $C_{20}$ heteroaryl, substituted or unsubstituted 6-membered heteroaryl, electron-withdrawing group, $NO_2$, partially fluorinated or perfluorinated $C_1$ to $C_6$ alkyl, $CH_2F$, $CHF_2$, CHDF, $CD_2F$, $CDF_2$, $CF_3$, substituted or unsubstituted $C_1$ to $C_6$ alkoxy, partially fluorinated or perfluorinated $C_1$ to $C_6$ alkoxy, $OCH_2F$, $OCHF_2$, OCHDF, $OCD_2F$, $OCDF_2$, $OCF_3$, For CN, wherein the one or more substituents on $R^1$, $R^2$, $R^3$, and $R^4$ is independently selected from D, electron-withdrawing group, F, CN, $NO_2$, substituted or unsubstituted $C_1$ to $C_6$ alkyl, partially fluorinated or perfluorinated $C_1$ to $C_6$ alkyl, $CH_2F$, $CHF_2$, CHDF, $CD_2F$, $CDF_2$, $CF_3$, substituted or unsubstituted $C_1$ to $C_6$ alkoxy, partially fluorinated or perfluorinated $C_1$ to $C_6$ alkoxy, $OCH_2F$, $OCHF_2$, OCHDF, $OCD_2F$, $OCDF_2$, $OCF_3$;

and wherein L comprises at least one F and/or CN.

**2.** The compound of claim 1, wherein the molecular mass of L is selected in the range of ≤ 700 Da and ≥ 150 Da.

**3.** The compound of claim 1 or 2 wherein the compound of Formula (I) has a difference between the LUMO energy level and HOMO energy level, i.e. an Egap of ≥ 3.3 eV.

**4.** The compound of any of the claims 1 to 3, wherein the compound of Formula (I) does not emit light in the wavelength range ≥400 to ≤780 nm.

**5.** The compound of any of the claims 1 to 4, whereby the compound of formula (I) comprises either fluorine or cyano groups, but not both.

**6.** The compound of any of the claims 1 to 5, wherein in ligand L either the moiety E or the moiety U comprises at least one fluorine or cyano group, but not both.

**7.** The compound of any of the claims 1 to 6, wherein $R^a$, and $R^b$ form together a substituted or unsubstituted aryl ring, substituted or unsubstituted aryl ring system, substituted or unsubstituted heteroaryl ring or substituted or unsubstituted heteroaryl ring system; wherein the one or more substituents on the aryl ring, the aryl ring system, the heteroaryl ring and heteroaryl ring system is independently selected from D, electron-withdrawing group, F, CN, $NO_2$, substituted or unsubstituted $C_1$ to $C_6$ alkyl, partially fluorinated or perfluorinated $C_1$ to $C_6$ alkyl, $CH_2F$, $CHF_2$, CHDF, $CD_2F$, $CDF_2$, $CF_3$, substituted or unsubstituted $C_1$ to $C_6$ alkoxy, partially fluorinated or perfluorinated $C_1$ to $C_6$ alkoxy, $OCH_2F$, $OCHF_2$, OCHDF, $OCD_2F$, $OCDF_2$, $OCF_3$.

**8.** The compound of any of the claims 1 to 7, wherein U is selected from formulae (VIb), (VIc), (VId), (VIe) and (VIf):

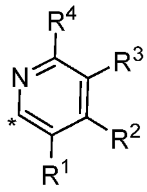 (VIb); 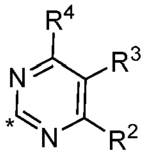 (VIc); 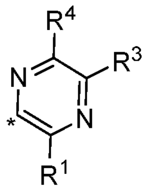 (VId); 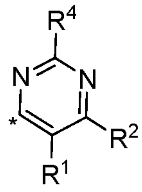 (VIe); 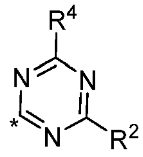 (VIf);

**9.** An organic semiconductor layer, whereby the organic semiconductor layer comprises a compound of formula (I) of any of the preceding claims 1 to 8.

**10.** An organic electronic device comprising an anode layer, a cathode layer, and an organic semiconductor layer according to claim 9, preferably the organic semiconductor layer is arranged between the anode layer and the cathode layer.

**11.** The organic electronic device according to claim 10, wherein the organic semiconductor layer is arranged adjacent to the anode layer, preferably the organic semiconductor layer is arranged in direct contact with the anode layer.

**12.** The organic electronic device of claim 10 or 11, whereby at least one organic semiconductor layer that is the organic semiconductor layer according to claim 10 is a hole injection layer.

**13.** The organic electronic device according to any of the claims 10 to 12, wherein the organic electronic device is an electroluminescent device, an organic light emitting diode (OLED), a light emitting device, thin film transistor, a battery, a display device, an organic photovoltaic cell (OPV), or an organic photodetector.

**14.** A display device comprising an organic electronic device according to any of the claims 10 to 13.

**15.** Use of a compound of formula (Ia)

$$M^{n\oplus}(L^{\ominus})_n\,(AL)_m \quad \text{(Ia)},$$

wherein:

M is a metal ion,
n is the valency of M and selected from 1 to 4;
AL is an ancillary ligand which coordinates to the metal ion M;
m is an integer selected from 0 to 2
L is a ligand of formula (II)

E-U (II)

wherein E is selected from formula (III)

$$^{\ominus}G{-}A \quad \text{(III)},$$

G is selected from O and S;
A is bound to U via a single or double bond and selected from formula (IV)

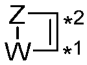 (IV)

wherein the asterisks "*1" denotes the binding position to U; and the asterisks "*2" denotes the binding position to G; whereby
W is selected from S, NR', C=S, and Q=O;
wherein Q is selected from C; S, SO, PR',
wherein R' is selected from substituted or unsubstituted $C_1$ to $C_{16}$ alkyl, substituted or unsubstituted $C_6$ to $C_{40}$ aryl, substituted or unsubstituted $C_3$ to $C_{40}$ heteroaryl;
wherein the one or more substituents on R' is independently selected from D, electron-withdrawing group, F, CN, $NO_2$, substituted or unsubstituted $C_1$ to $C_6$ alkyl, partially fluorinated or perfluorinated $C_1$ to $C_6$ alkyl, $CH_2F$, $CHF_2$, CHDF, $CD_2F$, $CDF_2$, $CF_3$, substituted or unsubstituted $C_1$ to $C_6$ alkoxy, partially fluorinated or perfluorinated $C_1$ to $C_6$ alkoxy, $OCH_2F$, $OCHF_2$, OCHDF, $OCD_2F$, $OCDF_2$, $OCF_3$;
Z is selected from formula (V);

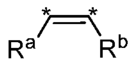 (V),

wherein the asterisks "*" denotes the binding positions to the other moieties of formula (IV);
$R^a$, and $R^b$ are independently selected from H, D, substituted or unsubstituted $C_1$ to $C_{12}$ alkyl, substituted or unsubstituted $C_6$ to $C_{19}$ aryl, substituted or unsubstituted $C_2$ to $C_{20}$ heteroaryl, substituted or unsubstituted 6-membered heteroaryl, electron-withdrawing group, $NO_2$, partially fluorinated or perfluorinated $C_1$ to $C_6$ alkyl, $CH_2F$, $CHF_2$, CHDF, $CD_2F$, $CDF_2$, $CF_3$, substituted or unsubstituted $C_1$ to $C_6$ alkoxy, partially fluorinated or perfluorinated $C_1$ to $C_6$ alkoxy, $OCH_2F$, $OCHF_2$, OCHDF, $OCD_2F$, $OCDF_2$, $OCF_3$, For CN, wherein the one or more substituents on $R^a$, and $R^b$ is independently selected from D, electron-withdrawing group, F, CN, $NO_2$, substituted or unsubstituted $C_1$ to $C_6$ alkyl, partially fluorinated or perfluorinated $C_1$ to $C_6$ alkyl, $CH_2F$, $CHF_2$, CHDF, $CD_2F$, $CDF_2$, $CF_3$, substituted or unsubstituted $C_1$ to $C_6$ alkoxy, partially fluorinated or perfluorinated $C_1$ to $C_6$ alkoxy, $OCH_2F$, $OCHF_2$, OCHDF, $OCD_2F$, $OCDF_2$, $OCF_3$;
or
$R^a$, and $R^b$ form together, irrespective of the previous definition, a substituted or unsubstituted aryl ring, substituted or unsubstituted aryl ring system, substituted or unsubstituted heteroaryl ring or substituted or unsubstituted heteroaryl ring system;
wherein the one or more substituents on the aryl ring, the aryl ring system, the heteroaryl ring and heteroaryl ring system is independently selected from D, electron-withdrawing group, F, CN, $NO_2$, substituted or unsubstituted $C_1$ to $C_6$ alkyl, partially fluorinated or perfluorinated $C_1$ to $C_6$ alkyl, $CH_2F$, $CHF_2$, CHDF, $CD_2F$, $CDF_2$, $CF_3$, substituted or unsubstituted $C_1$ to $C_6$ alkoxy, partially fluorinated or perfluorinated $C_1$ to $C_6$ alkoxy, $OCH_2F$, $OCHF_2$, OCHDF, $OCD_2F$, $OCDF_2$, $OCF_3$;
wherein U is selected from formula (VI)

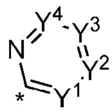 (VI)

wherein the asterisks "*" denotes the binding position;
$Y^1$ is selected from $CR^1$ or N;
$Y^2$ is selected from $CR^2$ or N;
$Y^3$ is selected from $CR^3$ or N;
$Y^4$ is selected from $CR^4$ or N;
wherein 0, 1, or 2 of the group consisting of $Y^1$ , $Y^2$,$Y^3$, $Y^4$ are selected from N;
$R^1$, $R^2$, $R^3$, and $R^4$ are independently selected from H, D, substituted or unsubstituted $C_1$ to $C_{12}$ alkyl, substituted or unsubstituted $C_6$ to $C_{19}$ aryl, substituted or unsubstituted $C_2$ to $C_{20}$ heteroaryl, substituted or unsubstituted 6-membered heteroaryl, electron-withdrawing group, $NO_2$, partially fluorinated or perfluorinated $C_1$ to $C_6$ alkyl, $CH_2F$, $CHF_2$, CHDF, $CD_2F$, $CDF_2$, $CF_3$, substituted or unsubstituted $C_1$ to $C_6$ alkoxy, partially fluorinated or perfluorinated $C_1$ to $C_6$ alkoxy, $OCH_2F$, $OCHF_2$, OCHDF, $OCD_2F$, $OCDF_2$, $OCF_3$, For CN, wherein the one or more substituents on $R^1$, $R^2$, $R^3$, and $R^4$ is independently selected from D, electron-withdrawing group, F, CN, $NO_2$,

substituted or unsubstituted $C_1$ to $C_6$ alkyl, partially fluorinated or perfluorinated $C_1$ to $C_6$ alkyl, $CH_2F$, $CHF_2$, CHDF, $CD_2F$, $CDF_2$, $CF_3$, substituted or unsubstituted $C_1$ to $C_6$ alkoxy, partially fluorinated or perfluorinated $C_1$ to $C_6$ alkoxy, $OCH_2F$, $OCHF_2$, OCHDF, $OCD_2F$, $OCDF_2$, $OCF_3$;
as:

- hole injection material;
- charge generation material;
- dopant for doping an organic semiconductor;
- p-dopant for a hole transport material;
- organic semiconductor material;
- electron transport material; and/ or
- electron injection material.

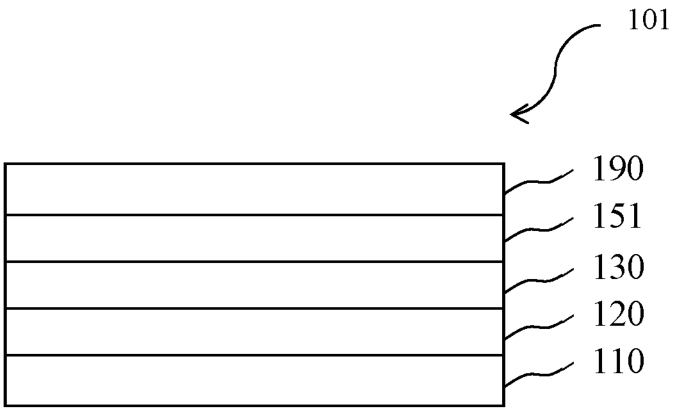


Fig.1

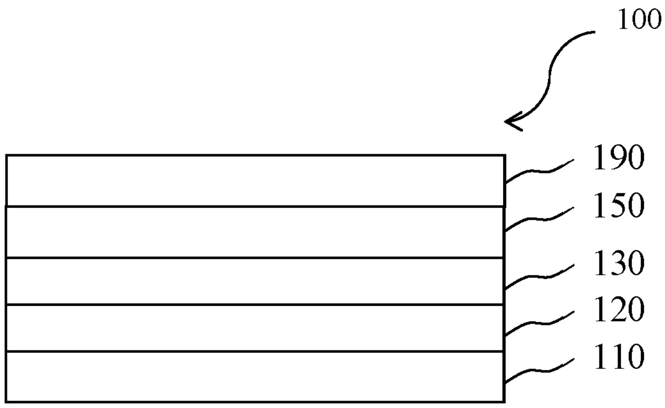


Fig. 2

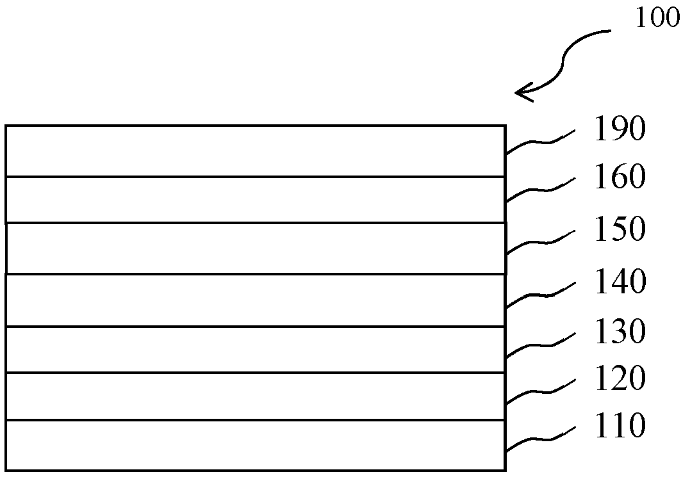
100
190
160
150
140
130
120
110

Fig. 3

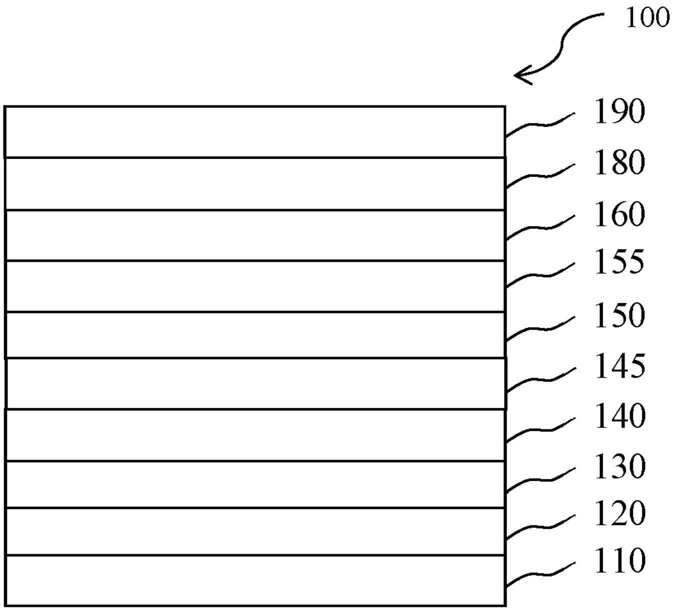
100
190
180
160
155
150
145
140
130
120
110

Fig. 4

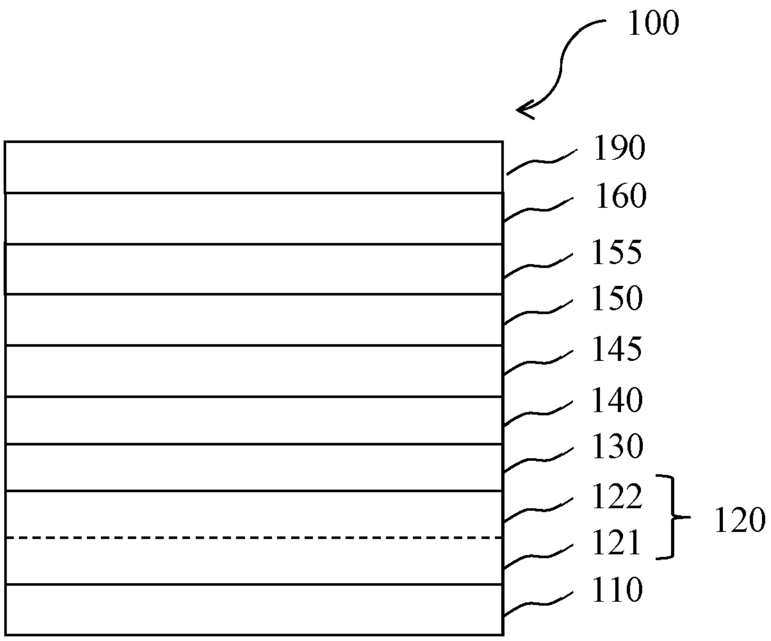
100
190
160
155
150
145
140
130
122
121
120
110

Fig. 5

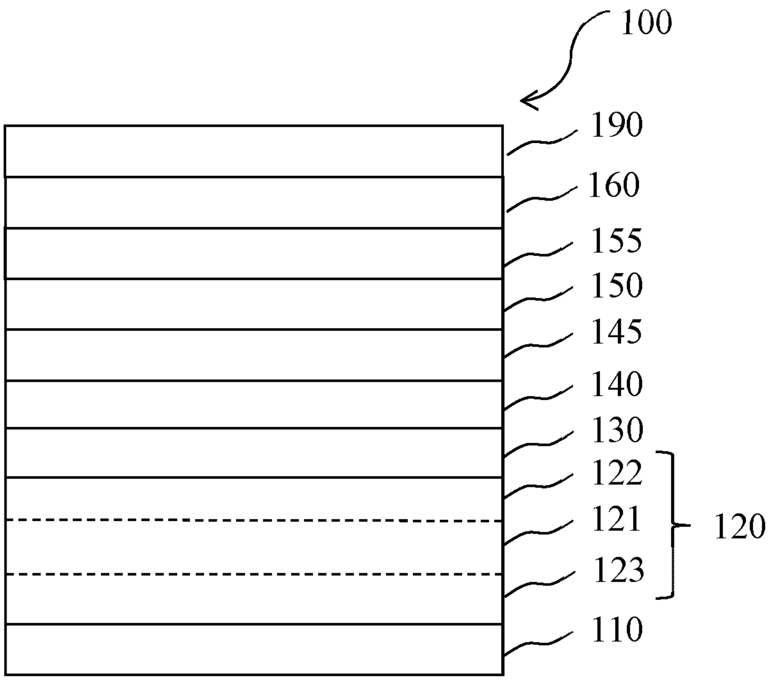
100
190
160
155
150
145
140
130
122
121
123
120
110

Fig. 6

Europäisches Patentamt
European Patent Office
Office européen des brevets

# EUROPEAN SEARCH REPORT

Application Number
EP 24 19 8221

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 3 945 090 A1 (NOVALED GMBH [DE]) 2 February 2022 (2022-02-02) * claims 1, 12-16 * * paragraph [0144] - paragraph [0146]; claim 11; table 2; compound E6 * ----- | 1-15 | INV. C07F9/6568 C07D213/50 C07D213/84 C07D213/89 C07D239/26 C07D241/12 C07D251/16 C07D409/04 C09K11/06 H05B33/14 H10K50/11 |
| A | OM PRAKASH ET AL: "Catalytic Synthesis of Bi- and Teraryls in Aqueous Medium with Palladium(II) Complexes of 2-(Pyridine-2-ylmethylsulfanyl)benzoic Acid", EUROPEAN JOURNAL OF INORGANIC CHEMISTRY, WILEY-VCH VERLAG , WENHEIM, DE, vol. 2015, no. 3, 18 December 2014 (2014-12-18), pages 520-526, XP072132879, ISSN: 1434-1948, DOI: 10.1002/EJIC.201403003 * scheme 2 * * compound 2 * * ligand L2 * ----- | 1-8,15 | |
| | | | TECHNICAL FIELDS SEARCHED (IPC) |
| A | SENNIKOVA E. V. ET AL: "2-(2-pyridyl)-3-thioindan-1-one: Synthesis, tautomerism, and complexing properties", RUSSIAN JOURNAL OF GENERAL CHEMISTRY, vol. 77, no. 10, 1 October 2007 (2007-10-01), pages 1802-1806, XP093262207, Moscow ISSN: 1070-3632, DOI: 10.1134/S1070363207100222 Retrieved from the Internet: URL:https://link.springer.com/content/pdf/10.1134/S1070363207100222.pdf> * page 1803 * * compounds VI, VII * ----- -/-- | 1-8,15 | C07F H05B C07D C09K H10K |

The present search report has been drawn up for all claims

1

| Place of search | Date of completion of the search | Examiner |
|---|---|---|
| The Hague | 25 March 2025 | Seitner, Irmgard |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

Europäisches Patentamt
European Patent Office
Office européen des brevets

# EUROPEAN SEARCH REPORT

Application Number
EP 24 19 8221

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | EP 4 321 506 A1 (NOVALED GMBH [DE]) 14 February 2024 (2024-02-14) * claims 1, 9, 20-22 * * formulas (I), (II) * ----- | 1-15 | |
| | | | TECHNICAL FIELDS SEARCHED (IPC) |

The present search report has been drawn up for all claims

1

| Place of search | Date of completion of the search | Examiner |
|---|---|---|
| The Hague | 25 March 2025 | Seitner, Irmgard |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

# ANNEX TO THE EUROPEAN SEARCH REPORT ON EUROPEAN PATENT APPLICATION NO.

EP 24 19 8221

This annex lists the patent family members relating to the patent documents cited in the above-mentioned European search report. The members are as contained in the European Patent Office EDP file on
The European Patent Office is in no way liable for these particulars which are merely given for the purpose of information.

25-03-2025

| Patent document cited in search report | Publication date | Patent family member(s) | Publication date |
|---|---|---|---|
| EP 3945090 A1 | 02-02-2022 | EP 3945090 A1 | 02-02-2022 |
| | | EP 4496458 A2 | 22-01-2025 |
| EP 4321506 A1 | 14-02-2024 | CN 119677721 A | 21-03-2025 |
| | | EP 4321506 A1 | 14-02-2024 |
| | | WO 2024033322 A1 | 15-02-2024 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 2722908 A1


### Non-patent literature cited in the description


- **ALLEN** ; **LELAND C.** Electronegativity is the average one-electron energy of the valence-shell electrons in ground-state free atoms. *Journal of the American Chemical Society*, 1989, vol. 111 (25), 9003-9014
- *CHEMICAL ABSTRACTS*, 207739-72-8
- **YASUHIKO SHIROTA** ; **HIROSHI KAGEYAMA**. *Chem. Rev.*, 2007, vol. 107, 953-1010